(19) Europäisches Patentamt
European Patent Office
Office européen des brevets

(11) EP 3 385 332 A1

(12) EUROPEAN PATENT APPLICATION
published in accordance with Art. 153(4) EPC

(43) Date of publication:
**10.10.2018 Bulletin 2018/41**

(21) Application number: **16870745.3**

(22) Date of filing: **30.11.2016**

(51) Int Cl.:
***C08L 101/00*** (2006.01) ***A01N 25/10*** (2006.01)
***A01N 25/34*** (2006.01) ***A01N 43/00*** (2006.01)
***A01N 53/08*** (2006.01) ***A01P 7/00*** (2006.01)
***A01P 13/00*** (2006.01) ***A61K 47/32*** (2006.01)

(86) International application number:
**PCT/JP2016/085651**

(87) International publication number:
**WO 2017/094808 (08.06.2017 Gazette 2017/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.11.2015 JP 2015232903**

(71) Applicant: **Sumitomo Chemical Company, Limited**
**Tokyo 104-8260 (JP)**

(72) Inventor: **KONISHI Shota**
**Niihama-shi**
**Ehime 792-8521 (JP)**

(74) Representative: **Vossius & Partner**
**Patentanwälte Rechtsanwälte mbB**
**Siebertstrasse 3**
**81675 München (DE)**

# (54) RESIN PRODUCT AND MEDICINAL COMPONENT DISPENSING DEVICE

(57) The present invention provides a resin product comprising: an active ingredient; a thermoplastic resin (2) having a melting enthalpy (ΔH) of 30 J/g or more, as observed by differential scanning calorimetry within a temperature range of 10°C or more and less than 60°C; and a thermoplastic resin (3) having a storage elastic modulus E' at 60°C of $5.0 \times 10^5$ Pa or more, as determined by dynamic viscoelasticity measurement at a frequency of 10 Hz. The present invention also provides a molded article comprising the resin product and a device for sustained-release of an active ingredient comprising a component consisting of the resin product.

EP 3 385 332 A1

## Description

### Technical Field

The present invention relates to a resin product and a device for sustained release of an active ingredient.

### Background Art

Resin compositions containing an active ingredient and a resin have been widely used for sustained-release of the active ingredient so as to exert its effect for a long period of time. Some of such resin compositions are required to release an active ingredient only at a required temperature range. For example, Patent Document 1 discloses a composition comprising a temperature-sensitive polymer compound in the form of gel at a low temperature and in the form of sol at a high temperature and an active ingredient, as a composition that releases a drug preventing frostbite when the body surface temperature decreases, that is, a composition that releases a drug when the temperature becomes lower than a certain level.

### Prior Art Documents

### Patent Documents

Patent Document 1: JP2003-128587

### Disclosure of the Invention

### Problems to be solved by the Invention

On the other hand, it may be required to release an active ingredient when the temperature becomes equal to or higher than a certain level. An object of the present invention is to provide a resin product and a sustained-release device which are prone to release an active ingredient at a high temperature and are less prone to release an active ingredient at a low temperature, allowing sustained-release of the active ingredient for a long period of time.

### Means for solving the Problems

To achieve the above object, the present invention provides followings.

[1] A resin product comprising
an active ingredient;
a thermoplastic resin (2) having a melting enthalpy ($\Delta H$) of 30 J/g or more, as observed by differential scanning calorimetry within a temperature range of 10°C or more and less than 60°C; and
a thermoplastic resin (3) having a storage elastic modulus E' at 60°C of $5.0 \times 10^5$ Pa or more, as determined by dynamic viscoelasticity measurement at a frequency of 10 Hz.

[2] The resin product according to [1] wherein the thermoplastic resin (2) is a polymer (11) comprising a constitutional unit (B) represented by formula (1):

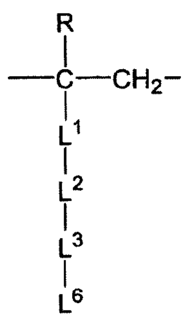

(1)

wherein

R represents hydrogen atom or methyl group,
$L^1$ represents a single bond, -CO-O-, -O-CO-, or -O-,
$L^2$ represents a single bond, $-CH_2-$, $-CH_2-CH_2-$, $-CH_2-CH_2-CH_2-$, $-CH_2-CH(OH)-CH_2-$, or $-CH_2-CH(CH_2OH)-$,
$L^3$ represents a single bond, -CO-O-, -O-CO-, -O-, -CO-NH-, -NH-CO-, -CO-NH-CO-, -NH-CO-NH-, -NH-, or $-N(CH_3)-$,
$L^6$ represents an alkyl group having 14 to 30 carbon atoms; in which the left side of the chemical formula recited for the definitions of the chemical structures of $L^1$, $L^2$ and $L^3$ corresponds to the top side of formula (1) and the right side thereof corresponds to the bottom side of formula (1).

[3] The resin product according to [2] wherein the polymer (11) further comprises a constitutional unit (A) derived from ethylene, and optionally, further at least one constitutional unit (C) selected from the group consisting of constitutional units represented by formula (2):

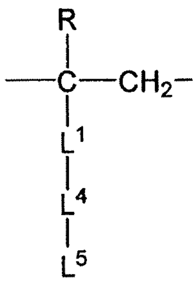

(2)

wherein

R represents hydrogen atom or methyl group,
$L^1$ represents a single bond, -CO-O-, -O-CO-, or -O-,
$L^4$ represents an alkylene group having 1 to 8 carbon atoms,
$L^5$ represents hydrogen atom, an epoxy group, $-CH(OH)-CH_2OH$, carboxyl group, hydroxyl group, amino group, or an alkylamino group having 1 to 4 carbon atoms,
in which the left side of the chemical formula recited for the definitions of the chemical structure of $L^1$ corresponds to the top side of formula (2) and the right side thereof corresponds to the bottom side of formula (2), and

the constitutional unit represented by formula (3):

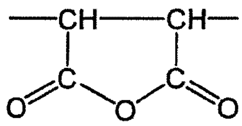

(3) ,

and the number of the constitutional unit (A) is 70% to 99% and the total number of the constitutional unit (B) and the constitutional unit (C) is 1% to 30%, based on 100% of the total number of the constitutional unit (A), the constitutional unit (B) and the constitutional unit (C), and
the number of the constitutional unit (B) is 1% to 100% and the number of the constitutional unit (C) is 0% to 99%, based on 100% of the total number of the constitutional unit (B) and the constitutional unit (C).

[4] The resin product according to [3] wherein the polymer (11) is a polymer wherein the total number of the constitutional unit (A), the constitutional unit (B) and the constitutional unit (C) is 90% or more, based on 100% of the total number of all constitutional units contained in the polymer (11).

[5] The resin product according to any one of [1] to [4] wherein the ratio A defined by the following equation (I) of the thermoplastic resin (2) or the polymer (11) is 0.95 or less

$$A = \alpha_1/\alpha_0 \qquad (I)$$

wherein
$\alpha_1$ is a value obtained by a method comprising: measuring the absolute molecular weight and the intrinsic viscosity of the thermoplastic resin (2) or the polymer (11) by gel permeation chromatography using an apparatus equipped with a light scattering detector and a viscosity detector, plotting the measured data with respect to the logarithm of the absolute molecular weight (horizontal axis) and the logarithm of the intrinsic viscosity (vertical axis), approximating according to the equation (I-I) in the least squares sense the logarithm of the absolute molecular weight and the logarithm of the intrinsic viscosity within the range of from the logarithm of the weight-average molecular weight of the thermoplastic resin (2) or the polymer (11) to the logarithm of the z-average molecular weight of the thermoplastic resin (2) or the polymer (11), and defining the slope of the line of the equation (I-I) as $\alpha_1$

$$\log[\eta_1] = \alpha_1 \log M_1 + \log K_1 \quad (I\text{-}I)$$

wherein $[\eta_1]$ represents the intrinsic viscosity (dl/g) of the thermoplastic resin (2) or the polymer (11), $M_1$ represents the absolute molecular weight of the thermoplastic resin (2) or the polymer (11), and $K_1$ is a constant, and
$\alpha_0$ is a value obtained by a method comprising: measuring the absolute molecular weight and the intrinsic viscosity of a polyethylene standard reference materials 1475a (available from National Institute of Standards and Technology) by gel permeation chromatography using an apparatus equipped with a light scattering detector and a viscosity detector, plotting the measured data with respect to the logarithm of the absolute molecular weight (horizontal axis) and the logarithm of the intrinsic viscosity (vertical axis), approximating according to the equation (I-II) in the least squares sense the logarithm of the absolute molecular weight and the logarithm of the intrinsic viscosity within the range of from the logarithm of the weight-average molecular weight of the polyethylene standard material 1475a to the logarithm of the z-average molecular weight of the polymer, and defining the slope of the line of the equation (I-II) as $\alpha_0$

$$\log[\eta_0] = \alpha_0 \log M_0 + \log K_0 \quad (I\text{-}II)$$

wherein $[\eta_0]$ represents the intrinsic viscosity (dl/g) of the polyethylene standard material 1475a, $M_0$ represents the absolute molecular weight of the polyethylene standard material 1475a, and $K_0$ is a constant, and
in the measurement of the absolute molecular weight and the intrinsic viscosity of the thermoplastic resin (2) or the polymer (11) and the polyethylene standard material 1475a by gel permeation chromatography, the mobile phase is orthodichlorobenzene and the measurement temperature is 155°C.

[6] The resin product according to any one of [1] to [5] wherein the thermoplastic resin (2) or the polymer (11) is a crosslinked polymer.

[7] The resin product according to any one of [1] to [6] wherein the gel fraction is 20% by weight or more, where the weight of the resin product is defined as 100% by weight.

[8] The resin product according to any one of [1] to [7] wherein the amount of the active ingredient contained in the resin product is 0.0001% to 50% by weight, based on 100% by weight of the total amount of the resin product, and the amount of the thermoplastic resin (2) or the polymer (11) is 1% to 99% by weight, based on 100% by weight of the total amount of the thermoplastic resin (2) or the polymer (11) and the thermoplastic resin (3).

[9] The resin product according to any one of [1] to [8], comprising a first layer containing the thermoplastic resin (2) or the polymer (11) and a second layer containing the thermoplastic resin (3) and an active ingredient.

[10] The resin product according to any one of [1] to [9] which is a molded article.

[11] A device for sustained-release of an active ingredient, comprising a component consisting of the resin product according to any one of [1] to [10].

**Effect of Invention**

According to the present invention, it is possible to obtain a resin product and a sustained-release device which are easy to release an active ingredient at a high temperature and are less prone to release an active ingredient at a

low temperature, allowing sustained release of the active ingredient for a long period of time.

**Description of Embodiments**

The embodiments of the present invention are described in detail below.
The resin product of the present invention comprises
an active ingredient,
a thermoplastic resin (2) having a melting enthalpy (ΔH) of 30 J/g or more observed in a temperature range of 10°C or more and less than 60°C by differential scanning calorimetry, and
a thermoplastic resin (3) having a storage elastic modulus E' of $5.0 \times 10^5$ Pa or more, which is determined by dynamic viscoelasticity measurement at a frequency of 10 Hz at 60°C.
The resin product of the present invention may be a resin composition or may form a multilayer structure.
The active ingredient of the present invention may be any substance that exhibits its effect when released outside the resin system, and examples of which include bioactive substances contained in agricultural chemicals, household control agents, antibacterial agents, mildew proofing agents, pharmaceuticals, fertilizers, fragrances, and the like.
Examples of the agricultural chemicals include insecticides, fungicides, insecticidal fungicides, herbicides, rodenticides, plant growth regulators, attractants, and the like.
Examples of the household control agents include pyrethroid compounds, organic phosphorus compounds, carbamate compounds, and the like.
From the viewpoint that the active ingredient is released to the outside of the system, either one of the melting point or the temperature of thermal decomposition is preferably 800°C or less.
The amount of the active ingredient to be contained in the resin product of the present invention is preferably 0.0001% to 50% by weight, more preferably 0.1% to 50% by weight, even more preferably 0.5% to 45% by weight, further preferably 1% to 45% by weight, and further preferably 2% to 40% by weight, based on 100% by weight of the total amount of the resin product.

<Thermoplastic resin (2)>

The thermoplastic resin (2) contained in the resin product of the present invention is that having a melting enthalpy (ΔH) of 30 J/g or more observed in a temperature range of 10°C or more and less than 60°C by differential scanning calorimetry. The ΔH observed in a temperature range of 10°C or more and less than 60°C is preferably 50 J/g or more, and more preferably 70 J/g or more. Also, the ΔH is usually 200 J/g or less.
As used herein, the melting enthalpy is referred to the heat of fusion determined by the analysis according to JIS K7122-1987 of a melt curve at the part within the range of 10°C to 60°C obtained by the differential scanning calorimetry as follows. When the thermoplastic resin (2) is a polymer (11) as follows, the ΔH can be brought into the above ranges by adjusting the number of the constitutional unit (B) in the polymer (11) and the number of carbon atoms of $L^6$ in formula (1) of the constitutional unit (B) .

[Differential scanning calorimetry]

An aluminum pan loaded with approximately 5 mg of sample is (1) held at 150°C for 5 minutes, then (2) cooled from 150°C to -50°C at a rate of 5°C/minute, then (3) held at -50°C for 5 minutes, and then (4) heated from -50°C to 150°C at a rate of 5°C/minute, using a differential scanning calorimeter under a nitrogen atmosphere. The differential scanning calorimetry curve produced by the calorimetric measurement in Step (4) is defined as a melt curve.
As used herein, the melting peak temperature of the thermoplastic resin is referred to a temperature which corresponds to the top of a melting peak, at which the melting endotherm is the maximum, determined by the analysis according to JIS K7121-1987 of a melt curve obtained by the differential scanning calorimetry as described above. When the melt curve has two or more melting peaks defined by JIS K7121-1987, the temperature of the top of the melting peak where the endotherm is the maximum is defined as the melting peak temperature.
The melting peak temperature of the thermoplastic resin (2) is preferably 10°C or more and less than 60°C, more preferably 10°C or more and less than 40°C, and even more preferably 10°C or more and less than 30°C.
When the thermoplastic resin (2) is a polymer (11) as follows, the melting peak temperature of the polymer (11) can be adjusted by adjusting the number of the constitutional unit (B) as follows in the polymer (11) and the number of carbon atoms of $L^6$ in the following formula (1) of the constitutional unit (B).
One embodiment of the thermoplastic resin (2) is a polymer comprising a constitutional unit having an alkyl group having 14 to 30 carbon atoms.
The thermoplastic resin (2) is preferably a polymer (11) comprising a constitutional unit (B) represented by the

following formula (1):

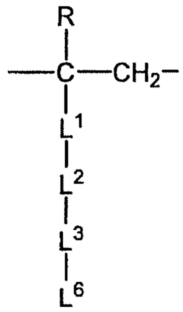

(1)

wherein

R represents hydrogen atom or methyl group,
$L^1$ represents a single bond, -CO-O-, -O-CO-, or -O-,
$L^2$ represents a single bond, $-CH_2-$, $-CH_2-CH_2-$, $-CH_2-CH_2-CH_2-$, $-CH_2-CH(OH)-CH_2-$, or $-CH_2-CH(CH_2OH)-$,
$L^3$ represents a single bond, -CO-O-, -O-CO-, -O-, -CO-NH-, -NH-CO-, -CO-NH-CO-, -NH-CO-NH-, -NH-, or $-N(CH_3)-$,
$L^6$ represents an alkyl group having 14 to 30 carbon atoms; in which the left side of the chemical formula recited for the definitions of the chemical structures of $L^1$, $L^2$ and $L^3$ corresponds to the top side of formula (1) and the right side thereof corresponds to the bottom side of formula (1).

Preferably, R is hydrogen atom.
$L^1$ is preferably -CO-O-, -O-CO- or -O-, more preferably -CO-O- or -O-CO-, and even more preferably -CO-O-.
$L^2$ is preferably a single bond, $-CH_2-$, $-CH_2-CH_2-$, or
$-CH_2-CH_2-CH_2-$, and more preferably a single bond.
$L^3$ is preferably a single bond, -O-CO-, -O-, -NH-, or $-N(CH_3)-$, and more preferably a single bond.
$L^6$ in formula (1) is an alkyl group having 14 to 30 carbon atoms. Examples of the alkyl group having 14 to 30 carbon atoms include straight chain alkyl groups having 14 to 30 carbon atoms and branched chain alkyl groups having 14 to 30 carbon atoms. $L^6$ is preferably a straight chain alkyl group having 14 to 30 carbon atoms, more preferably a straight chain alkyl group having 14 to 24 carbon atoms, and even more preferably a straight chain alkyl group having 16 to 22 carbon atoms.
Examples of the straight chain alkyl group having 14 to 30 carbon atoms include n-tetradecyl group, n-pentadecyl group, n-hexadecyl group, n-heptadecyl group, n-octadecyl group, n-nonadecyl group, n-eicosyl group, n-heneicosyl group, n-docosyl group, n-tricosyl group, n-tetracosyl group, n-pentacosyl group, n-hexacosyl group, n-heptacocyl group, n-octacosyl group, n-nonacosyl group and n-triacontyl group.
Examples of the branched chain alkyl group having 14 to 30 carbon atoms include isotetradecyl group, isopentadecyl group, isohexadecyl group, isoheptadecyl group, isooctadecyl group, isononadecyl group, isoeicosyl group, isoheneicosyl group, isodocosyl group, isotricosyl group, isotetracosyl group, isopentacosyl group, isohexacosyl group, isoheptacocyl group, isooctacosyl group, isononacosyl group, and isotriacontyl group.
Examples of the constitutional unit (B) represented by formula (1) include the followings.

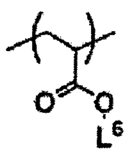 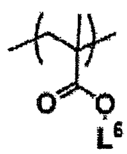 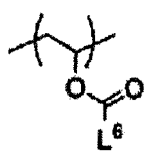 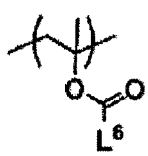 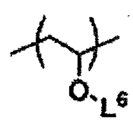 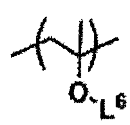

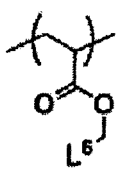 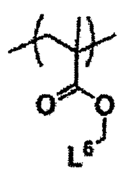 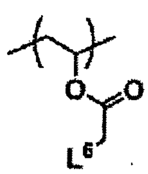 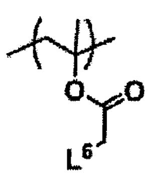 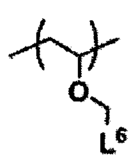 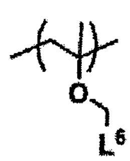

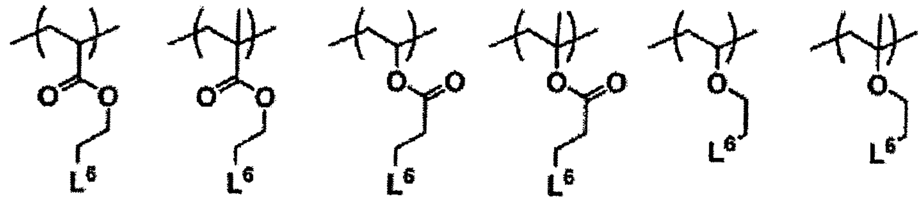

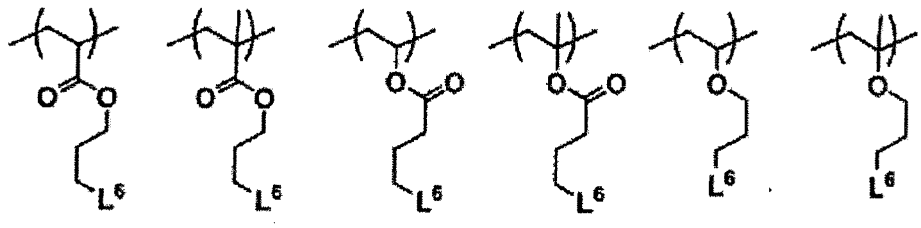

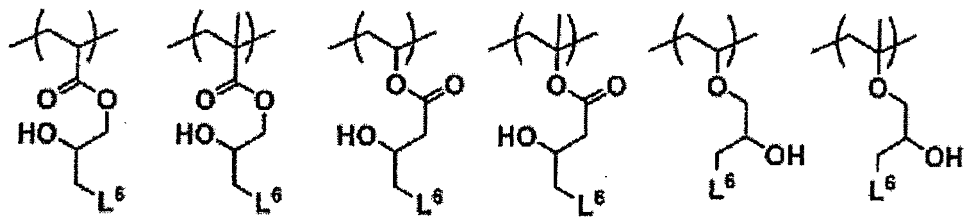

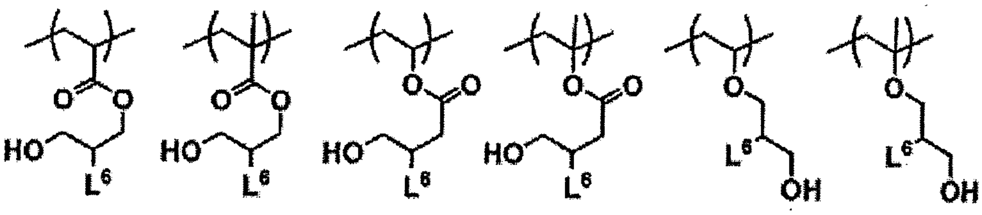

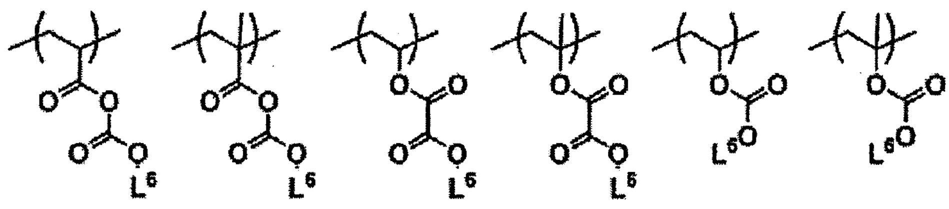

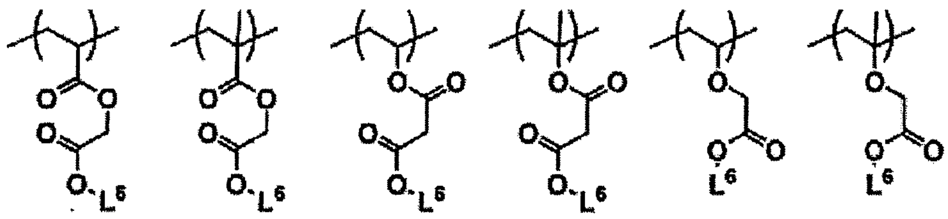

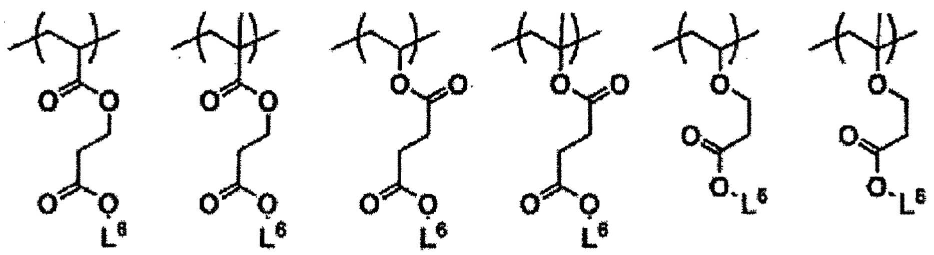

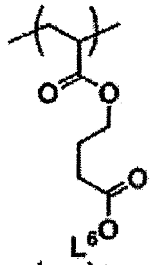 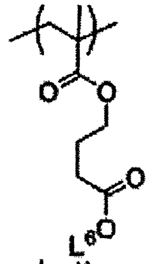 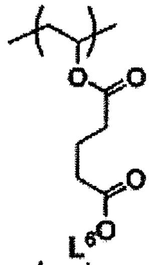 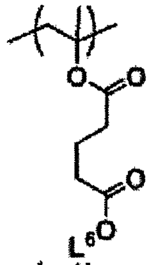 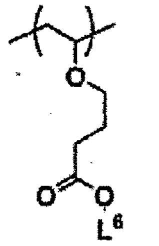 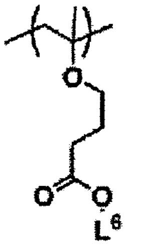

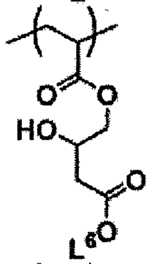 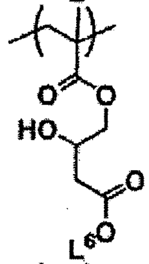 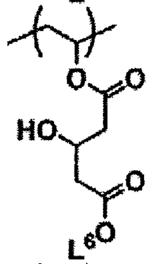 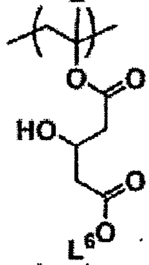 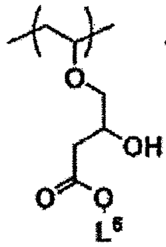 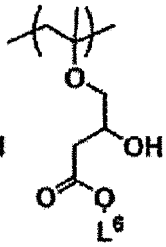

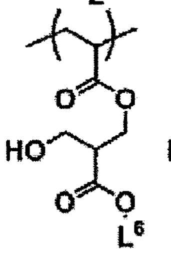 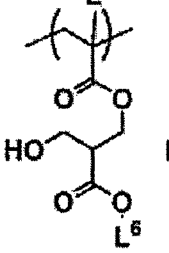 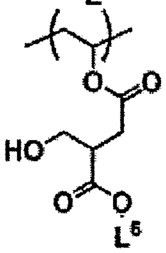 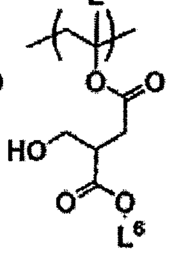 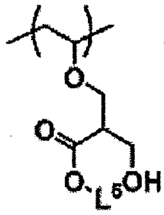 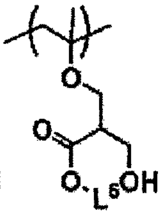

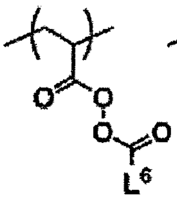 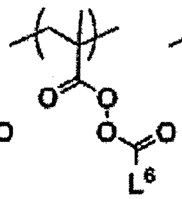 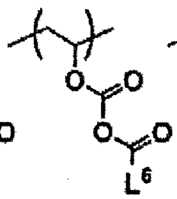 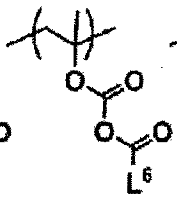 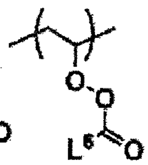 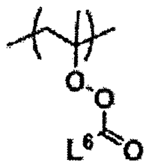

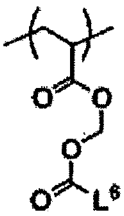 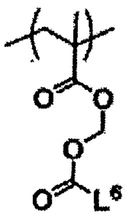 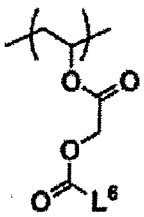 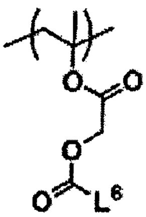 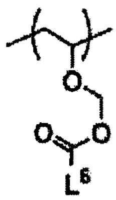 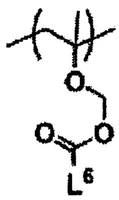

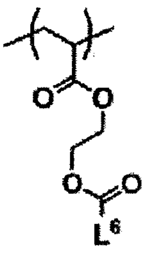 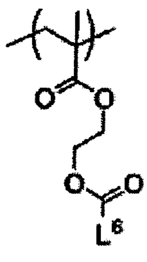 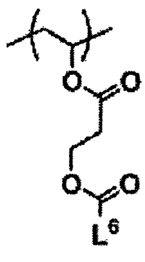 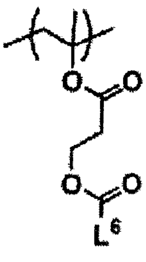 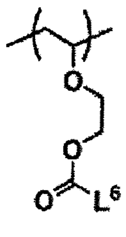 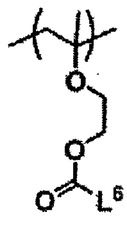

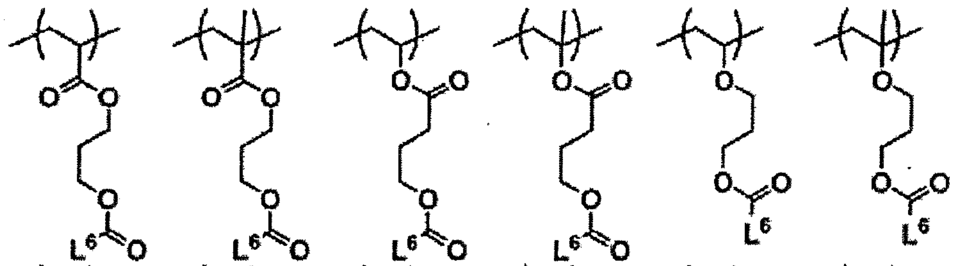

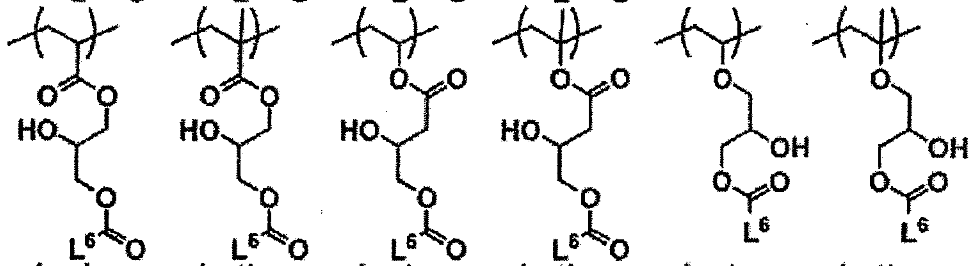

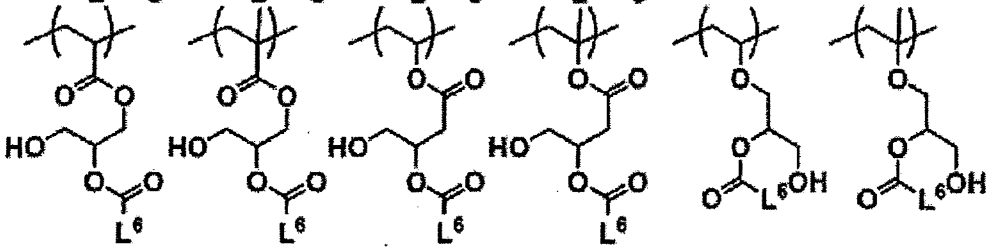

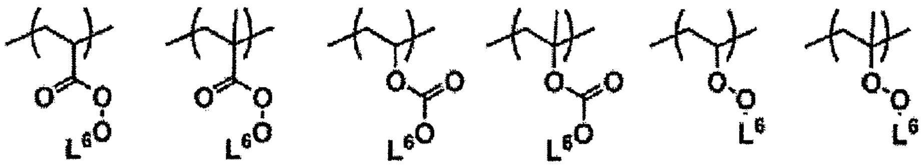

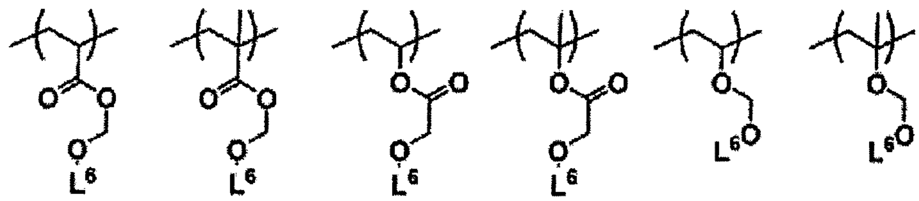

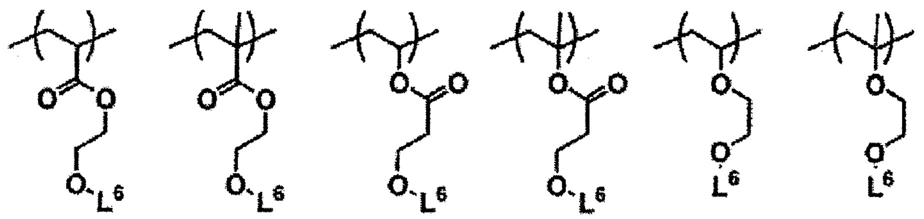

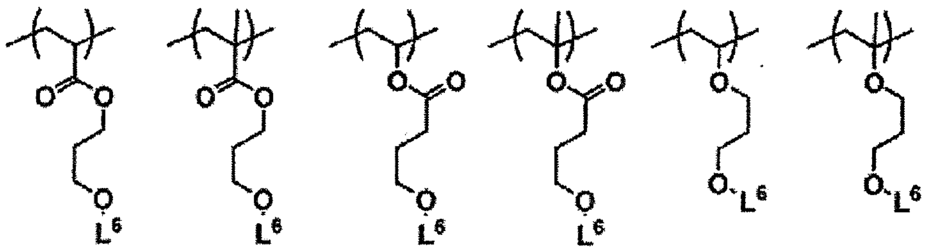

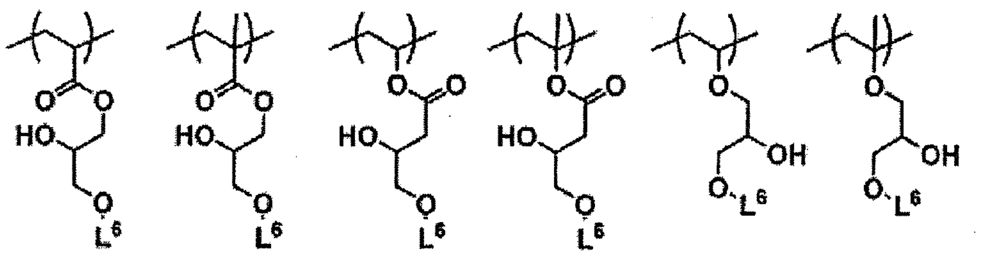

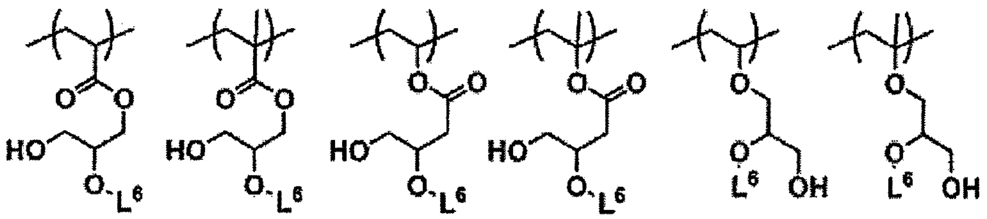

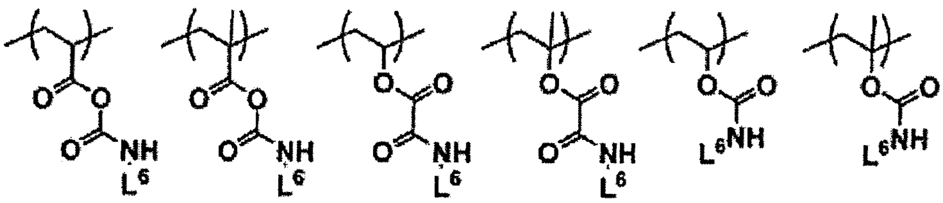

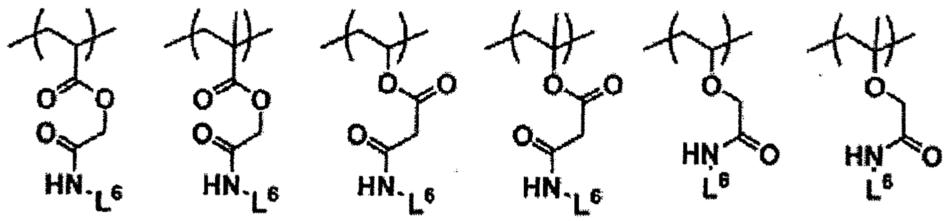

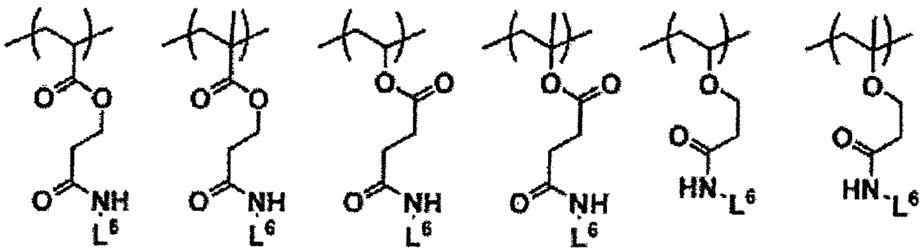

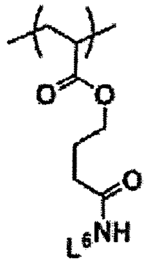 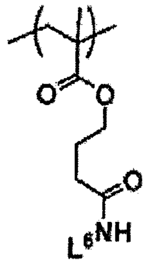 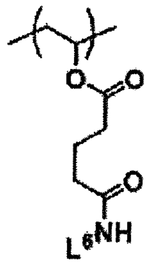 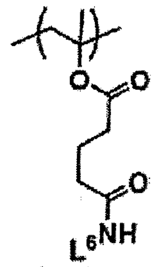 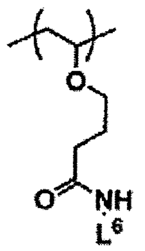 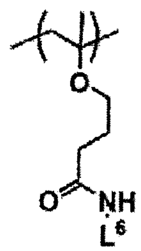

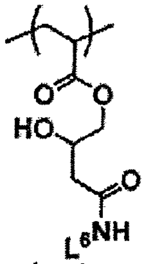 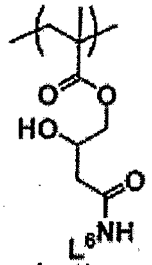 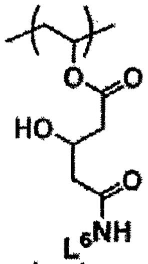 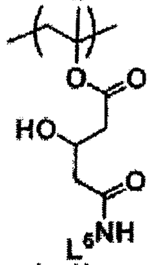 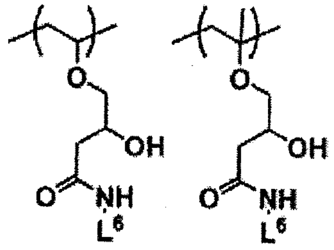

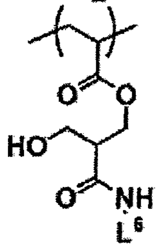 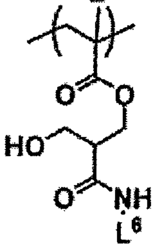 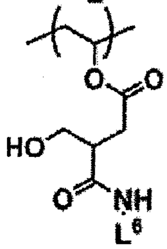 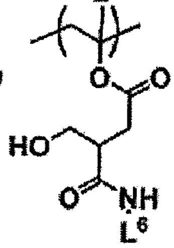 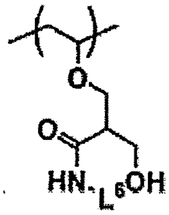 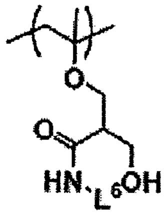

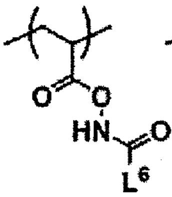 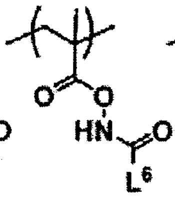 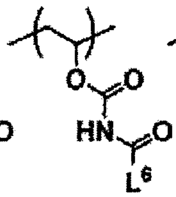 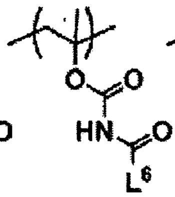 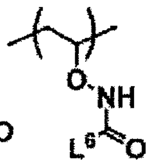 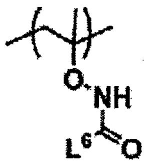

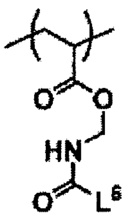 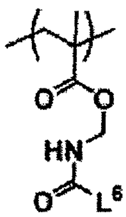 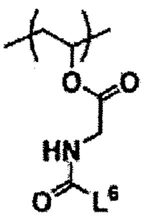 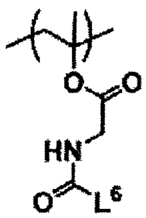 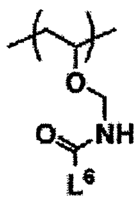 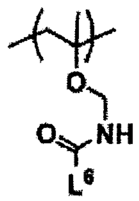

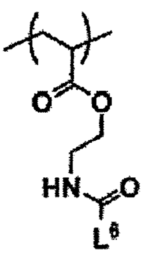 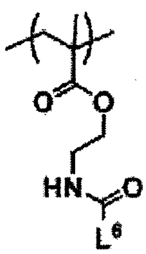 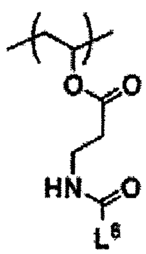 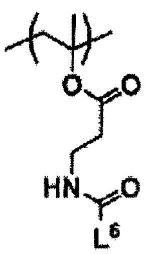 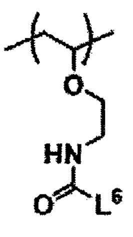 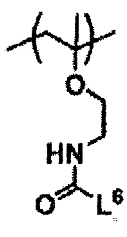

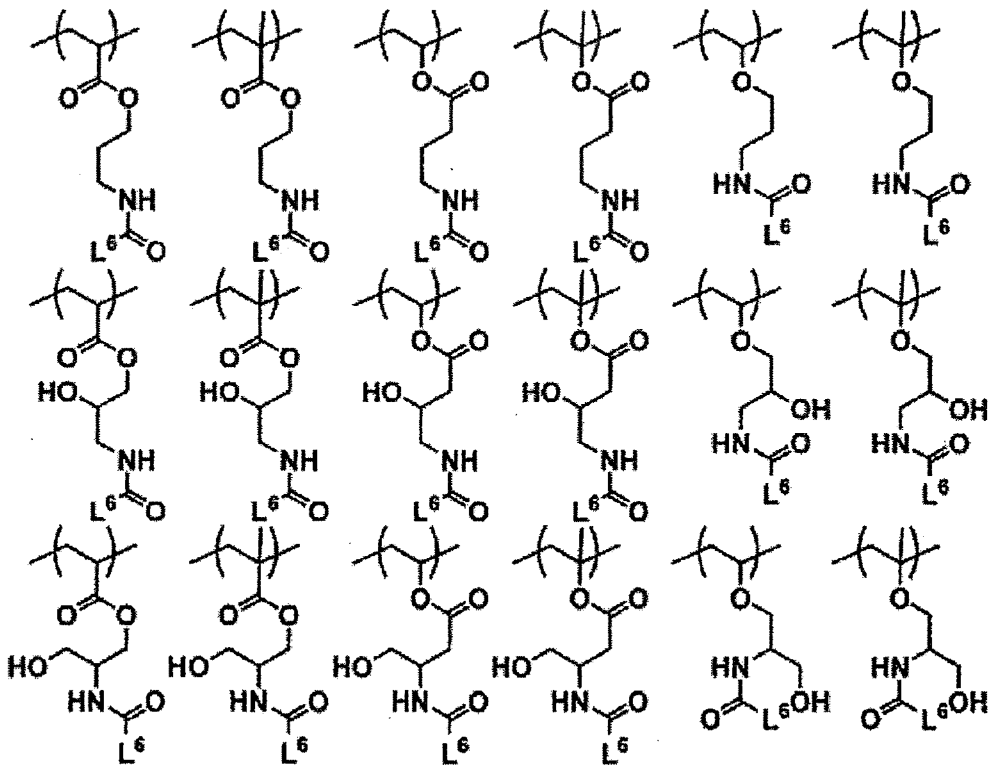

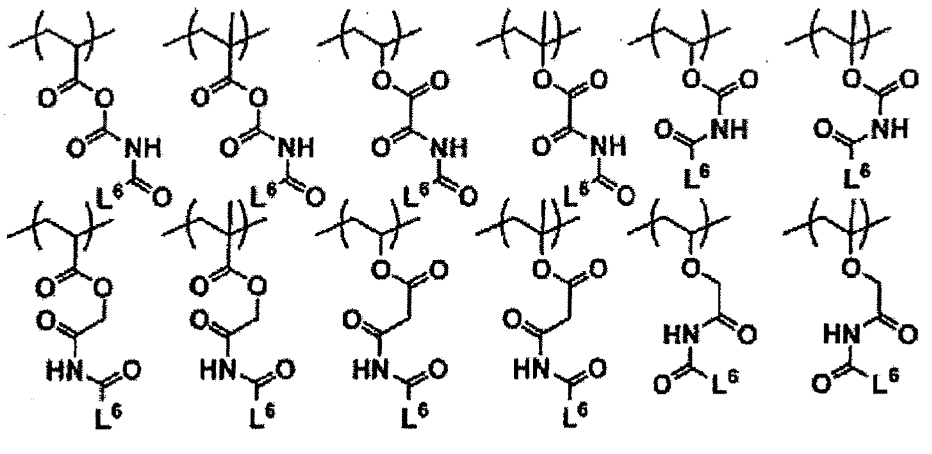

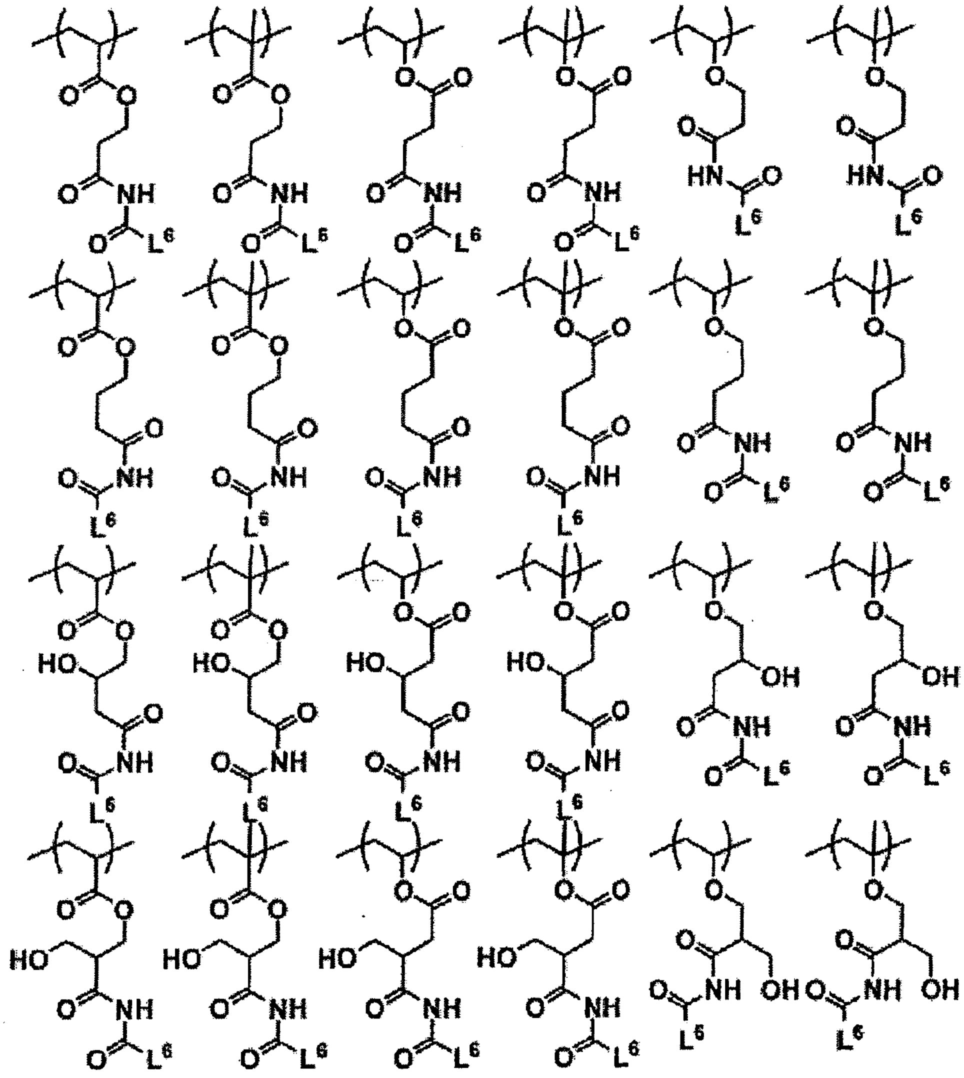

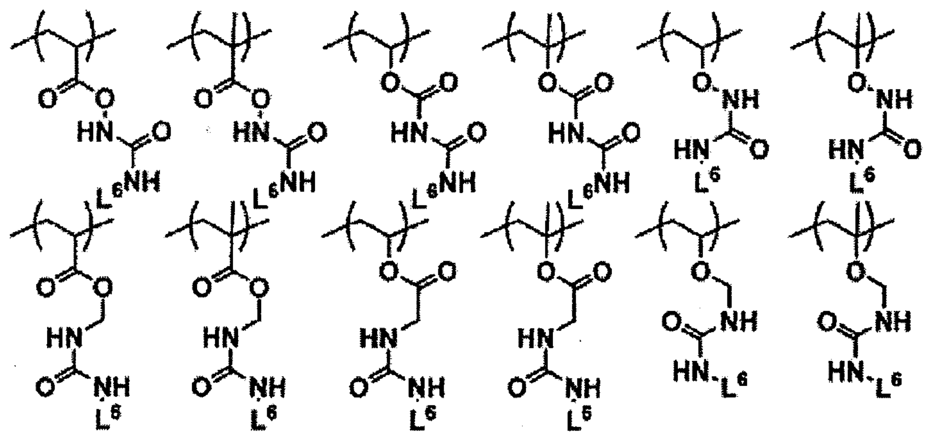

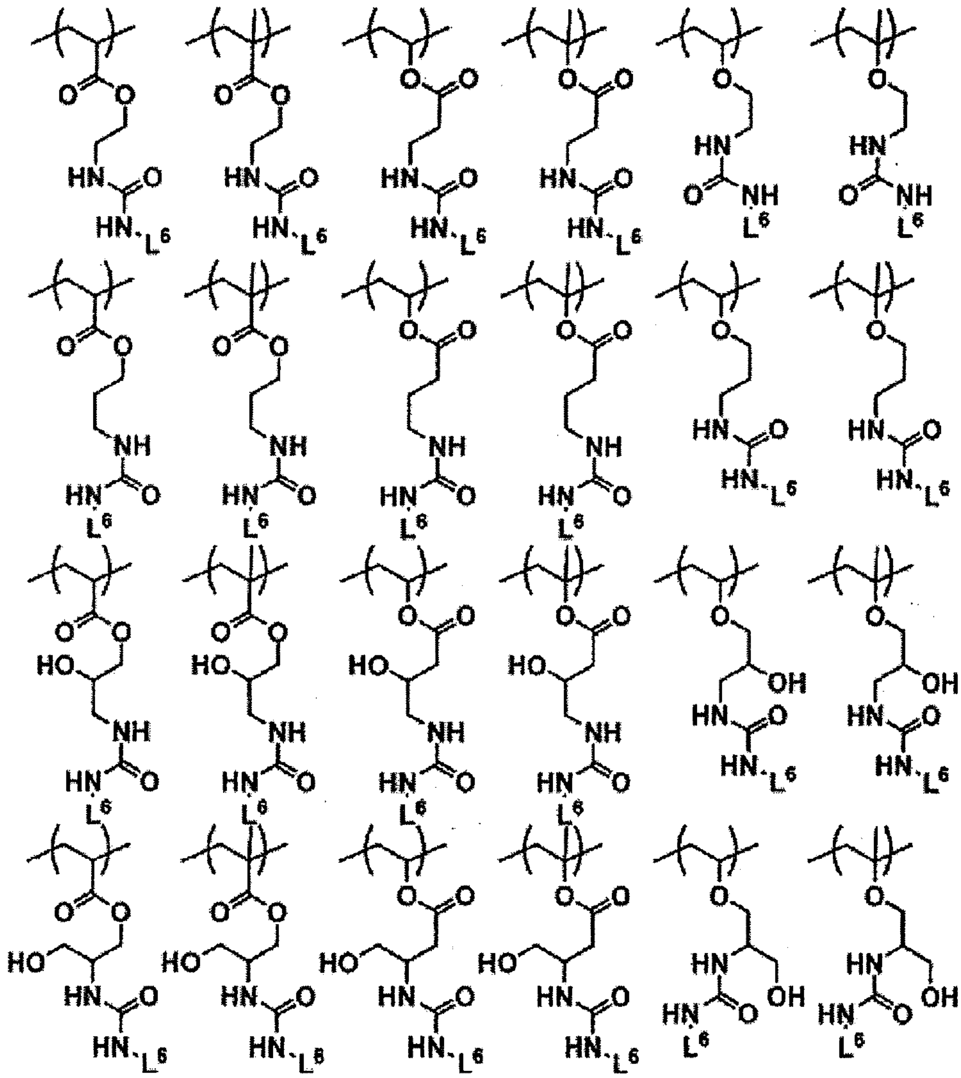

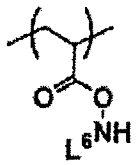 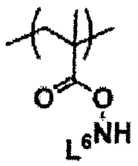 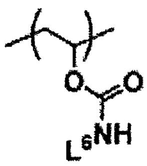 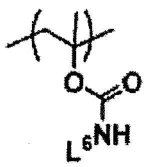 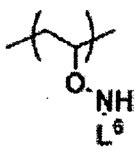 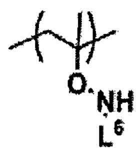

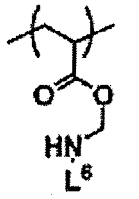 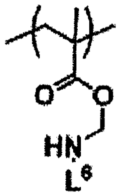 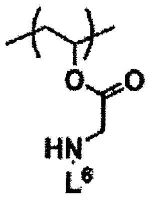 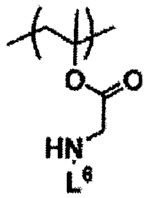 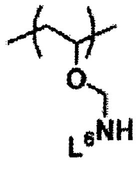 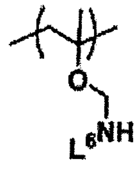

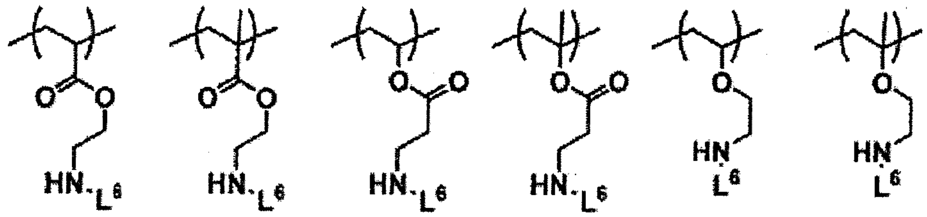

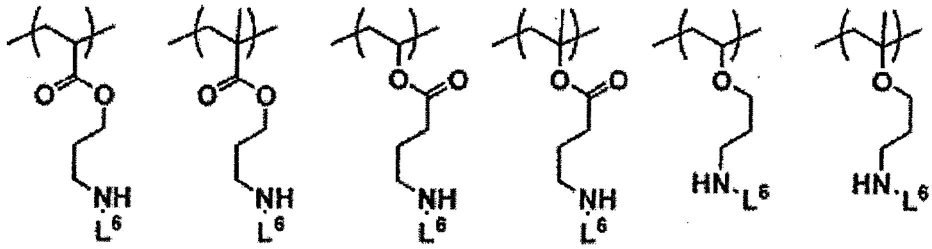

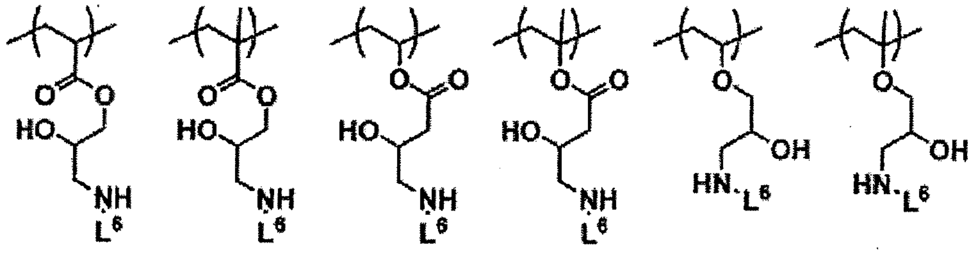

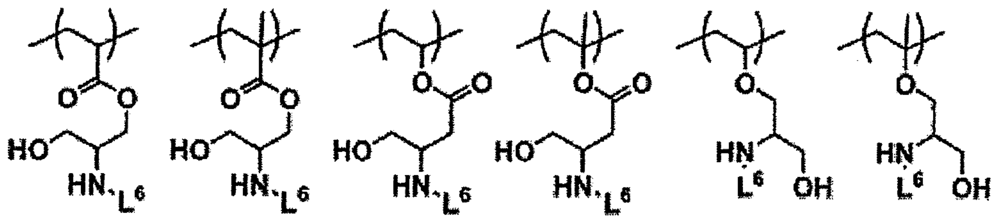

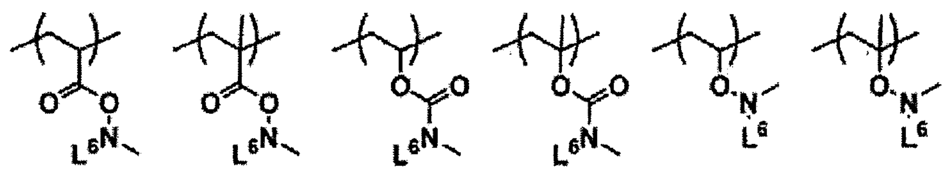

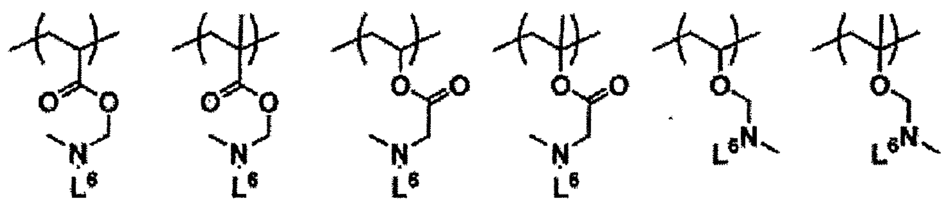

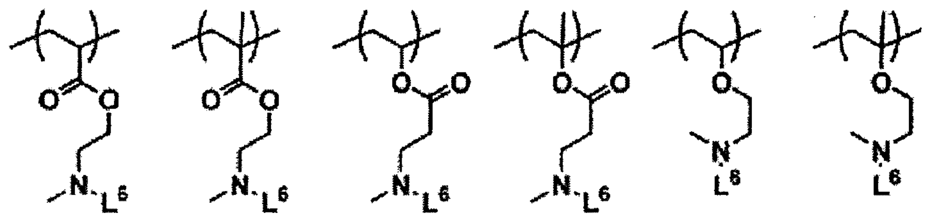

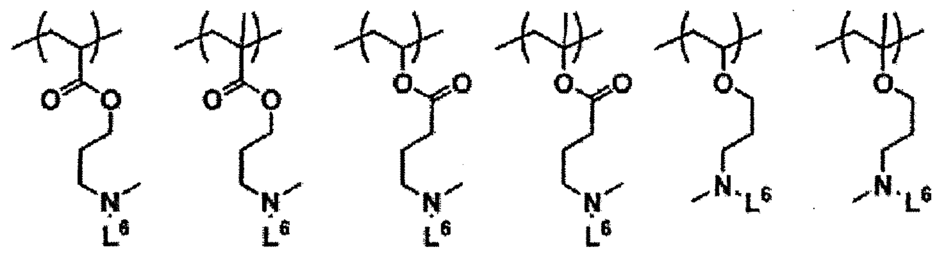

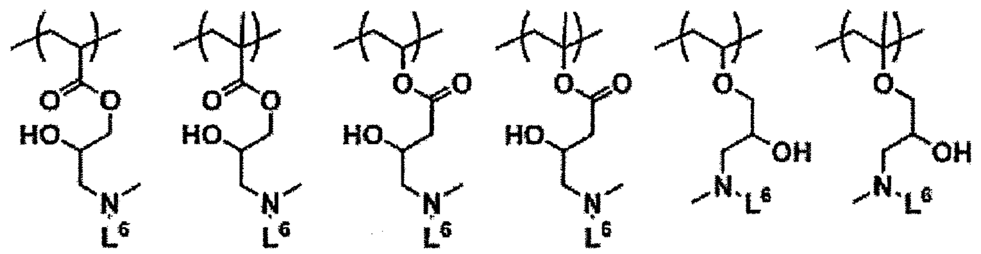

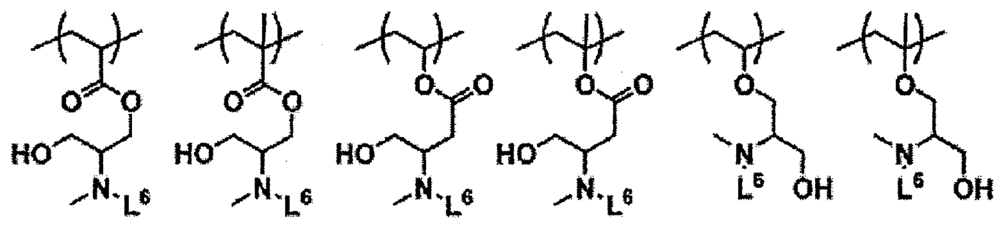

Preferably, examples of the constitutional unit (B) represented by formula (1) include the followings.

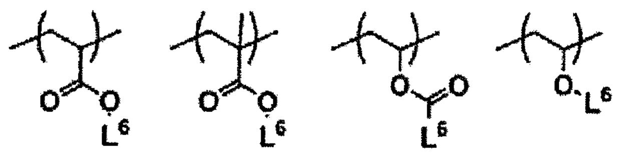

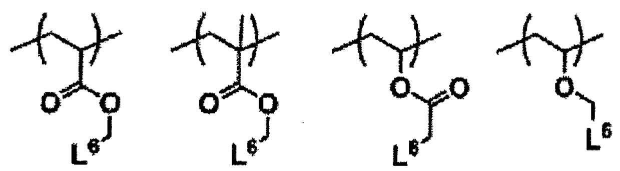

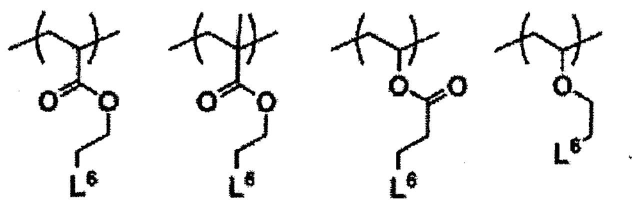

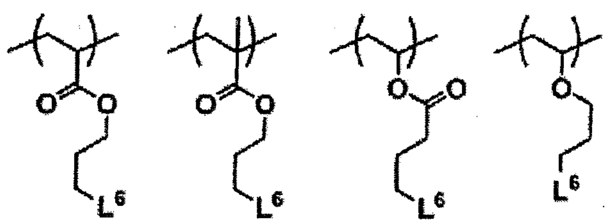

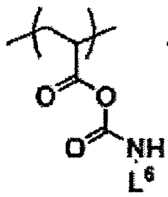
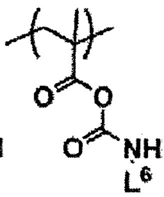
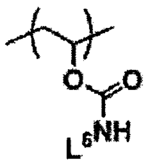

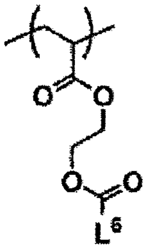
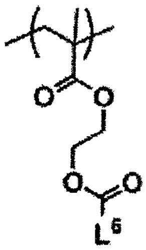
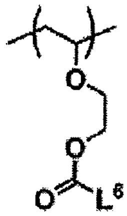

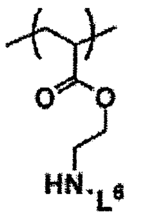
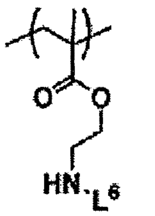
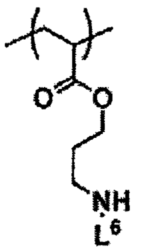
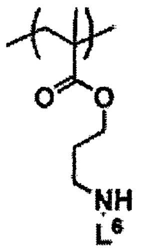

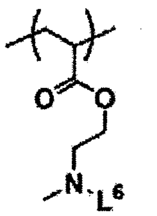
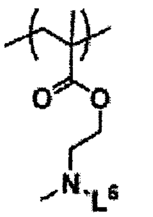
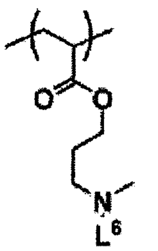
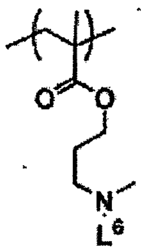

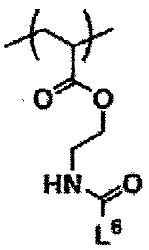
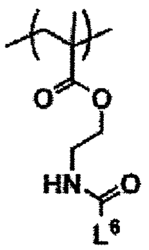
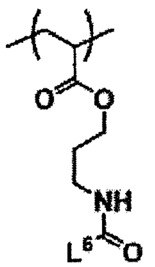
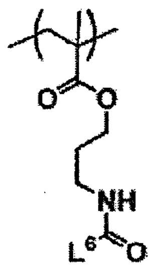

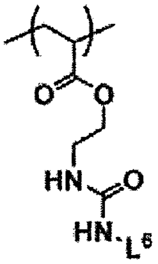
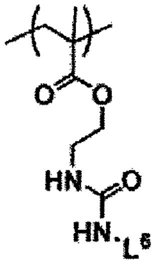
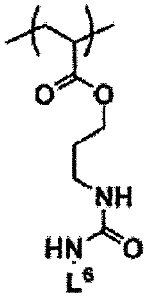
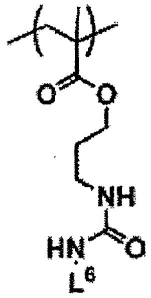

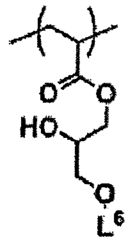 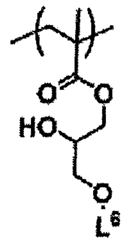 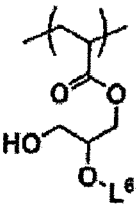 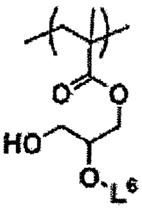

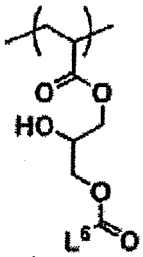 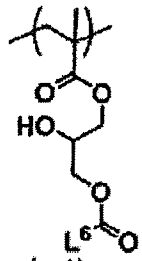 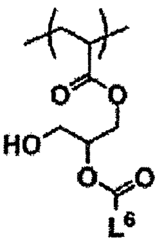 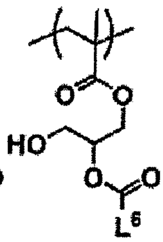

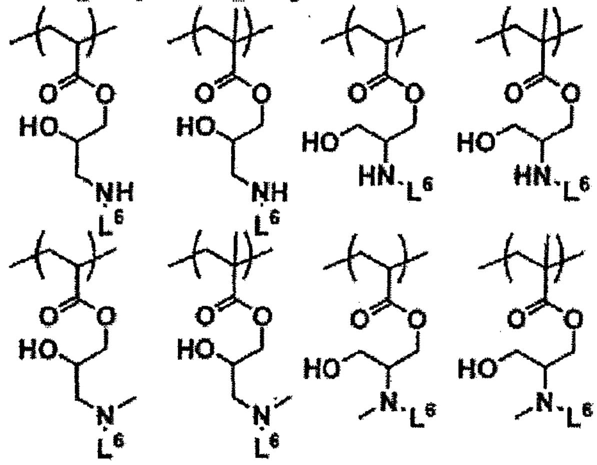

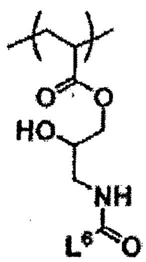 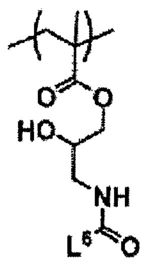 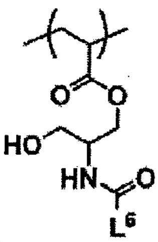 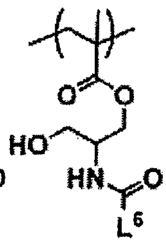

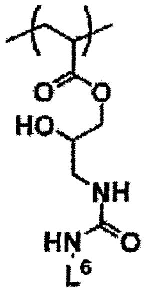 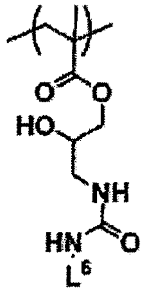 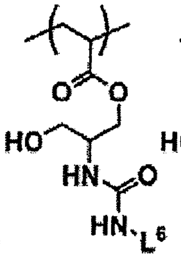 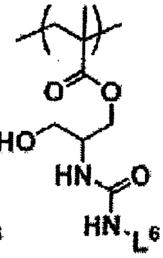

Examples of the constitutional unit (B) represented by formula (1) also include those wherein R is hydrogen atom, $L^1$, $L^2$ and $L^3$ are a single bond; and $L^6$ is an alkyl group having 14 to 30 carbon atoms, or those wherein R is hydrogen atom or methyl group; $L^1$ is -CO-O-; $L^2$ and $L^3$ are a single bond; and $L^6$ is an alkyl group having 14 to 30 carbon atoms.

More preferably, examples of the constitutional unit (B) represented by formula (1) include the followings.

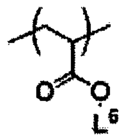 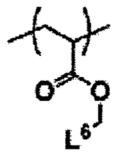 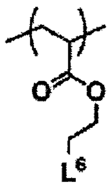 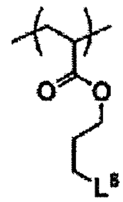

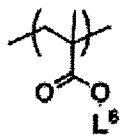 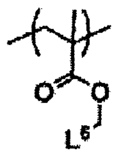 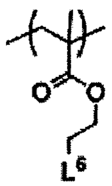 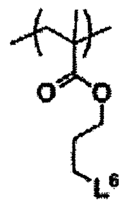

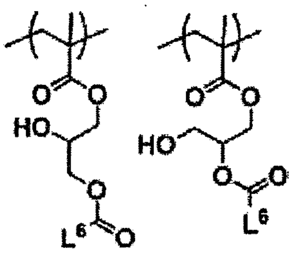

Even more preferably, examples of the constitutional unit (B) represented by formula (1) include the following.

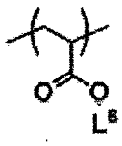

Preferably, the constitutional unit (B) is a constitutional unit derived from n-hexadecene, a constitutional unit derived from n-octadecene, a constitutional unit derived from n-eicosene, a constitutional unit derived from n-docosene, a constitutional unit derived from n-tetracosene, a constitutional unit derived from n-hexacosene, a constitutional unit derived from n-octacosene, a constitutional unit derived from n-triacontene, a constitutional unit derived from n-dotriacontene, a constitutional unit derived from n-tetradecyl acrylate, a constitutional unit derived from n-pentadecyl acrylate, a constitutional unit derived from n-hexadecyl acrylate, a constitutional unit derived from n-heptadecyl acrylate, a constitutional unit derived from n-octadecyl acrylate, a constitutional unit derived from n-nonadecyl acrylate, a constitutional unit derived from n-eicosyl acrylate, a constitutional unit derived from n-heneicosyl acrylate, a constitutional unit derived from n-docosyl acrylate, a constitutional unit derived from n-tricosyl acrylate, a constitutional unit derived from n-tetracosyl acrylate, a constitutional unit derived from n-pentacosyl acrylate, a constitutional unit derived from n-hexacosyl acrylate, a constitutional unit derived from n-heptacosyl acrylate, a constitutional unit derived from n-octacosyl acrylate, a constitutional unit derived from n-nonacosyl acrylate, a constitutional unit derived from n-triacontyl acrylate, a constitutional unit derived from n-tetradecyl methacrylate, a constitutional unit derived from n-pentadecyl methacrylate, a constitutional unit derived from n-hexadecyl methacrylate, a constitutional unit derived from n-heptadecyl methacrylate, a constitutional unit derived from n-octadecyl methacrylate, a constitutional unit derived from n-nonadecyl methacrylate, a constitutional unit derived from n-eicosyl methacrylate, a constitutional unit derived from n-heneicosyl methacrylate, a constitutional unit derived from n-docosyl methacrylate, a constitutional unit derived from n-tricosyl methacrylate, a constitutional unit derived from n-tetracosyl methacrylate, a constitutional unit derived from n-pentacosyl methacrylate, a constitutional unit derived from n-hexacosyl methacrylate, a constitutional unit derived from n-heptacosyl methacrylate, a constitutional unit derived from n-octacosyl methacrylate, a constitutional unit derived from n-nonacosyl methacrylate, a constitutional unit derived from n-triacontyl methacrylate, a constitutional unit derived from n-vinyl tetradecylate, a constitutional unit derived from n-vinyl hexadecylate, a constitutional unit derived from n-vinyl octadecylate, a constitutional unit derived

from n-vinyl eicosylate, a constitutional unit derived from n-vinyl docosylate, a constitutional unit derived from n-tetradecyl vinyl ether, a constitutional unit derived from n-hexadecyl vinyl ether, a constitutional unit derived from n-octadecyl vinyl ether, a constitutional unit derived from n- eicosyl vinyl ether, or a constitutional unit derived from n-docosyl vinyl ether.

The constitutional unit derived from a specific compound as referred to herein is a constitutional unit produced by polymerizing said compound.

The polymer (11) may have two or more of the constitutional unit (B), such as that having a constitutional unit derived from n-eicosyl acrylate and a constitutional unit derived from n-octadecyl acrylate.

The polymer (11) preferably has further a constitutional unit (A) derived from ethylene so as to provide good shape retention and molding processability of the resin product of the invention at a temperature higher than the melting peak temperature of the polymer (11). Such constitutional unit (A) is a constitutional unit produced by polymerizing ethylene, and also, the constitutional unit (A) may form a branched structure in the polymer.

The polymer (11) is preferably a polymer comprising a constitutional unit (B) represented by formula (1) and a constitutional unit (A) derived from ethylene.

The polymer (11) may have at least one constitutional unit (C) selected from the group consisting of constitutional units represented by formula (2):

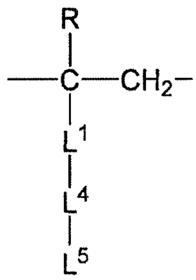

(2)

wherein

R represents hydrogen atom or methyl group,
$L^1$ represents a single bond, -CO-O-, -O-CO-, or -O-,
$L^4$ represents an alkylene group having 1 to 8 carbon atoms,
$L^5$ represents hydrogen atom, an epoxy group, $-CH(OH)-CH_2OH$, carboxyl group, hydroxyl group, amino group, or an alkylamino group having 1 to 4 carbon atoms;

in which the left side of the chemical formula recited for the definitions of the chemical structures of $L^1$, $L^2$ and $L^3$ corresponds to the top side of formula (2) and the right side thereof corresponds to the bottom side of formula (2); and the constitutional unit represented by formula (3):

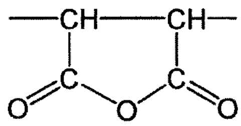

(3) .

In formula (2), R is preferably hydrogen atom.

In formula (2), $L^1$ is preferably -CO-O-, -O-CO- or -O-, more preferably -CO-O- or -O-CO-, and even more preferably -CO-O-.

In formula (2), examples of the alkylene group having 1 to 8 carbon atoms as $L^4$ include methylene group, ethylene group, n-propylene group, 1-methylethylene group, n-butylene group, 1,2-dimethylethylene group, 1,1-dimethylethylene group, 2, 2-dimethylethylene group, n-pentylene group, n-hexylene group, n-heptalene group, n-octylene group, and 2-ethyl-n-hexylene group.

$L^4$ is preferably methylene group, ethylene group, or n-propylene group, and more preferably methylene group.

In formula (2), examples of the alkylamino group having 1 to 4 carbon atoms as $L^5$ include methylamino group, ethylamino group, propylamino group, butylamino group, dimethylamino group, and diethylamino group.

In formula (2), $L^5$ is preferably hydrogen atom, an epoxy group, or $-CH(OH)-CH_2OH$, and more preferably hydrogen atom.

Examples of the constitutional unit represented by formula (2) include the followings.

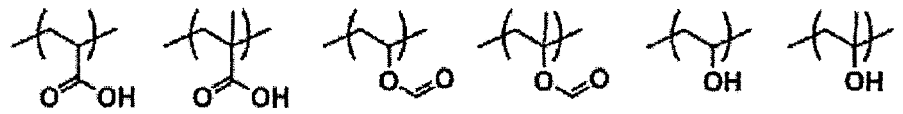

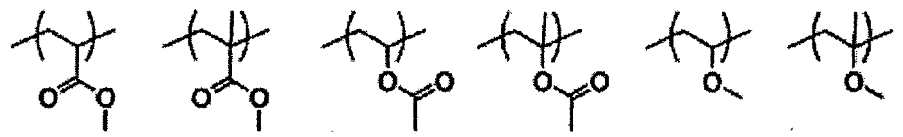

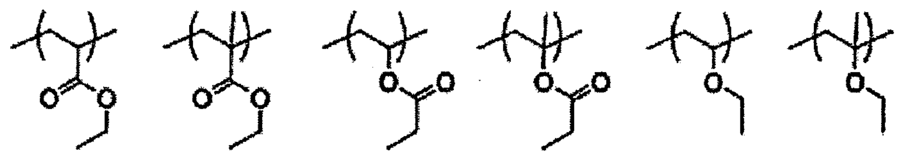

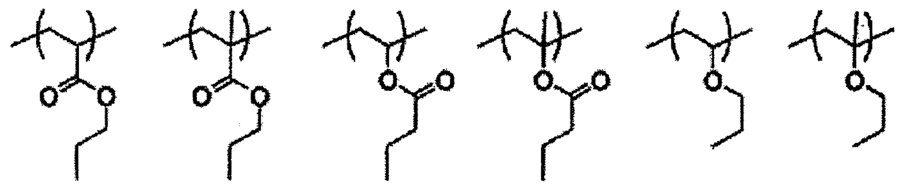

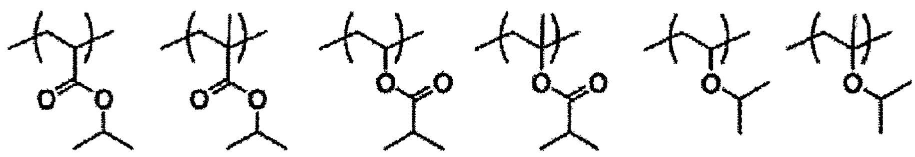

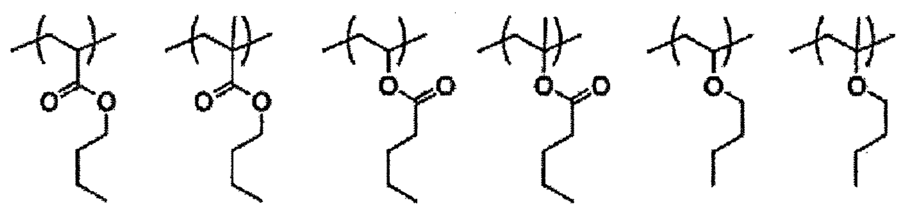

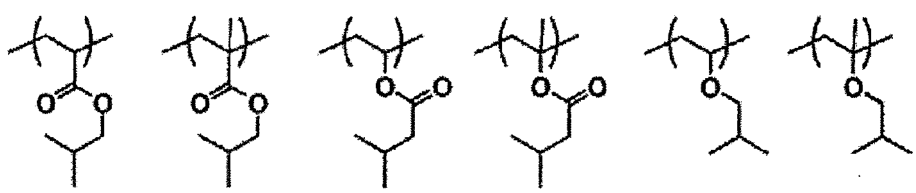

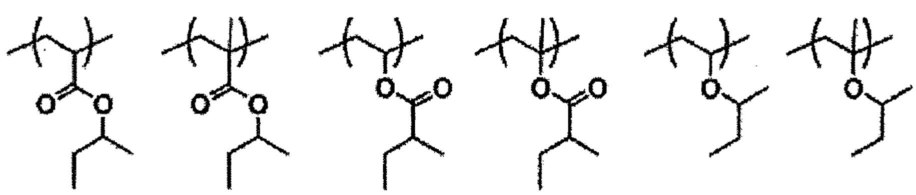

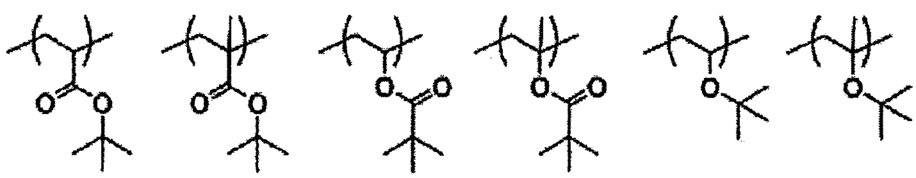

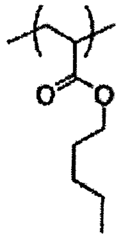
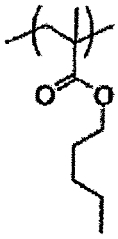
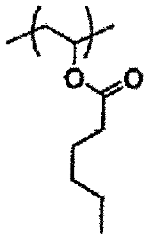
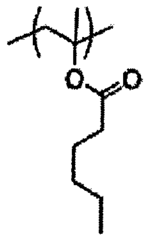
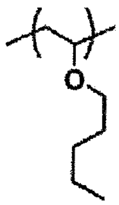
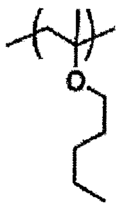

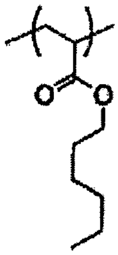
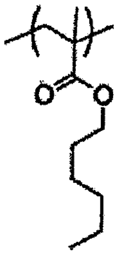
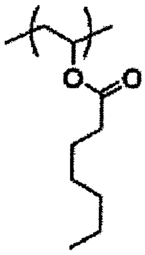
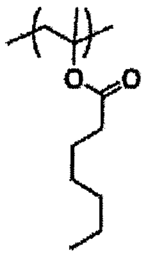
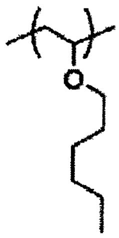
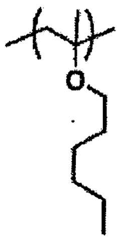

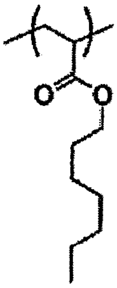
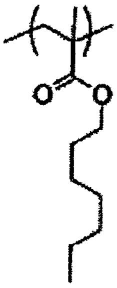
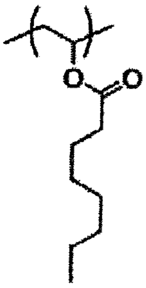
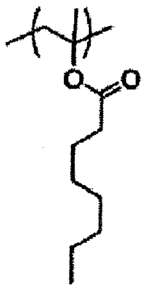
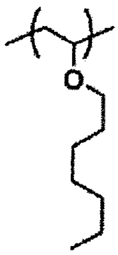
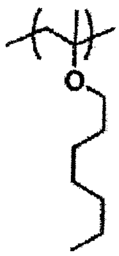

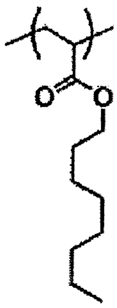
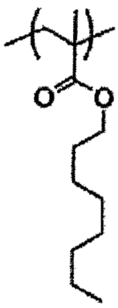
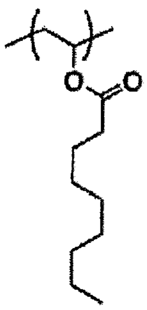
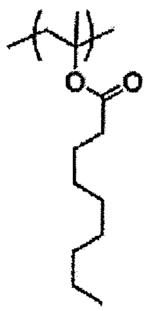
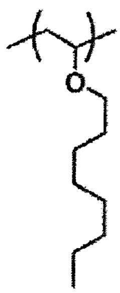
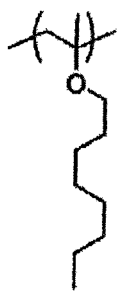

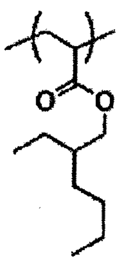
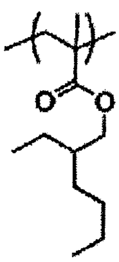
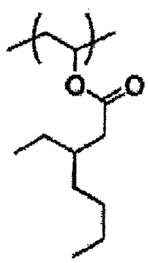
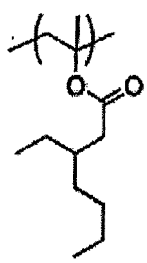
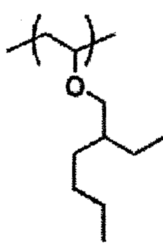
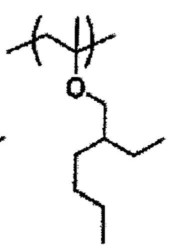

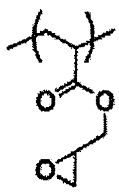
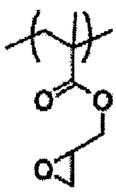
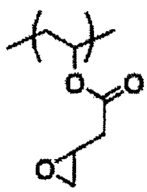
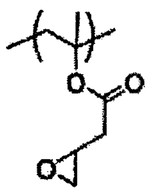
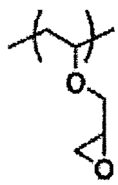
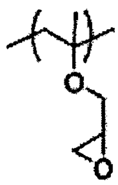

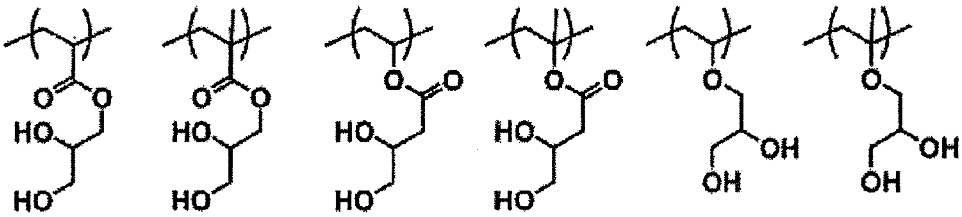

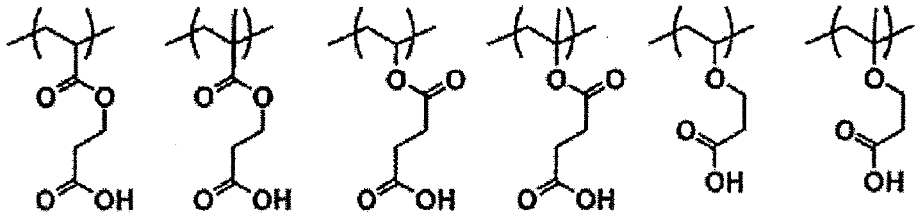

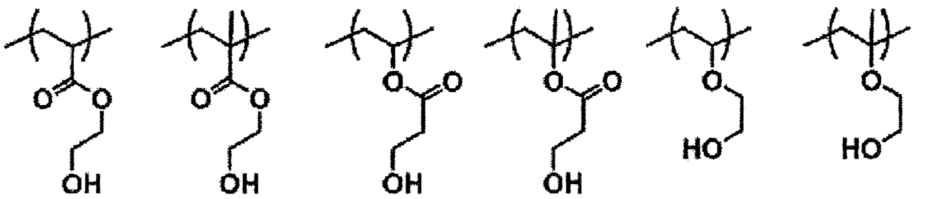

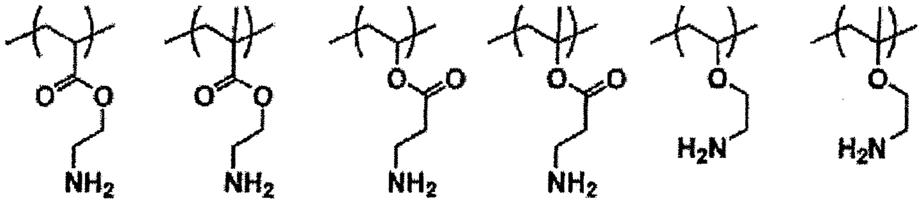

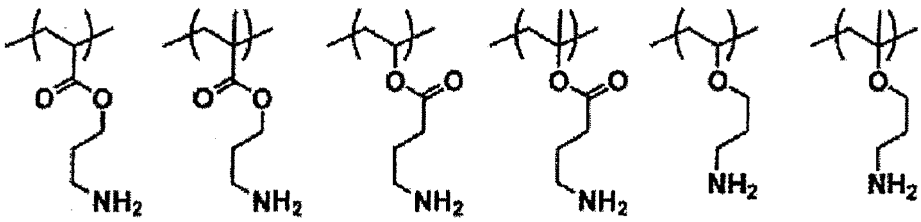

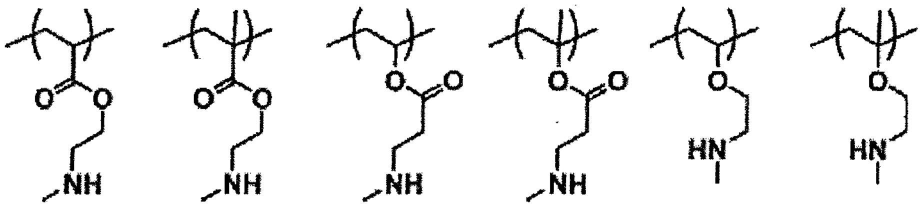

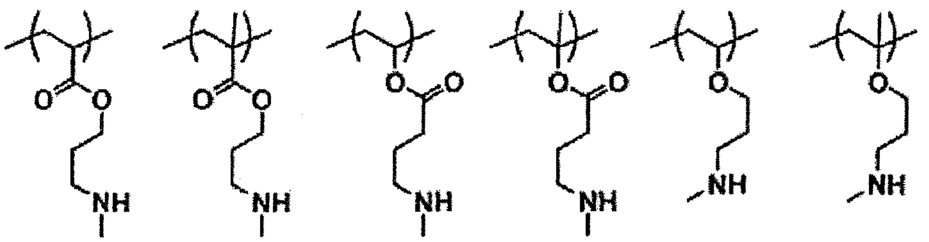

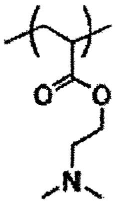 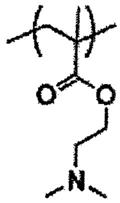 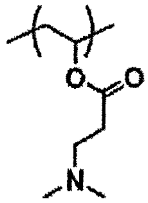 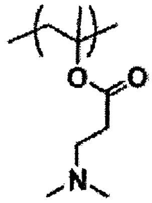 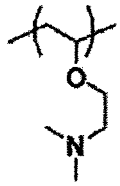 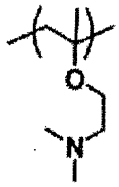

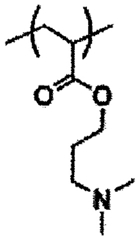 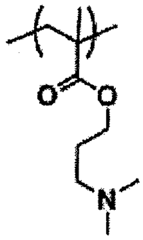 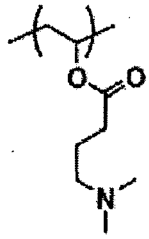 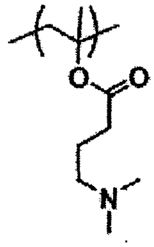 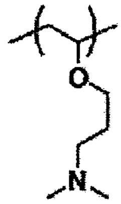 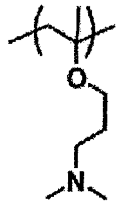

Preferably, examples of the constitutional unit represented by formula (2) include the followings.

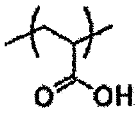 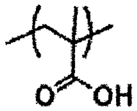 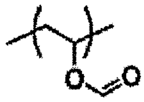 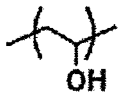

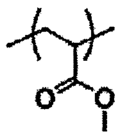 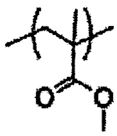 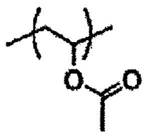 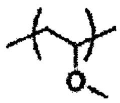

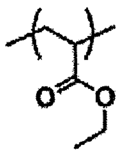 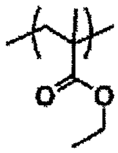 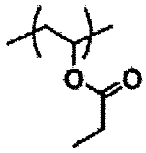 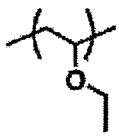

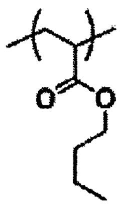 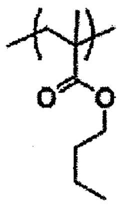 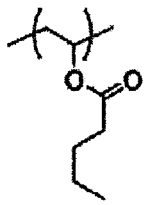 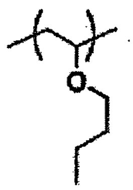

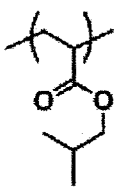 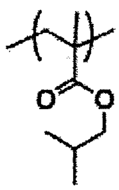 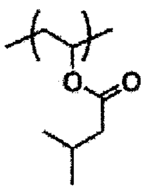 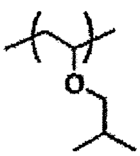

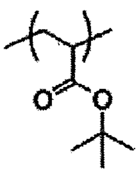 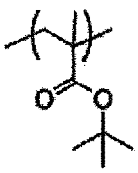 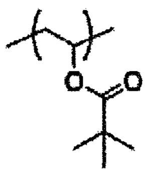 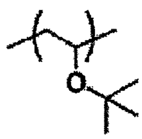

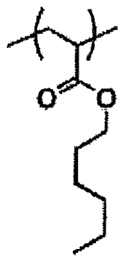
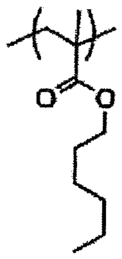
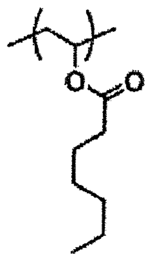
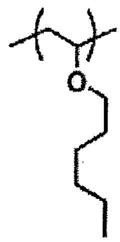

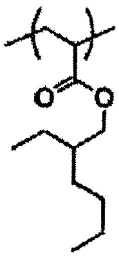
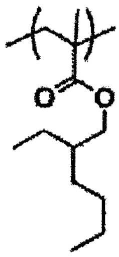
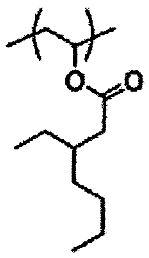
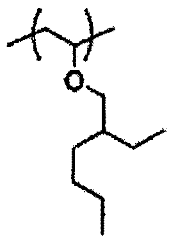

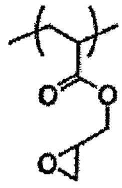
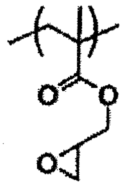
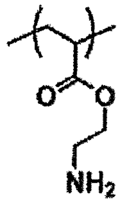
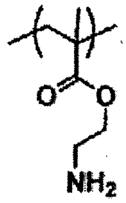

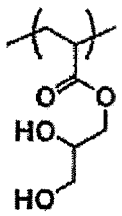
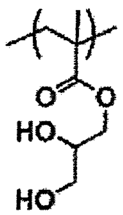
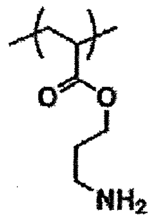
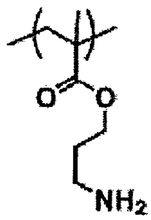

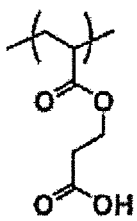
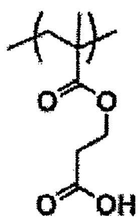
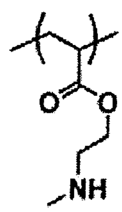
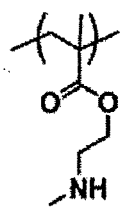

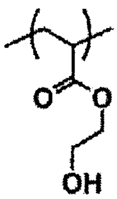 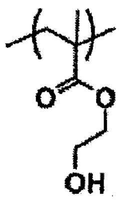 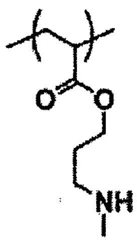 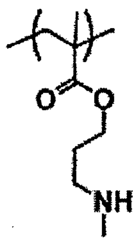

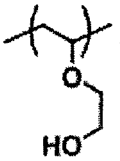 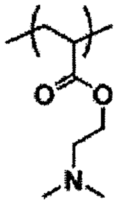 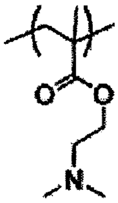

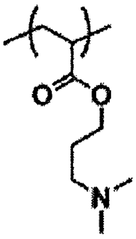 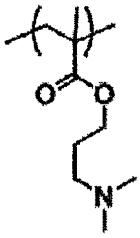

More preferably, examples of the constitutional unit represented by formula (2) include the followings.

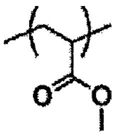 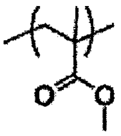 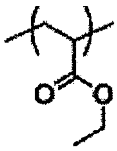 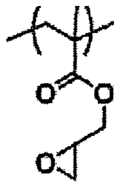 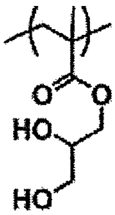

Even more preferably, examples of the constitutional unit represented by formula (2) include the following.

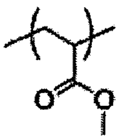

Examples of the constitutional unit represented by formula (2) include a constitutional unit derived from propylene, a constitutional unit derived from butene, a constitutional unit derived from 1-pentene, a constitutional unit derived from 1-hexene, a constitutional unit derived from 1-heptene, a constitutional unit derived from 1-octene, a constitutional unit derived from acrylic acid, a constitutional unit derived from methacrylic acid, a constitutional unit derived from vinyl alcohol, a constitutional unit derived from methyl acrylate, a constitutional unit derived from ethyl acrylate, a constitutional unit derived from n-propyl acrylate, a constitutional unit derived from isopropyl acrylate, a constitutional unit derived from n-butyl acrylate, a constitutional unit derived from isobutyl acrylate, a constitutional unit derived from sec-butyl acrylate, a constitutional unit derived from tert-butyl acrylate, a constitutional unit derived from methyl methacrylate, a constitutional unit derived from ethyl methacrylate, a constitutional unit derived from n-propyl methacrylate, a constitutional unit derived from isopropyl methacrylate, a constitutional unit derived from n-butyl methacrylate, a constitutional unit derived from isobutyl methacrylate, a constitutional unit derived from sec-butyl methacrylate, a constitutional unit derived from tert-butyl methacrylate, a constitutional unit derived from vinyl formate, a constitutional unit derived from vinyl acetate, a constitutional unit derived from vinyl propionate, a constitutional unit derived from vinyl (n-butylate), a constitutional unit derived from vinyl (isobutylate), a constitutional unit derived from methylvinyl ether, a constitutional unit derived from ethylvinyl ether, a constitutional unit derived from n-propylvinyl ether, a constitutional unit derived from isopropylvinyl

ether, a constitutional unit derived from n-butylvinyl ether, a constitutional unit derived from isobutylvinyl ether, a constitutional unit derived from sec-butylvinyl ether, a constitutional unit derived from tert-butylvinyl ether, a constitutional unit derived from glycidyl acrylate, a constitutional unit derived from glycidyl methacrylate, a constitutional unit derived from 2,3-dihydroxypropyl acrylate, a constitutional unit derived from 2,3-dihydroxypropyl methacrylate, a constitutional unit derived from 3-(dimethylamino)propyl acrylate, and a constitutional unit derived from 3-(dimethylamino)propyl methacrylate.

The constitutional unit represented by formula (3) is a constitutional unit derived from maleic anhydride.

The polymer (11) may be a polymer having two or more of the constitutional unit (C), such as that having a constitutional unit derived from methyl acrylate, a constitutional unit derived from ethyl acrylate and a constitutional unit derived from glycidyl methacrylate.

In the polymer (11), the number of the constitutional unit (A) is 0% to 99% and the total number of the constitutional unit (B) and the constitutional unit (C) is 1% to 100%, where the total number of the constitutional unit (A), the constitutional unit (B) and the constitutional unit (C) is 100%; and the number of the constitutional unit (B) is 1% to 100% and the number of the constitutional unit (C) is 0% to 99%, based on 100% of the total number of the constitutional unit (B) and the constitutional unit (C) .

Preferably, the polymer (11) may have constitutional units (A) and (B), and optionally, further a constitutional unit (C) wherein the number of the constitutional unit (A) is 70% to 99% and the total number of the constitutional unit (B) and the constitutional unit (C) is 1% to 30%, where the total number of the constitutional unit (A), the constitutional unit (B) and the constitutional unit (C) is 100%, and the number of the constitutional unit (B) is 1% to 100% and the number of the constitutional unit (C) is 0% to 99%, based on 100% of the total number of the constitutional unit (B) and the constitutional unit (C).

The number of the constitutional unit (A) in the polymer (11) is preferably 70% to 99%, more preferably 80% to 97.5%, and even more preferably 85% to 92.5%, where the total number of the constitutional unit (A), the constitutional unit (B) and the constitutional unit (C) is 100%, in view of shape retention of the resin product of the invention. The total number of the constitutional unit (B) and the constitutional unit (C) in the polymer (11) is preferably 1% to 30%, more preferably 2.5% to 20%, and even more preferably 7.5% to 15%, where the total number of the constitutional unit (A), the constitutional unit (B) and the constitutional unit (C) is 100%, in view of shape retention of the resin product of the invention.

The number of the constitutional unit (B) in the polymer (11) is 1% to 100%, preferably 60% to 100%, more preferably 80% to 100%, based on 100% of the total number of the constitutional unit (B) and the constitutional unit (C). The number of the constitutional unit (C) in the polymer (11) is 0% to 99%, preferably 0% to 40%, more preferably 0% to 20%, based on 100% of the total number of the constitutional unit (B) and the constitutional unit (C).

Preferably, the polymer (11) has a constitutional units (A) and (B), and optionally, further a constitutional unit (C) wherein the total number of the constitutional unit (A), the constitutional unit (B) and the constitutional unit (C) is 90% or more, where the total number of all constitutional units is 100%.

The number of the constitutional unit (A), the number of the constitutional unit (B), and the number of the constitutional unit (C) can be determined by well-known methods from the integral values of the signals of $^{13}C$ nuclear magnetic resonance spectrum (hereinafter referred to as $^{13}C$-NMR spectrum) or $^{1}H$ nuclear magnetic resonance spectrum (hereinafter referred to as $^{1}H$-NMR spectrum) assigned to each of the constitutional units.

If the polymer (11) is that prepared as described below by reacting a polymer having at least one constitutional unit (C) selected from the group consisting of constitutional units represented by formula (2) and constitutional units represented by formula (3) and optionally having a constitutional unit (A) derived from ethylene (hereinafter referred to as pre-polymer (1)) with at least one compound ($\alpha$) described below, the number of the constitutional unit (A), the number of the constitutional unit (B), and the number of the constitutional unit (C) are determined, for example, by the following method.

In case that the pre-polymer (1) comprises the constitutional unit (A) derived from ethylene, the numbers of the constitutional unit (A) and the constitutional unit (C) in the pre-polymer (1) are firstly determined. For calculation from a $^{13}C$-NMR spectrum, the numbers of diads (AA, AC, CC) of the constitutional units (A) and (C) are determined from the spectrum, and the numbers are substituted into the following equations to obtain the numbers of the constitutional unit (A) and the constitutional unit (C). Herein, AA is a unit (A)-(A) diad, AC is a unit (A)-(C) diad, and CC is a unit (C)-(C) diad.

$$\text{Number of unit (A)} = 100 - \text{Number of unit (C)}$$

$$\text{Number of unit (C)} = 100 \times (AC/2 + CC)/(AA + AC + CC)$$

As the constitutional unit (B) in the polymer (11) are formed by the reaction of the constitutional unit (C) contained in the pre-polymer (1) with the compound (α), the conversion of the constitutional unit (C) by the reaction is determined by the following method.

The integral value of a signal assigned to specified carbon contained in the side chains of the constitutional units (C) of the pre-polymer (1) (hereinafter, integral value Y) and the integral value of a signal assigned to specific carbon contained in the side chains of the constitutional units (B) of the polymer (11) (hereinafter, integral value Z) are substituted into the following equation to calculate the conversion.

$$\text{Conversion} = Z/(Y + Z)$$

Since the constitutional unit (A) contained in the pre-polymer (1) are not changed by the reaction of the pre-polymer (1) and the compound (α), the number of the constitutional unit (A) in the polymer (11) shall be equal to the number of the constitutional unit (A) in the pre-polymer (1). The number of the constitutional units (B) in the polymer (11) is determined as the product of the number of the constitutional unit (C) in the pre-polymer (1) and the conversion described above. The number of the constitutional unit (C) in the polymer (11) is determined as the difference between the number of the constitutional unit (C) in the pre-polymer (1) and the number of the constitutional unit (B) in the polymer (11).

Examples of the polymer (11) include polymers consisting of a constitutional unit (B), polymers consisting having constitutional units (B) and (A), polymers consisting having constitutional units (B) and (C), and polymers consisting having constitutional units (B), (A) and (C) .

Examples of the polymer consisting of a constitutional unit (B) include

polymers consisting of a constitutional unit (B) of the formula (1) wherein $L^1$, $L^2$ and $L^3$ are a single bond, and $L^6$ is an alkyl group having 14 to 30 carbon atoms, and

polymers consisting of a constitutional unit (B) of the formula (1) wherein $L^1$ is -CO-O-, $L^2$ and $L^3$ are a single bond, and $L^6$ is an alkyl group having 14 to 30 carbon atoms.

Examples of the polymer comprising constitutional units (B) and (A) include

polymers comprising a constitutional unit (B) of the formula (1) wherein $L^1$, $L^2$ and $L^3$ are a single bond, and $L^6$ is an alkyl group having 14 to 30 carbon atoms, and a constitutional unit (A), in which the total number of the units (A) and (B) is 90% or more, where the total number of all the constitutional units contained in the polymer is 100%, and

polymers comprising a constitutional unit (B) of the formula (1) wherein $L^1$ is -CO-O-, $L^2$ and $L^3$ are a single bond, and $L^6$ is an alkyl group having 14 to 30 carbon atoms, and a constitutional unit (A), in which the total number of the units (A) and (B) is 90% or more, where the total number of all the constitutional units in the polymer is 100%.

In another embodiment of the polymer comprising the constitutional units (B) and (A), the polymer may be an olefin polymer having a main chain comprising monomer units derived from ethylene and a branched chain having 5 or more carbon atoms wherein the number of the branched chain is 20 to 40 per 1000 carbon atoms in the olefin polymer. The number of the branched chain having 5 or more carbon atoms in the olefin polymer is preferably 25 to 35 per 1000 carbon atoms in the olefin polymer. The limiting viscosity [η] of the olefin polymer is preferably 1.0 to 5.0, in view of keeping strength and preventing impaired moldability. The olefin polymer is preferably a polymer comprising ethylene and α-olefin of 10 to 30 carbon atoms, more preferably comprising ethylene and α-olefin of 18 to 26 carbon atoms.

Such olefin polymer can be prepared according to the method as described in WO2015/156416.

In view of increasing the ΔH of the polymer (11), the polymer (11) is preferably a polymer wherein the number of the constitutional unit (B) is 50% to 80%, based on 100% of the total number of the constitutional units (B) and (A) in the polymer.

In view of molding processability, the polymer (11) is preferably a polymer wherein the number of the constitutional unit (B) is 10% to 50%, based on 100% of the total number of the constitutional units (B) and (A) in the polymer.

Preferred examples of the polymer comprising the constitutional units (B) and (C) include polymers comprising the constitutional unit (B) of the formula (1) wherein $L^1$ is -CO-O-, $L^2$ and $L^3$ are a single bond, and $L^6$ is an alkyl group having 14 to 30 carbon atoms, and the constitutional units (C) of the formula (3). In this case, the number of the constitutional unit (B) is preferably 80% or more, based on 100% of the total number of the constitutional units (B) and (C) in the polymer.

Preferably, the polymer (11) also comprises a constitutional unit (A) derived from ethylene so as to provide good shape retention and molding processability of the resin product of the invention at a temperature higher than the melting peak temperature of the polymer (11). More preferably, the constitutional unit (A) derived from ethylene form a branched structure in a polymer, and even more preferably, said branched structure is a long chain branched structure so that macromolecular chains can be entangled due to the branched structure, to provide good blow moldability and foamability.

The ratio A defined by the following equation (I) of the thermoplastic resin (2) or the polymer (11) is preferably 0.95 or less, more preferably 0.90 or less, and even more preferably 0.80 or less.

$$A = \alpha_1/\alpha_0 \qquad (I)$$

wherein $\alpha_1$ is a value obtained by a method comprising: measuring the absolute molecular weight and the intrinsic viscosity of the thermoplastic resin (2) or the polymer (11) by gel permeation chromatography using an apparatus equipped with a light scattering detector and a viscosity detector, plotting the measured data with respect to the logarithm of the absolute molecular weight (horizontal axis) and the logarithm of the intrinsic viscosity (vertical axis), approximating according to the equation (I-I) in the least squares sense the logarithm of the absolute molecular weight and the logarithm of the intrinsic viscosity within the range of from the logarithm of the weight-average molecular weight of the thermoplastic resin (2) or the polymer (11) to the logarithm of the z-average molecular weight of the thermoplastic resin (2) or the polymer (11), and defining the slope of the line of the equation (I-I) as $\alpha_1$

$$\log[\eta_1] = \alpha_1 \log M_1 + \log K_1 \qquad (I\text{-}I)$$

wherein $[\eta_1]$ represents the intrinsic viscosity (dl/g) of the thermoplastic resin (2) or the polymer (11), $M_1$ represents the absolute molecular weight of the thermoplastic resin (2) or the polymer (11), and $K_1$ is a constant.

In the equation (I), $\alpha_0$ is a value obtained by a method comprising: measuring the absolute molecular weight and the intrinsic viscosity of a polyethylene standard reference materials 1475a (available from National Institute of Standards and Technology) by gel permeation chromatography using an apparatus equipped with a light scattering detector and a viscosity detector, plotting the measured data with respect to the logarithm of the absolute molecular weight (horizontal axis) and the logarithm of the intrinsic viscosity (vertical axis), approximating according to the equation (I-II) in the least squares sense the logarithm of the absolute molecular weight and the logarithm of the intrinsic viscosity within the range of from the logarithm of the weight-average molecular weight of the polyethylene standard material 1475a to the logarithm of the z-average molecular weight of the polymer, and defining the slope of the line of the equation (I-II) as $\alpha_0$.

$$\log[\eta_0] = \alpha_0 \log M_0 + \log K_0 \qquad (I\text{-}II)$$

wherein $[\eta_0]$ represents the intrinsic viscosity (dl/g) of the polyethylene standard material 1475a, $M_0$ represents the absolute molecular weight of the polyethylene standard material 1475a, and $K_0$ is a constant.

In the measurement of the absolute molecular weight and the intrinsic viscosity of the polymer and the polyethylene standard material 1475a by gel permeation chromatography, the mobile phase is orthodichlorobenzene and the measurement temperature is 155°C.

For determination of the absolute molecular weight from the data obtained by a light scattering detector and determination of the intrinsic viscosity ($[\eta]$) by a viscosity detector, calculation is carried out using data-processing software OmniSEC (version 4.7) available from Malvern, with reference to "Size Exclusion Chromatography, Springer (1999)".

The above-mentioned polyethylene standard material 1475a (available from National Institute of Standards and Technology) is a high-density polyethylene having no branching. The equations (I-I) and (I-II) are referred to as Mark-Hauwink-Sakurada equations, which represent the relationship between the intrinsic viscosity and the molecular weight of polymer; and the smaller the $\alpha_1$, the larger the number of macromolecular chain entanglements due to a branching structure. Since the polyethylene standard material 1475a form no branching structure, no macromolecular chain entanglements due to branching structure are formed. The smaller the A ratio, which is a ratio of $\alpha_1$ to $\alpha_0$ of the polyethylene standard material 1475a, the larger the amount of long chain branching structure is in the thermoplastic resin (2) or the polymer (11). When the thermoplastic resin (2) is a polymer (11) comprising constitutional units (A) derived from ethylene, the smaller the A value, the larger the amount of long chain branching structure formed by the units (A) in the polymer (11).

The average molecular weight of the thermoplastic resin (2) or the polymer (11) is preferably 10,000 to 1,000,000, more preferably 50,000 to 750,000, and even more preferably 100,000 to 500,000, as measured by gel permeation chromatography using an apparatus equipped with a light scattering detector. In the measurement of the average molecular weight of the thermoplastic resin (2) or the polymer (11) by gel permeation chromatography, the mobile phase is orthodichlorobenzene and the measurement temperature is 155°C.

From the viewpoint of more reducing the extrusion load at the time of molding, the activation energy of flow ($E_a$) of the thermoplastic resin (2) or the polymer (11) is preferably 40 kJ/mol or more, more preferably 50 kJ/mol or more, and even more preferably 60 kJ/mol or more. For obtaining the resin product by extrusion molding, the activation energy of flow ($E_a$) of the thermoplastic resin (2) or the polymer (11) is preferably 100 kJ/mol or less, more preferably 90 kJ/mol or less, and even more preferably 80 kJ/mol or less so as to obtain a molded article produced by extrusion having good appearance. The $E_a$ primarily depends on the number of long chain branching in a polymer. The larger the number of long chain branching is in a polymer, the greater the $E_a$ is.

The activation energy of flow ($E_a$) is determined by the method described below. First, melt complex viscosity-angular frequency curves of a polymer are obtained by measuring at three or more temperatures including 170°C (T, unit: °C) selected from 90°C, 110°C, 130°C, 150°C and 170°C. Said melt complex viscosity-angular frequency curve is a log-log curve in which the melt complex viscosity (unit: Pa•sec) (horizontal axis) and the angular frequency (unit: rad/sec) (vertical axis) are plotted. Then, for each of the melt complex viscosity-angular frequency curves measured at each temperature other than 170°C, the angular frequency is multiplied by $a_T$ and the melt complex viscosity is multiplied by $1/a_T$ such that the curve superposes the melt complex viscosity-angular frequency curve at 170°C. The $a_T$ value is appropriately determined so that the melt complex viscosity-angular frequency curves at each temperature other than 170°C superpose the melt complex viscosity-angular frequency curve at 170°C.

The $a_T$ is generally called shift factor, and the value varies depending on the measurement temperature of the melt complex viscosity-angular frequency curve.

Then, at each temperature (T), [$\ln(a_T)$] and [1/(T + 273.16)] are determined and [$\ln(a_T)$] and [1/(T + 273.16)] are approximated in the least squares sense according to the following equation (II), and then the slope m of the equation (II) is determined. The m is substituted into the following equation (III) to calculate $E_a$.

$$\ln(a_T) = m(1/(T + 273.16)) + n \quad \text{(II)}$$

$$E_a = |0.008314 \times m| \quad \text{(III)}$$

$a_T$: shift factor
$E_a$ : activation energy of flow (unit: kJ/mol)
T: temperature (unit: °C)

The above calculation may be conducted using a commercially available calculation software, examples of which include TA instruments Ochestrator software.

The method described above is based on the following principle.

It is known that melt complex viscosity-angular frequency curves (log-log curves) measured at different temperatures are superposed on one parent curve (referred to as "master curve") by horizontally moving the curves at the individual temperatures by prescribed distances, and this is called "principal of temperature-time superposition". The horizontal movement distance is called "shift factor," which is a value that depends on temperature. The temperature dependency of the shift factor is known to be represented by the above equations (II) and (III), which are called "Arrhenius-type equations".

The correlation factor when approximating [$\ln(a_T)$] and [1/(T + 273.16)] in the least squares sense according to the equation (II) is adjusted to 0.9 or more.

The measurement of the melt complex viscosity-angular frequency curve as mentioned is performed using a viscoelasticity measuring apparatus (e.g. ARES, manufactured by TA instruments), usually, under the conditions including: geometry: parallel plates, plate diameter: 25 mm, plate interval: 1.2 to 2 mm, strain: 5%, and angular frequency: 0.1 to 100 rad/sec. The measurement is performed under a nitrogen atmosphere. It is preferable to add previously an appropriate amount (e.g., 1,000 ppm by weight) of an antioxidant in a measurement sample.

The extensional viscosity nonlinear index k, which indicates a degree of strain hardening of the thermoplastic resin (2) or the polymer (11) is preferably 0.85 or more, more preferably 0.90 or more, and even more preferably 0.95 or more, in view of superior moldability, such as causing small neck-in during T-die film processing, making a resulting film small in thickness variation, and being less prone to foam breakage during foam molding. Strain hardening of a polymer means that the extensional viscosity of the polymer increases sharply at above a certain amount of strain when a strain is applied to the polymer. In view of ease of molding the resin product of the present invention in a desired shape, the index k is preferably 2.00 or less, more preferably 1.50 or less, even more preferably 1.40 or less, further preferably 1.30 or less, and particularly preferably 1.20 or less.

The extensional viscosity nonlinear index k can be determined by the following method.

The viscosity $\eta_E1(t)$ at extension time t when the polymer is uniaxially stretched at a temperature of 110°C and a strain rate of 1 $sec^{-1}$ and the viscosity $\eta_E0.1(t)$ at an extension time t when the polymer is uniaxially stretched at a temperature of 110°C and a strain rate of 0.1 $sec^{-1}$ are determined. The $\eta_E1$ (t) and the $\eta_E0.1$ (t) determined at the same extension time t are substituted into the following equation to calculate $\alpha(t)$.

$$\alpha(t) = \eta_E1(t)/\eta_E0.1(t)$$

The logarithm of $\alpha(t)$, $\ln(\alpha(t))$, is plotted versus the extension time t, and $\ln(\alpha(t))$ and t are approximated in the least squares sense according to the following equation within a range of t of from 2.0 sec to 2.5 sec. The value k is the slope of line of the equation.

$$\ln(\alpha(t)) = kt$$

The value k, where the correlation function r2 used for the approximation in the least squares sense in the above equation is 0.9 or more, is applied.

The measurement of viscosity during the uniaxial stretching is carried out under a nitrogen atmosphere using a viscoelasticity measuring apparatus (e.g., ARES, manufactured by TA instruments) .

In the extensional viscosity measurement, a polymer having long chain branchings has a property that its extensional viscosity rises sharply to deviate from the linear region at a high strain region, namely, a strain hardening property. In the case of a polymer having a strain hardening property, it is known that the logarithm of $\alpha(t)$, $\ln(\alpha(t))$, increases in proportion to $\ln(l/l_0)$, wherein $l_0$ and 1 are the lengths of a sample at extension times 0 and t, respectively [reference: Kiyohito Koyama, Osamu Ishizuka, Journal of Fiber Science and Technology, 37, T-258 (1981)]. In the case of a polymer having no strain hardening property, $\alpha(t)$ is 1 at any extension time, and the slope k of the line produced by plotting the logarithm of $\alpha(t)$, $\ln(\alpha(t))$, versus the extension time is 0. In the case of a polymer having a strain hardening property, the slope k of the line plot is not 0 especially in a high strain region. In the present invention, the slope k of the line by plotting the logarithm of a nonlinear parameter $\alpha(t)$, $\ln(\alpha(t))$, versus the extension time is defined as a parameter that indicates a degree of strain hardening property.

Examples of methods for producing the polymer (11) include a method comprising reacting the pre-polymer (1) with at least one compound selected from the group consisting of alcohols having an alkyl group of 14 to 30 carbon atoms, amines having an alkyl group of 14 to 30 carbon atoms, alkyl halides having an alkyl group of 14 to 30 carbon atoms, carboxylic acids having an alkyl group of 14 to 30 carbon atoms, carboxylic acid amides having an alkyl group of 14 to 30 carbon atoms, carboxylic acid halides having an alkyl group of 14 to 30 carbon atoms, carbamic acids having an alkyl group of 14 to 30 carbon atoms, alkylureas having an alkyl group of 14 to 30 carbon atoms, and isocyanates having an alkyl group of 14 to 30 carbon atoms (hereinafter referred to as "compound $\alpha$"), and a method comprising polymerizing a monomer that serves as a raw material of the constitutional unit (B) or copolymerizing ethylene with the monomer that serves as a raw material of the constitutional unit (B).

The alkyl group of the compound $\alpha$ may be a straight chain alkyl group or a branched chain alkyl group and preferably is a straight chain alkyl group.

The pre-polymer (1) is a raw material for producing the polymer (11), and the pre-polymer (1) does not contain a constitutional unit (B) of the formula (1). The pre-polymer (1) may contain a constitutional unit which is none of the constitutional unit (A), (B) and (C).

In the pre-polymer (1), preferably, the number of the constitutional unit (A) is 0% to 99% and the number of the constitutional unit (C) is 1% to 100%, based on 100% of the total number of the constitutional unit (A) and the constitutional unit (C). More preferably, the number of the constitutional unit (A) is 70% to 99% and the number of the constitutional unit (C) is 1% to 30%.

Examples of methods to form a constitutional unit (B) in the polymer (11) include a method comprising reacting the constitutional unit (C) contained in the pre-polymer (1) with the compound ($\alpha$) as mentioned and a method comprising polymerizing a monomer that serves as a raw material of the constitutional unit (B) or copolymerizing ethylene with the monomer that serves as a raw material of the constitutional unit (B). The alkyl group of the compound ($\alpha$) preferably is a straight chain alkyl group.

Examples of the pre-polymer (1) include acrylic acid polymer, methacrylic acid polymer, vinyl alcohol polymer, methyl acrylate polymer, ethyl acrylate polymer, n-propyl acrylate polymer, n-butyl acrylate polymer, methyl methacrylate polymer, ethyl methacrylate polymer, n-propyl methacrylate polymer, n-butyl methacrylate polymer, vinyl formate polymer, vinyl acetate polymer, vinyl propionate polymer, vinyl(n-butylate) polymer, methyl vinyl ether polymer, ethyl vinyl ether polymer, n-propyl vinyl ether polymer, n-butyl vinyl ether polymer, maleic anhydride polymer, glycidyl acrylate

polymer, glycidyl methacrylate polymer, 3- (dimethylamino) propyl acrylate polymer, 3-(dimethylamino)propyl methacrylate polymer, ethylene-acrylic acid copolymer, ethylene-methacrylic acid copolymer, ethylene-vinyl alcohol copolymer, ethylene-methyl acrylate copolymer, ethylene-ethyl acrylate copolymer, ethylene-n-propyl acrylate copolymer, ethylene-n-butyl acrylate copolymer, ethylene-methyl methacrylate copolymer, ethylene-ethyl methacrylate copolymer, ethylene-n-propyl methacrylate copolymer, ethylene-n-butyl methacrylate copolymer, ethylene-vinyl formate copolymer, ethylene-vinyl acetate copolymer, ethylene-vinyl propionate copolymer, ethylene-vinyl(n-butylate) copolymer, ethylene-methyl vinyl ether copolymer, ethylene-ethyl vinyl ether copolymer, ethylene-n-propyl vinyl ether copolymer, ethylene-n-butyl vinyl ether copolymer, ethylene-maleic anhydride copolymer, ethylene-glycidyl acrylate copolymer, ethylene-glycidyl methacrylate copolymer, ethylene-3-(dimethylamino) propyl acrylate copolymer, and ethylene-3-(dimethylamino) propyl methacrylate copolymer.

Examples of alcohols having a straight chain alkyl group of 14 to 30 carbon atoms include n-tetradecyl alcohol, n-pentadecyl alcohol, n-hexadecyl alcohol, n-heptadecyl alcohol, n-octadecyl alcohol, n-nonadecyl alcohol, n-eicosyl alcohol, n-heneicosyl alcohol, n-docosyl alcohol, n-tricosyl alcohol, n-tetracosyl alcohol, n-pentacosyl alcohol, n-hexacosyl alcohol, n-heptacosyl alcohol, n-octacosyl alcohol, n-nonacosyl alcohol, and n-triacontyl alcohol.

Examples of alcohols having a branched chain alkyl group of 14 to 30 carbon atoms include isotetradecyl alcohol, isopentadecyl alcohol, isohexadecyl alcohol, isoheptadecyl alcohol, isooctadecyl alcohol, isononadecyl alcohol, isoeicosyl alcohol, isoheneicosyl alcohol, isodocosyl alcohol, isotricosyl alcohol, isotetracosyl alcohol, isopentacosyl alcohol, isohexacosyl alcohol, isoheptacosyl alcohol, isooctacosyl alcohol, and isononacosyl alcohol, and isotriacontyl alcohol.

Examples of amines having a straight chain alkyl group of 14 to 30 carbon atoms include n-tetradecylamine, n-pentadecylamine, n-hexadecylamine, n-heptadecylamine, n-octadecylamine, n-nonadecylamine, n-eicosylamine, n-heneicosylamine, n-docosylamine, n-tricosylamine, n-tetracosylamine, n-pentacosylamine, n-hexacosylamine, n-heptacosylamine, n-octacosylamine, n-nonacosylamine, and n-triacontylamine.

Examples of amines having a branched chain alkyl group of 14 to 30 carbon atoms include isotetradecylamine, isopentadecylamine, isohexadecylamine, isoheptadecylamine, isooctadecylamine, isononadecylamine, isoeicosylamine, isoheneicosylamine, isodocosylamine, isotricosylamine, isotetracosylamine, isopentacosylamine, isohexacosylamine, isoheptacosylamine, isooctacosylamine, isononacosylamine, and isotriacontylamine.

Examples of alkyl halide having a straight chain alkyl group of 14 to 30 carbon atoms include n-tetradecyl iodide, n-pentadecyl iodide, n-hexadecyl iodide, n-heptadecyl iodide, n-octadecyl iodide n-nonadecyl iodide, n-eicosyl iodide, n-heneicosyl iodide, n-docosyl iodide, n-tricosyl iodide, n-tetracosyl iodide, n-pentacosyl iodide, n-hexacosyl iodide, n-heptacosyl iodide, n-octacosyl iodide, n-nonacosyl iodide, and n-triacontyl iodide.

Examples of alkyl halide having a branched chain alkyl group of 14 to 30 carbon atoms include isotetradecyl iodide, isopentadecyl iodide, isohexadecyl iodide, isoheptadecyl iodide, isooctadecyl iodide, isononadecyl iodide, isoeicosyl iodide, isoheneicosyl iodide, isodocosyl iodide, isotricosyl iodide, isotetracosyl iodide, isopentacosyl iodide, isohexacosyl iodide, isoheptacosyl iodide, isooctacosyl iodide, isononacosyl iodide, and isotriacontyl iodide.

Examples of carboxylic acids having a straight chain alkyl group of 14 to 30 carbon atoms include n-tetradecanoic acid, n-pentadecanoic acid, n-hexadecanoic acid, n-heptadecanoic acid, n-octadecanoic acid, n-nonadecanoic acid, n-eicosanoic acid, n-heneicosanoic acid, n-docosanoic acid, n-tricosanoic acid, n-tetracosanoic acid, n-pentacosanoic acid, n-hexacosanoic acid, n-heptacosanoic acid, n-octacosanoic acid, n-nonacosanoic acid, and n-triacontanoic acid.

Examples of carboxylic acids having a branched chain alkyl group of 14 to 30 carbon atoms include isotetradecanoic acid, isopentadecanoic acid, isohexadecanoic acid, isoheptadecanoic acid, isooctadecanoic acid, isononadecanoic acid, isoeicosanoic acid, isoheneicosanoic acid, isodocosanoic acid, isotricosanoic acid, isotetracosanoic acid, isopentacosanoic acid, isohexacosanoic acid, isoheptacosanoic acid, isooctacosanoic acid, isononacosanoic acid, and isotriacontanoic acid.

Examples of carboxylic acid amides having a straight chain alkyl group of 14 to 30 carbon atoms include n-tetradecanoic acid amide, n-pentadecanoic acid amide, n-hexadecanoic acid amide, n-heptadecanoic acid amide, n-octadecanoic acid amide, n-nonadecanoic acid amide, n-eicosanoic acid amide, n-heneicosanoic acid amide, n-docosanoic acid amide, n-tricosanoic acid amide, n-tetracosanoic acid amide, n-pentacosanoic acid amide, n-hexacosanoic acid amide, n-heptacosanoic acid amide, n-octacosanoic acid amide, n-nonacosanoic acid amide and n-triacontanoic acid amide.

Examples of carboxylic acid amides having a branched chain alkyl group of 14 to 30 carbon atoms include isotetradecanoic acid amide, isopentadecanoic acid amide, isohexadecanoic acid amide, isoheptadecanoic acid amide, isooctadecanoic acid amide, isononadecanoic acid amide, isoeicosanoic acid amide, isoheneicosanoic acid amide, isodocosanoic acid amide, isotricosanoic acid amide, isotetracosanoic acid amide, isopentacosanoic acid amide, isohexacosanoic acid amide, isoheptacosanoic acid amide, isooctacosanoic acid amide, isononacosanoic acid amide and isotriacontanoic acid amide.

Examples of carboxylic acid halides having a straight chain alkyl group of 14 to 30 carbon atoms include n-tetradecanoyl chloride, n-pentadecanoyl chloride, n-hexadecanoyl chloride, n-heptadecanoyl chloride, n-octadecanoyl

chloride, n-nonadecanoyl chloride, n-eicosanoyl chloride, n-heneicosanoyl chloride, n-docosanoyl chloride, n-tricosanoyl chloride, n-tetracosanoyl chloride, n-pentacosanoyl chloride, n-hexacosanoyl chloride, n-heptacosanoyl chloride, n-octacosanoyl chloride, n-nonacosanoyl chloride, and n-triacontanoyl chloride.

Examples of carboxylic acid halides having a branched chain alkyl group of 14 to 30 carbon atoms include isotetradecanoyl chloride, isopentadecanoyl chloride, isohexadecanoyl chloride, isoheptadecanoyl chloride, isooctadecanoyl chloride, isononadecanoyl chloride, isoeicosanoyl chloride, isoheneicosanoyl chloride, isodocosanoyl chloride, isotricosanoyl chloride, isotetracosanoyl chloride, isopentacosanoyl chloride, isohexacosanoyl chloride, isoheptacosanoyl chloride, isooctacosanoyl chloride, isononacosanoyl chloride and isotriacontanoyl chloride.

Examples of carbamic acids having a straight chain alkyl group of 14 to 30 carbon atoms include n-tetradecylcarbamic acid, n-pentadecylcarbamic acid, n-hexadecylcarbamic acid, n-heptadecylcarbamic acid, n-octadecylcarbamic acid, n-nonadecylcarbamic acid, n-eicosylcarbamic acid, n-heneicosylcarbamic acid, n-docosylcarbamic acid, n-tricosylcarbamic acid, n-tetracosylcarbamic acid, n-pentacosylcarbamic acid, n-hexacosylcarbamic acid, n-heptacosylcarbamic acid, n-octacosylcarbamic acid, n-nonacosylcarbamic acid, and n-triacontylcarbamic acid.

Examples of carbamic acid having a branched chain alkyl group of 14 to 30 carbon atoms include isotetradecylcarbamic acid, isopentadecylcarbamic acid, isohexadecylcarbamic acid, isoheptadecylcarbamic acid, isooctadecylcarbamic acid, isononadecylcarbamic acid, isoeicosylcarbamic acid, isoheneicosylcarbamic acid, isodocosylcarbamic acid, isotricosylcarbamic acid, isotetracosylcarbamic acid, isopentacosylcarbamic acid, isohexacosylcarbamic acid, isoheptacosylcarbamic acid, isooctacosylcarbamic acid, isononacosylcarbamic acid and isotriacontylcarbamic acid.

Examples of alkylurea having a straight chain alkyl group of 14 to 30 carbon atoms include n-tetradecylurea, n-pentadecylurea, n-hexadecylurea, n-heptadecylurea, n-octadecylurea, n-nonadecylurea, n-eicosylurea, n-heneicosylurea, n-docosylurea, n-tricosylurea, n-tetracosylurea, n-pentacosylurea, n-hexacosylurea, n-heptacosylurea, n-octacosylurea, n-nonacosylurea and n-triacontylurea.

Examples of alkylurea having a branched chain alkyl group of 14 to 30 carbon atoms include isotetradecylurea, isopentadecylurea, isohexadecylurea, isoheptadecylurea, isooctadecylurea, isononadecylurea, isoeicosylurea, isoheneicosylurea, isodocosylurea, isotricosylurea, isotetracosylurea, isopentacosylurea, isohexacosylurea, isoheptacosylurea, isooctacosylurea, isononacosylurea, and isotriacontylurea.

Examples of isocyanate having a straight chain alkyl group of 14 to 30 carbon atoms include n-tetradecyl isocyanate, n-pentadecyl isocyanate, n-hexadecyl isocyanate, n-heptadecyl isocyanate, n-octadecyl isocyanate, n-nonadecyl isocyanate, n-eicosyl isocyanate, n-heneicosyl isocyanate, n-docosyl isocyanate, n-tricosyl isocyanate, n-tetracosyl isocyanate, n-pentacosyl isocyanate, n-hexacosyl isocyanate, n-heptacosyl isocyanate, n-octacosyl isocyanate, n-nonacosyl isocyanate and n-triacontyl isocyanate.

Examples of isocyanate having a branched chain alkyl group of 14 to 30 carbon atoms include isotetradecyl isocyanate, isopentadecyl isocyanate, isohexadecyl isocyanate, isoheptadecyl isocyanate, isooctadecyl isocyanate, isononadecyl isocyanate, isoeicosyl isocyanate, isoheneicosyl isocyanate, isodocosyl isocyanate, isotricosyl isocyanate, isotetracosyl isocyanate, isopentacosyl isocyanate, isohexacosyl isocyanate, isoheptacosyl isocyanate, isooctacosyl isocyanate, isononacosyl isocyanate and isotriacontyl isocyanate.

In case that the pre-polymer (1) comprises a constitutional unit (A) derived from ethylene, the product of the reactivity ratios r1r2 is preferably 0.5 to 5.0, and more preferably 0.5 to 3.0, where r1 is the reactivity ratio of ethylene used as a raw material in the production of the pre-polymer (1) and r2 is the reactivity ratio of the monomer to form the constitutional unit (C) .

The reactivity ratio r1 of ethylene is a value defined by the equation: r1 = k11/k12 wherein k11 is the reaction velocity of ethylene to bond to a polymer terminated by the constitutional unit (A) and k12 is the reaction velocity of the monomer of the constitutional unit (C) to bond to a polymer terminated by the constitutional unit (A), during copolymerizing ethylene with the monomer of the constitutional unit (C). The reactivity ratio r1 is an index indicating which of ethylene or the monomer of the constitutional unit (C) is more prone to react with the constitutional unit (A) during the copolymerization of ethylene with the monomer of the constitutional unit (C). The grater the value of r1, the more easily the polymer terminated by the constitutional unit (A) reacts with ethylene, and the more easily the sequence of the constitutional unit (A) is formed.

The reactivity ratio r2 of the monomer of the constitutional unit (C) is a value defined by the equation: r2 = k21/k22 wherein k21 is the reaction velocity of ethylene to bond to a polymer terminated by the constitutional unit (C) and k22 is the reaction velocity of the monomer of the constitutional unit (C) to bond to a polymer terminated by the constitutional unit (C), during copolymerizing ethylene with the monomer of the constitutional unit (C) . The reactivity ratio r2 is an index indicating which of ethylene or the monomer of the constitutional unit (C) is more prone to react with the polymer terminated by the constitutional unit (C) during the copolymerization of ethylene with the monomer of the constitutional unit (C). The grater the value of r2, the more easily the polymer terminated by the constitutional unit (C) reacts with the monomer of the constitutional unit (C), and the more easily the sequence of the constitutional unit (C) is formed.

The product of the reactivity ratios rlr2 is calculated by the method described in the reference "Kakugo, M.;

Naito, Y.; Mizunuma, K.; Miyatake, T., Macromolecules, 1982, 15, 1150". In the present invention, rlr2 is determined by substituting the numbers of AA, AC and CC, which are diads of the constitutional unit (A) and the constitutional unit (C), calculated from $^{13}$C nuclear magnetic resonance spectrum of the pre-polymer (1) into the following equation:

$$r1r2 = AA[CC/(AC/2)^2]$$

The product of the reactivity ratios rlr2 is an index indicating monomer sequence distribution in a copolymer. The closer the value of r1r2 to 1, the higher the degree of randomness of the monomer sequence distribution in the copolymer; the closer the value of rlr2 to 0, the higher the degree of alternate copolymerization of the monomer sequence distribution in the copolymer; and the larger the value of rlr2 than 1, the higher the degree of block copolymerization of the monomer sequence distribution in the copolymer.

The melt flow rate of the pre-polymer (1) measured at a temperature of 190°C under a load of 21 N in accordance with JIS K7210 is preferably 0.1 g/10 min to 500 g/10 min.

Examples of methods for producing the pre-polymer (1) include a coordination polymerization method, a cationic polymerization method, an anionic polymerization method and a radical polymerization method, and preferably a radical polymerization method under high pressure.

The reaction temperature when reacting the pre-polymer (1) with at least one compound (α) is usually 40°C to 250°C. The reaction may be carried out in the presence of a solvent, and examples of the solvent include hexane, heptane, octane, nonane, decane, toluene and xylene. In case that a by-product is generated in the reaction, the reaction may be carried out while distilling the by-product under reduced pressure, in order to promote the reaction. The reaction may be carried out while azeotropically distilling the by-product with the solvent, cooling the vaporized by-product and solvent, separating resulted distillate containing the by-product and the solvent into a by-product layer and a solvent layer, and returning only the corrected solvent to the reaction system as a refluxing liquid.

The reaction of the pre-polymer (1) with the at least one compound (α) may be carried out with while melt-kneading the pre-polymer (1) and the compound (α). In case that a by-product is generated in the reaction of the pre-polymer (1) with the compound (α) during the melt-kneading, the reaction may be carried out while distilling the by-product under reduced pressure, in order to promote the reaction. Examples of melt-kneading apparatus for used in the melt-kneading include known apparatuses, such as single screw extruder, twin screw extruder, Banbury mixer, and the like. The temperature of the melt-kneading apparatus is preferably 100°C to 250°C.

For the reaction of the pre-polymer (1) with at least one compound (α), a catalyst may be added to promote the reaction. Examples of the catalyst include alkali metal salts and Group 4 metal complexes. Examples of the alkali metal salts include alkali metal hydroxides such as lithium hydroxide, sodium hydroxide and potassium hydroxide, and alkali metal alkoxides such as lithium methoxide and sodium methoxide. Examples of the Group 4 metal complexes include tetra(isopropyl) orthotitanate, tetra(n-butyl) orthotitanate and tetraoctadecyl orthotitanate. The amount of the catalyst is preferably 0.01 parts by weight to 50 parts by weight, and more preferably 0.01 parts by weight to 5 parts by weight, relative to the total amount of 100 parts by weight of the pre-polymer (1) and the at least one compound (α) to be used for the reaction.

The polymer (11) may form a mixture with an unreacted compound (α) or a catalyst added for promoting the reaction. The amount of the unreacted compound (α) in the mixture is preferably less than 3 parts by weight, relative to 100 parts by weight of the polymer (11).

The polymer (11) may be a crosslinked polymer or an uncrosslinked polymer.

In one embodiment, the polymer (11) is an uncrosslinked polymer (hereinafter referred to as polymer (α)).

The polymer (α) has a gel fraction, as described below, of less than 20% by weight.

In the polymer (α), the total number of the constitutional units (A), (B) and (C) is preferably 90% or more, more preferably 95% or more, and even more preferably 100%, based on 100% of the total number of all constitutional units contained in the polymer.

<Crosslinked polymer>

In one embodiment, the polymer (11) is crosslinked. That is, at least a part of the polymer (11) molecule is connected by a covalent bond between molecules. The term "the polymer (11) is crosslinked" means that the polymers (11) are linked intermolecularly by a covalent bond and/or that the polymer (11) and a different polymer (as described below) are linked intermolecularly by a covalent bond.

Examples of a method for crosslinking the polymer include a method using ionizing radiation and a method using an organic peroxide.

For crosslinking a polymer by ionizing radiation, ionizing radiation is applied to a polymer (α) previously molded

into a desired shape. For molding, known methods may be used, and extrusion forming, injection molding and press molding are preferred. The molded article to be irradiated with ionizing radiation may be either a molded article comprising only polymer (α) as a polymer component or a molded article of resin composition comprising a polymer (α) and a polymer different from the polymer (α). In the latter case, examples of the polymer different from polymer (α) include a thermoplastic resin (3) described below. In case that the molded article comprises the polymer (α) and the thermoplastic resin (3), the amount of the polymer (α) is preferably 1% by weight to 99% by weight, more preferably 5% by weight to 95% by weight, even more preferably 10% by weight to 90% by weight, and further preferably 15% by weight to 85% by weight, based on 100% by weight of the total amount of the polymer (α) and the thermoplastic resin (3).

Examples of the ionizing radiation include α rays, β rays, γ rays, electron rays, neutrons, and X-rays, and γ rays of cobalt-60 or electron rays are preferable. In case that the molded article comprising a polymer is in a sheet form, the ionizing radiation may be applied on at least one side of the sheet of the molded article.

The irradiation of ionizing radiation is carried out using a known ionizing radiation irradiation apparatus, and the dose of irradiation is usually 5 to 300 kGy, and preferably 10 to 150 kGy.

For obtaining a crosslinked polymer by irradiation with ionizing radiation, a polymer crosslinked with a higher degree of crosslinking can be obtained by incorporating a crosslinking aid in a molded article to be irradiated with ionizing radiation. The crosslinking aid is an agent for increasing the degree of crosslinking of a polymer and improving the mechanical property of the polymer, and a compound having a plurality of double bonds in its single molecule is preferably used. Examples of the crosslinking aid include N,N'-m-phenylenebismaleimide, toluylene bismaleimide, triallyl isocyanurate, triallyl cyanurate, p-quinonedioxime, nitrobenzene, diphenylguanidine, divinylbenzene, ethylene glycol dimethacrylate, polyethylene glycol dimethacrylate, trimethylolpropane trimethacrylate, trimethylolpropane triacrylate, and allyl methacrylate.

The crosslinking aids may be used in combination.

The amount of the crosslinking aid is preferably 0.01 to 4.0 parts by weight, and more preferably 0.05 to 2.0 parts by weight, where the total weight of the polymer (α) and the polymer different from the polymer (α) in the molded article to be irradiated with ionizing radiation is 100 parts by weight.

Examples of a method of crosslinking using an organic peroxide include a method of crosslinking a composition comprising the polymer (α) and the organic peroxide by a known molding method by heating to crosslink the polymer (α). Examples of such known molding method by heating include extrusion molding, injection molding and press molding. The resin composition comprising a polymer (α) and an organic peroxide may contain only the polymer (α) as a resin component or may contain the polymer (α) and a polymer different from the polymer (α).

In case that the resin composition comprising a polymer (α) and an organic peroxide contains a polymer different from the polymer (α), examples of the different polymer include a thermoplastic resin (3) described below, and the amount of the polymer (α) is preferably 1% by weight to 99% by weight, more preferably 5% by weight to 95% by weight, even more preferably 10% by weight to 90% by weight, and further preferably 15% by weight to 85% by weight, based on 100% by weight of the total amount of the polymer (α) and the thermoplastic resin (3).

For crosslinking using an organic peroxide, it is preferred to use an organic peroxide having a decomposition temperature equal to or higher than the flow onset temperatures of the resin component contained in the composition comprising a polymer (α) and an organic peroxide. Examples of preferred organic peroxide include dicumylperoxide,
2,5-dimethyl-2,5-di-tert-butylperoxyhexane,
2,5-dimethyl-2,5-di-tert-butylperoxyhexyne,
α,α-di-tert-butylperoxyisopropylbenzene,
tert-butylperoxy-2-ethylhexyl carbonate, and the like.

If necessary, known additives may be added and kneaded with a polymer (α) before crosslinking. Examples of such additive include flame retardants, antioxidants, weathering agents, lubricants, antiblocking agents, antistatic agents, anticlouding agents, antidripping agents, pigments, and fillers.

The crosslinked polymer (11) preferably has a gel fraction of 20% by weight or more, preferably 40% by weight or more, more preferably 60% by weight or more and most preferably 70% by weight or more, based on 100% by weight of the weight of the crosslinked polymer (11). The gel fraction indicates a degree of crosslinking of a crosslinked polymer, and a higher degree of the gel fraction of the polymer means that the polymer has a larger amount of crosslinked structure and forms a stronger network structure. The higher the gel fraction of the polymer, the polymer is superior in shape retention and less prone to deform.

The gel fraction of the polymer (11) can be determined by the method described below. Approximately 500 mg of the polymer and an empty wire net basket (opening: 400 meshes) are weighed. The net basket loaded with the polymer and 50 mL of xylene (Kanto Chemical Co., Inc., Cica Special Grade or equivalent; a mixture of o-, m- and p-xylene and ethylbenzene; the total content of o-, m- and p-xylene is 85% by weight or more) are introduced into a 100-mL test tube and extracted with heating at 110°C for 6 hours. After the extraction, the net basket containing extraction residue was removed from the test tube and dried under reduced pressure at 80°C for 8 hours in a vacuum dryer, and the net basket containing the extraction residue thus dried is weighed. The weight of gel is determined from the difference of the weight

of the net basket containing extraction residue after drying and the weight of the empty net basket. The gel fraction (% by weight) is calculated according to the following equation.

$$\text{Gel fraction} = \text{weight of gel} / \text{weight of sample} \times 100$$

The resin product of the invention preferably has a gel fraction of 20% by weight or more, more preferably 40% by weight or more, even more preferably 60% by weight or more, and most preferably 70% by weight or more, based on 100% by weight of the weight of the resin product.

The resin product having a gel fraction of 20% by weight or more may be obtained by a method, such as
a method using the crosslinked polymer (11) described above as a raw material for the resin product,
a method by irradiating a molded article of resin composition comprising a polymer ($\alpha$) and a thermoplastic resin (3) with ionizing radiation to form crosslinking, and
a method by heating a resin composition comprising a polymer ($\alpha$), a thermoplastic resin (3) and an organic peroxide to form crosslinking.

The gel fraction of the resin product can be determined by the method described below. Approximately 500 mg of the resin product and an empty wire net basket (opening: 400 meshes) are weighed. The net basket loaded with the resin product and 50 mL of xylene (Kanto Chemical Co., Inc., Cica Special Grade or equivalent; a mixture of o-, m- and p-xylene and ethylbenzene; the total content of o-, m- and p-xylene is 85% by weight or more) are introduced into a 100-mL test tube and extracted with heating at 110°C for 6 hours. After the extraction, the net basket containing extraction residue removed from the test tube and dried under reduced pressure at 80°C for 8 hours in a vacuum dryer, and the net basket containing the extraction residue thus dried is weighed. The weight of gel is determined from the difference of the weight of the net basket containing extraction residue after drying and the weight of the empty net basket. The gel fraction (% by weight) of the resin product is calculated according to the following equation.

$$\text{Gel fraction of the resin product} = \text{weight of gel in the resin product} / \text{weight of sample (resin product)} \times 100$$

<Thermoplastic resin (3)>

The thermoplastic resin (3) contained in the resin product of the invention is a thermoplastic resin having a storage elastic modulus E' at 60°C of $5.0 \times 10^5$ Pa or more, as determined by dynamic viscoelasticity measurement at a frequency of 10 Hz. The storage elastic modulus E' at 60°C of the thermoplastic resin (3) is preferably $1.0 \times 10^6$ Pa or more.

Examples of the thermoplastic resin (3) include polyethylene, polypropylene, polyvinyl chloride, polystyrene, acrylonitrile/butadiene/styrene copolymer, acrylonitrile/styrene copolymer, polymethylmethacrylate, polyvinyl alcohol, polyvinylidene chloride, polyethylene terephthalate, polyamide, polyacetal, polycarbonate, polyphenylene ether, polybutylene terephthalate, polyvinylidene fluoride, polysulfone, polyethersulfone, polyphenylene sulfide, polyarylate, polyamide imide, polyetherimide, polyether ether ketone, polyimide, liquid crystal polymer, polytetrafluoroethylene, phenol resin, urea resin, melamine resin, unsaturated polyester, epoxy resin, silicon resin, and the like.

Examples of polyethylene include high-density polyethylene, linear low-density polyethylene, high-pressure process low-density polyethylene, ethylene-$\alpha$-olefin copolymer, and ethylene-vinyl acetate copolymer.

The ethylene-$\alpha$-olefin copolymer is a copolymer comprising a constitutional unit derived from ethylene and a constitutional unit derived from an $\alpha$-olefin. Examples of the $\alpha$-olefin include propylene, 1-butene, 1-pentene, 1-hexene, 1-heptene, 1-octene, 4-methyl-1-pentene and 4-methyl-1-hexene, and these may be alone or in combination of two or more. The $\alpha$-olefin is preferably an $\alpha$-olefin having 4 to 8 carbon atoms, and more preferably 1-butene, 1-hexene or 1-octene.

The density of the high-density polyethylene, the linear low-density polyethylene, the high-pressure process low-density polyethylene and ethylene-$\alpha$-olefin copolymer is 860 $kg/m^3$ to 970 $kg/m^3$.

Polypropylene is a polymer having 50% by weight or more of a constitutional unit derived from propylene, and is propylene homopolymer or a copolymer having a constitutional unit derived from propylene and another constitutional unit. The copolymer may be a random copolymer or a block copolymer.

Examples of the propylene random copolymer include a propylene-ethylene random copolymer comprising a constitutional unit derived from propylene and a constitutional unit derived from ethylene, a propylene-$\alpha$-olefin random copolymer comprising a constitutional unit derived from propylene and a constitutional unit derived from $\alpha$-olefin having 4 or more carbon atoms, a propylene-ethylene-$\alpha$-olefin copolymer comprising a constitutional unit derived from propylene

and a constitutional unit derived from ethylene and a constitutional unit derived from α-olefin having 4 or more carbon atoms.

Examples of the propylene block copolymer include a polymer material consisting of "a polymer component comprising propylene homopolymer component or a constitutional unit mainly derived from propylene (hereinafter referred to as polymer component (I))" and "a copolymer component consisting of one or more constitutional units selected from the group consisting of a constitutional unit derived from propylene, a constitutional unit derived from ethylene and/or a constitutional unit derived from an α-olefin having 4 or more carbon atoms (hereinafter referred to as copolymer component (II))".

Specific examples of the α-olefin having 4 or more carbon atoms that constitutes polypropylene include 1-butene, 1-pentene, 1-hexene, 4-methyl-1-pentene and 1-decene. The number of carbon atoms of the α-olefin is preferably 4 to 20, more preferably 4 to 12.

Examples of the propylene-α-olefin random copolymer include propylene-1-butene random copolymer and propylene-1-hexene random copolymer. Examples of the propylene-ethylene-α-olefin copolymer include propylene-ethylene-1-butene copolymer and propylene-ethylene-1-hexene copolymer.

In the polymer material consisting of the polymer component (I) and the copolymer component (II), the amount of the constitutional unit derived from propylene in "a polymer component comprising a constitutional unit mainly derived from propylene" as a polymer component (I) is more than 90% by weight to 99.9% by weight, based on 100% by weight of the weight of the polymer component (I). Examples of "a polymer component comprising a constitutional unit mainly derived from propylene" as a polymer component (I) include propylene-ethylene copolymer component, propylene-1-butene copolymer component and propylene-1-hexene copolymer component. Examples of the copolymer component (II) include propylene-ethylene copolymer component, propylene-ethylene-1-butene copolymer component, propylene-ethylene-1-hexene copolymer component, propylene-1-butene copolymer component and propylene-1-hexene copolymer component. The amount of the constitutional unit derived from propylene contained in the copolymer component (II) is 30% to 90% by weight, based on 100% by weight of the weight of the copolymer component (II).

Examples of the polymer material consisting of the polymer component (I) and the copolymer component (II) include
(propylene)-(propylene-ethylene) copolymer,
(propylene)-(propylene-ethylene-1-butene) copolymer,
(propylene)-(propylene-ethylene-1-hexene) copolymer,
(propylene)-(propylene-1-butene) copolymer,
(propylene)-(propylene-1-hexene) copolymer,
(propylene-ethylene)-(propylene-ethylene) copolymer,
(propylene-ethylene)-(propylene-ethylene-1-butene) copolymer,
(propylene-ethylene)-(propylene-ethylene-1-hexene) copolymer,
(propylene-ethylene)-(propylene-1-butene) copolymer,
(propylene-ethylene)-(propylene-1-hexene) copolymer,
(propylene-1-butene)-(propylene-ethylene) copolymer,
(propylene-1-butene)-(propylene-ethylene-1-butene) copolymer,
(propylene-1-butene)-(propylene-ethylene-1-hexene) copolymer,
(propylene-1-butene)-(propylene-1-butene) copolymer, and
(propylene-1-butene)-(propylene-1-hexene) copolymer.

The polypropylene is preferably propylene homopolymer, propylene-ethylene random copolymer, propylene-1-butene random copolymer, propylene-ethylene-1-butene copolymer, (propylene)-(propylene-ethylene) copolymer.

The isotactic pentad fraction of the polypropylene as measured by $^{13}$C-NMR is preferably 0.95 or more, more preferably 0.98 or more.

The isotactic pentad fraction refers to a fraction, as measured by $^{13}$C-NMR, of isotactic chain in the form of pentad unit in the polypropylene molecular chain, in other words, a fraction of the constitutional unit derived from propylene present at the center of a chain consisting of five constitutional units derived from propylene contiguously meso-bonded. The isotactic pentad fraction is determined by the method disclosed by A. Zambelli et al. in Macromolecules 6, 925 (1973), specifically, the ratio of the peak area of absorption assigned to methyl carbon of the constitutional unit derived from propylene present at the center of a chain consisting of five constitutional units derived from propylene contiguously meso-bonded to the peak area of absorption within the methyl carbon region, as measured by $^{13}$C-NMR spectrum.

Examples of methods for producing polypropylene include a method of homopolymerization using a Ziegler-Natta type catalyst or a metallocene catalyst, a method of copolymerizing propylene and one or more olefins selected from olefins other than propylene, and the like. Examples of the Ziegler-Natta type catalyst include a catalyst system containing a titanium-containing solid transition metal component and an organometallic component. Examples of the metallocene catalyst include a catalyst system comprising a transition metal compound of groups 4 to 6 of the periodic table having at least one cyclopentadienyl skeleton and a promoter component.

Examples of methods of polymerization include slurry polymerization or solution polymerization carried out in an inert hydrocarbon solvent, liquid phase polymerization or gas phase polymerization carried out in the absence of a solvent, and gas-gas phase polymerization or liquid-gas phase polymerization caring out these polymerizations continuously. The method may be batch type or continuous type. Also, the method may produce polypropylene in one step or in multiple, i.e., two or more, steps.

Particularly, preferred Examples of methods for producing a polymer material consisting of the polymer component (I) and the copolymer component (II) include a method comprising at least two steps, one of which is a step of preparing the polymer component (I) and the other is a step of preparing the copolymer component (II).

The amount of the thermoplastic resin (2) in the resin product of the invention is preferably 1% to 99% by weight, more preferably 5% to 95% by weight, even more preferably 10% to 90% by weight, and further preferably 15% to 85% by weight, based on 100% by weight of the total amount of the thermoplastic resin (2) and the thermoplastic resin (3).

The total amount of the thermoplastic resin (2) and the thermoplastic resin (3) in the resin product is preferably 50% by weight or more, more preferably 55% by weight or more, and even more preferably 60% by weight or more, based on 100% by weight of the total amount of the resin product of the invention. The total amount of the thermoplastic resin (2) and the thermoplastic resin (3) in the resin product is preferably 99.9% by weight or less, more preferably 99.5% by weight or less, and even more preferably 98% by weight or less, based on 100% by weight of the total amount of the resin product of the invention.

The resin product of the invention may comprise only one of the thermoplastic resin (2) or may comprise two or more of the thermoplastic resin (2). The resin product of the invention may comprise only one of the thermoplastic resin (3) or may comprise two or more of the thermoplastic resin (3).

The resin product of the invention may contain known additives such as antioxidant, neutralizing agent, crosslinking agent, heat-resistant stabilizer, weathering stabilizer, pigment, filler, lubricant and flame retardant.

The resin product of the invention may comprise further a thermoplastic resin, which is different from the thermoplastic resin (2) and the thermoplastic resin (3).

One embodiment of the resin product of the invention is a resin composition comprising an active ingredient, and the thermoplastic resin (2) and the thermoplastic resin (3) as described above.

The amount of the active ingredient contained in the resin composition is preferably 0.0001% to 50% by weight, more preferably 0.1% to 50% by weight, even more preferably 0.5% to 45% by weight, further preferably 1% to 45% by weight, and further preferably 2% to 40% by weight, based on 100% by weight of the total amount of the resin composition.

The amount of the thermoplastic resin (2) contained in the above resin composition is preferably 1% to 99% by weight, more preferably from 5% to 95% by weight, even more preferably 10% to 90% by weight, and further preferably from 15% to 85% by weight, based on 100% by weight of the total amount of the thermoplastic resin (2) and the thermoplastic resin (3).

The total amount of the thermoplastic resin (2) and the thermoplastic resin (3) contained in the resin composition is preferably 50% by weight or more, more preferably 55% by weight or more, and even more preferably 60% or more, based on 100% by weight of the total amount of the resin composition of the invention. The total amount of the thermoplastic resin (2) and the thermoplastic resin (3) in the resin composition is preferably 99.9% by weight or less, more preferably 99.5% by weight or less, and even more preferably 98% by weight or less, based on 100% by weight of the total amount of the resin composition of the invention.

The resin composition may comprise only one of the thermoplastic resin (2) or may comprise two or more of the thermoplastic resin (2). The resin composition may comprise only one of the thermoplastic resin (3) or may comprise two or more of the thermoplastic resin (3).

The resin composition may contain known additives such as antioxidant, neutralizing agent, crosslinking agent, heat-resistant stabilizer, weathering stabilizer, pigment, filler, lubricant and flame retardant.

The resin composition may comprise further a thermoplastic resin, which is different from the thermoplastic resin (2) and the thermoplastic resin (3).

In view of molding processability, the melt flow rate (MFR) of the resin composition, as measured at a temperature of 190°C under a load of 21.18 N in accordance with JIS K7210, is preferably 0.1 g/10 min to 500 g/10 min, more preferably 0.5 g/10 min to 300 g/10 min, and even more preferably 1.0 g/10 min to 200 g/10 min.

Examples of methods for producing a resin composition include a method comprising melt-kneading of the thermoplastic resin (2), the thermoplastic resin (3) and an active ingredient to obtain the resin composition. Examples of methods of melt-kneading include a method using an extruder, Banbury mixer, a roll-type kneading machine, and the like. All the polymers and the active ingredient to be contained in the resin composition may be melt-kneaded together at once to obtain a resin composition, or one of the polymers to be contained in the resin composition and an active ingredient may be melt-kneaded to obtain a melt-kneaded material, followed by melt-kneading with another polymer to obtain the resin composition.

The resin composition may be molded by known methods to obtain a molded article. Examples of methods for producing the molded article include known molding methods such as injection molding, extrusion molding, press molding,

and powder molding.

Examples of the molded article include sheets, fibers, tubes, pellets and the like.

The molded article comprising the above resin composition may form a core-shell structure in which the resin composition is covered with a material different from said resin composition, or a core-shell structure in which a material different from the resin composition is covered with said resin composition. The material different from the above resin composition is a single thermoplastic resin, a resin composition comprising a component different from the component of the above resin composition, a resin composition containing no active ingredient, a metal or a non-metal inorganic material.

The molded article comprising the above resin composition may form a multilayer structure in which both sides or one side of the resin composition are covered with a layer comprising a material different from said resin composition or form a multilayer structure in which a layer comprising the above resin composition covers both sides of a material different from said resin composition.

In another embodiment of the resin product of the invention, the resin product has a first layer comprising the above thermoplastic resin (2) and a second layer comprising the above thermoplastic resin (3) and an active ingredient. Specifically, the resin product may be a resin product having multilayered structure comprising a first layer of the thermoplastic resin (2) and a second layer of the thermoplastic resin (3) and an active ingredient. The first layer may comprise only one of the thermoplastic resin (2) or may comprise two or more of the thermoplastic resin (2). The second layer may comprise only one of the thermoplastic resin (3) or may comprise two or more of the thermoplastic resin (3) .

The amount of the active ingredient in the second layer is preferably 0.0001% to 50% by weight, based on 100% by weight of the total amount of the second layer.

The first layer in the multilayered structure may comprise an active ingredient. In case that the first layer comprises an active ingredient, the active ingredient contained in the first layer is usually the same as that contained in the second layer. In case that the first layer comprises an active ingredient, the amount of the active ingredient in the first layer is preferably less than the amount of the active ingredient in the second layer.

The multilayered structure may contain known additives such as antioxidant, neutralizing agent, crosslinking agent, heat-resistant stabilizer, weathering stabilizer, pigment, filler, lubricant and flame retardant.

Each layer of the multilayered structure of the invention may comprise a thermoplastic resin different from the thermoplastic resin (2) or the thermoplastic resin (3).

Examples of methods for producing the multilayered structure include a multilayer extrusion molding method and a press molding method. A resin composition may be prepared by known method from the thermoplastic resin (3) and an active ingredient and used as a raw material for preparing the second layer.

Examples of the shape of the multilayered structure include sheet, fiber, tube, pellet and the like. The multilayered structure has a core-sheath structure in which the second layer is covered with the first layer.

The resin product of the invention is prone to release the active ingredient at a high temperature, but is less prone to release at a low temperature. The degree of which is expressed as a temperature switch performance. The temperature switch performance is the ratio of the release rate of the active ingredient at a high temperature to the release rate of the active ingredient at a low temperature. The greater the temperature switch performance, the greater the difference of release rate between higher temperature and lower temperature, thus, indicating that the active ingredient is released preferentially at a high temperature.

The temperature at which the release rate is measured may be varied depending on the type and the use of the resin product. The temperature switch performance <40°C/25°C> is the ratio of the release rate of the active ingredient at 40°C to that at 25°C. The temperature switch performance <60°C/40°C> is the ratio of the release rate of the active ingredient at 60°C to that at 40°C. The temperature switch performance <60°C/25°C> is the ratio of the release rate of the active ingredient at 60°C to that at 25°C.

In view of preferential release of the active ingredient at a high temperature, the temperature switch performance <40°C/25°C> is preferably is 3.0 or more, and more preferably 3.5 or more. The temperature switch performance <60°C/40°C> is preferably 3.5 or more, and more preferably 4.0 or more. The temperature switch performance <60°C/25°C> is preferably 10 or more, and more preferably 15 or more, and even more preferably 20 or more.

Examples of devices for sustained-release of an active ingredient utilizing the present invention include goods for agriculture, forestry and fishery, building materials, household items, daily goods, outdoor goods, sanitary goods, insect proofing materials, bird proofing materials, animal proofing materials, fragrances, clothing, pharmaceuticals, packaging films, cosmetics, antibacterial materials, mildew proofing materials, paints, coating agents, pet-related products, and the like. Examples of the goods for agriculture, forestry and fishery include mulch films, coating fertilizers and fishing nets, and the like. Examples of the building material include screen door, packing, joint material, wallpaper, and the like, in terms of proofing of pests, fungus and bacteria. Examples of the household item and daily goods include mosquito nets, insect-proofing nets, bedding covers, bedding bags, curtains, cushions, floor pillows, sofa covers, kitchen mats, bath mats, washstand mats, toilet mats, toilet sheet covers, clothing covers and carpets, and the like. Examples of the outdoor goods include tent, hammock, rope, sleeping bag, and the like. Examples of the sanitary goods include mask,

adhesive plaster, bandage, and the like. Examples of the insect proofing material include insect proofing sheet, bait agent, insect repellents for space, insect repellents for clothing and insect repellents for foods, and the like. Examples of the pet-related products include pet care goods such as toilet/toilet sheet, pet diaper, deodorant, insect repellent, insecticide, shampoo, and dental care products, and pet daily goods such as collar, bed/mat, gate/cage/circle, carry, and the like.

## Examples

The present invention is described in more detail with reference to the following Examples and Comparative Examples. The methods for the measurement of physical properties used in the Examples and the Comparative Examples are as follows.

(1) Storage Elastic Modulus

The storage elastic modulus was measured using Rheogel-E 4000 (manufactured by UBM). The resin composition was preheated at 180°C for 5 minutes and press-molded by pressing at the temperature of 180°C and the pressure of 10 MPa for 5 minutes and then cooling at the temperature of 30°C and the pressure of 5 MPa for 5 minutes. The obtained molded article was cut into a rectangular shape of 50 mm length × 3 mm width × 1 mm thickness and used as a measurement sample. The measurement was carried out at the frequency of 10 Hz under a tensile measurement mode. During the measurement, the temperature was raised from -100°C to 65°C stepwise at the temperature rise rate of 3°C/min. The storage elastic modulus was measured at intervals at which the temperature interval was 1.5°C or less.

(2) Temperature switch performance <40°C/25°C>

The temperature switch performance <40°C/25°C> was calculated by dividing the released amount of herbicidal heterocyclic compound per 20 hours at 40°C by the released amount of herbicidal heterocyclic compound per 20 hours at 25°C.

(3) Temperature switch performance <40°C/10°C>

The temperature switch performance <40°C/10°C> was measured at two time points. The temperature switch performance <40°C/10°C> (6 hours) was calculated by dividing the released amount of herbicidal heterocyclic compound per 6 hours at 40°C by the released amount of herbicidal heterocyclic compound per 6 hours at 10°C. The temperature switch performance <40°C/10°C> (20 hours) was calculated by dividing the released amount of herbicidal heterocyclic compound per 20 hours at 40°C by the released amount of herbicidal heterocyclic compound per 20 hours at 10°C.

(4) Temperature switch performance <60°C/25°C>

The temperature switch performance <60°C/25°C> was measured at two time points. The temperature switch performance <60°C/25°C> (6 hours) was calculated by dividing the released amount of herbicidal heterocyclic compound per 6 hours at 60°C by the released amount of herbicidal heterocyclic compound per 6 hours at 25°C. The temperature switch performance <60°C/25°C> (20 hours) was calculated by dividing the released amount of herbicidal heterocyclic compound per 20 hours at 60°C by the released amount of herbicidal heterocyclic compound per 20 hours at 25°C.

The release amount of the herbicidal heterocyclic compound was measured by the following method. A sheet containing the active ingredient was cut into 5 cm squares and entered into a LABORAN screw tube No.8 (manufactured by AS ONE Corporation), and 60 mL of ethanol was poured to make the sheet immersed in ethanol. The screw tube was closed with a lid and allowed to stand at a predetermined temperature for a predetermined time, and then the sheet was removed from the ethanol. The ethanol solution was measured using an ultraviolet-visible spectrophotometer UV-2450 (manufactured by Shimadzu Corporation) . The concentration of the herbicidal heterocyclic compound in the measurement sample was determined by comparing the absorbance at 289 nm of the herbicidal heterocyclic compound with the previously measured absorbance of the herbicidal heterocyclic compound/ethanol solution of known concentration, and the amount of the herbicidal heterocyclic compound released over the predetermined time was calculated.

(5) Limiting viscosity ([η], unit: dl/g)

A sample solution was prepared by dissolving 100 mg of the polymer at 135°C in 100 mL of tetralin containing 5% by weight of butylhydroxytoluene (BHT) as a thermal degradation inhibitor, and a blank solution consisting of 100 mL of tetralin containing 0.5% by weight of BHT was prepared. The relative viscosity (ηrel) of the polymer was determined

from the time of flow of the sample solution and the blank solution, which were measured using an Ubbelohde viscometer, and [η] was calculated using the equation (III).

$$[\eta] = 23.3 \times \log(\eta rel) \qquad (III)$$

(6) The number of branched chain having 5 or more carbon atoms per 1,000 carbon atoms

The carbon nuclear magnetic resonance spectrum ($^{13}C$-NMR) was obtained by carbon nuclear magnetic resonance method under the following measurement conditions, and the number was determined in accordance with the following calculation method.

<Measurement Conditions>

Apparatus: AVANCE 600 (Bruker Corporation)
Measurement solvent: Liquid mixture of 1,2-dichlorobenzene/1,2-dichlorobenzene-d4 = 75/25 (v/v)
Measurement temperature: 130°C
Measurement method: Proton decoupling method
Pulse width: 45 degrees
Pulse repetition time: 4 seconds
Measurement standard: tetramethylsilane
Window function: negative exponential function

<Calculation Method>

The number of branched chain having 5 or more carbon atoms per 1,000 carbon atoms was calculated as the sum of the two peak areas of the peak at around 38.20 to 39.0 ppm and that of the peak at around 35.8 to 36.5 ppm, assuming that the sum of the peak areas of all the peaks at 5 to 50 ppm was 1000.

(7) Melt flow rate (MFR, unit: g/10 minutes)

The melt flow rate was measured at the temperature of 190°C under a load of 21.18 N in accordance with JIS K7210.

[1] The number of constitutional unit (A) derived from ethylene, constitutional unit (B) of the formula (1) and constitutional unit (C) of the formula (2) in the polymer (unit: %)

Using a nuclear magnetic resonance spectrometer (NMR), a nuclear magnetic resonance spectrum (hereinafter referred to as NMR spectrum) was obtained under the following measurement conditions.
Hereinafter, "Polymer (11)" is Polymer A, Polymer B, Polymer C, and Polymer D as mentioned below.

<Measurement conditions for carbon nuclear magnetic resonance ($^{13}C$-NMR)>

Device: AVANCE III 600HD (Bruker BioSpin K.K.)

Measurement probe: 10mm cryoprobe

Measurement solvent: Liquid mixture of 1,2-dichlorobenzene/1,1,2,2-tetrachloroethane-$d_2$ = 85/15 (v/v)

Sample concentration: 100 mg/mL

Measurement temperature: 135°C

Measurement method: proton decoupling method

Number of transients: 256

Pulse width: 45 degrees

Pulse repetition time: 4 seconds

Measurement standard: tetramethylsilane

<The number of constitutional unit ($A_1$) derived from ethylene and constitutional unit ($C_1$) derived from methyl acrylate in ethylene-methyl acrylate copolymer> (unit: %)

With respect to the $^{13}$C-NMR spectrum of ethylene-methyl acrylate copolymer obtained under the above-described $^{13}$C-NMR measurement conditions, integral values within the following ranges $a_1$, $b_1$, $c_1$, $d_1$ and $e_1$ were determined, and the numbers of constitutional unit ($A_1$) derived from ethylene and the constitutional unit ($C_1$) derived from methyl acrylate were determined from the contents (numbers) of three types of diad (EE, EA, AA), which were calculated from the following equation. Herein, EE represents ethylene-ethylene diad, EA represents ethylene-methyl acrylate diad, and AA represents methyl acrylate-methyl acrylate diad.

$a_1$: 28.1-30.5 ppm

$b_1$: 31.9-32.6 ppm

$c_1$: 41.7 ppm

$d_1$: 43.1-44.2 ppm

$e_1$: 45.0-46.5 ppm

$EE = a_1/4 + b_1/2$

$EA = e_1$

$AA = c_1 + d_1$

$$\text{The number of constitutional unit } (A_1) = 100 - \text{the number of constitutional unit } (C_1)$$

$$\text{The number of constitutional unit } (C_1) = 100 \times (EA/2 + AA)/(EE + EA + AA)$$

<Conversion ($X_1$) of constitutional unit ($C_1$) derived from methyl acrylate to constitutional unit ($B_2$) of formula (1)> (unit: %)

In the Example in which ethylene-methyl acrylate copolymer was reacted with a long chain alkyl alcohol to obtain a polymer comprising constitutional unit ($A_2$) derived from ethylene, constitutional unit ($B_2$) of the formula (1) and constitutional unit ($C_2$) derived from methyl acrylate, the following integral values within the ranges $f_1$ and $g_1$ were determined with respect to the $^{13}$C-NMR spectrum of the polymer measured under the above $^{13}$C-NMR measurement conditions.

Then, the conversion ($X_1$) of the constitutional unit ($C_1$) derived from methyl acrylate contained in the ethylene-methyl acrylate copolymer, which were converted to the constitutional unit ($B_2$) of the formula (1) of the polymer (11), was calculated from the following equation.

$f_1$: 50.6-51.1 ppm

$g_1$: 63.9-64.8 ppm

$$\text{Conversion } (X_1) = 100 \times g_1/(f_1 + g_1)$$

<The number of constitutional unit ($A_2$) derived from ethylene, constitutional unit ($B_2$) of the formula (1), and constitutional unit ($C_2$) derived from methyl acrylate contained in polymer (11) > (unit: %)

The number of constitutional unit ($A_2$) derived from ethylene, the number of constitutional unit ($B_2$) of the formula (1), and the number of constitutional unit ($C_2$) derived from methyl acrylate contained in polymer (11) were calculated, respectively, from the following equations.
The number of constitutional unit ($A_2$) contained in polymer (11) = the number of constitutional units ($A_1$) contained in ethylene-methyl acrylate copolymer

The number of constitutional unit ($B_2$) contained in polymer (11) = (the number of constitutional unit ($C_1$) contained in ethylene-methyl acrylate copolymer) × conversion ($X_1$)/100

The number of constitutional unit ($C_2$) contained in polymer (11) = (the number of constitutional unit ($C_1$) contained in ethylene-methyl acrylate copolymer) - (the number of constitutional unit ($B_2$) contained in polymer (11))

The obtained number of the constitutional unit ($A_2$), the number of constitutional unit ($B_2$) and the number of constitutional unit ($C_2$) correspond, respectively, to the number of the constitutional units (A), the number of the constitutional unit (B) of the formula (1) and the constitutional unit (C) of the formula (2) (unit:%) .

<The number of constitutional unit (A3) derived from ethylene and constitutional unit (B3) derived from long chain $\alpha$-olefin contained in ethylene-long chain $\alpha$-olefin copolymer> (unit: %)

With respect to the $^{13}$C-NMR spectrum of the ethylene-long chain $\alpha$-olefin copolymer obtained under the above $^{13}$C-NMR measurement conditions, integral values within the following ranges a3, b3, c3, d3, d'3, e3, f3, g3, h3, i3 and j3 were determined, and the numbers of constitutional unit (A3) derived from ethylene and constitutional unit (B3) derived from long chain $\alpha$-olefin were determined from the contents (numbers) of eight types of triad (EEE, EEL, LEE, LEL, ELE, ELL, LLE, LLL) calculated from the following equations. Herein, E represents ethylene, and L represents $\alpha$-olefin.

a3: 40.6-40.1 ppm
b3: 38.5-38.0 ppm
c3: 36.3-35.8 ppm
d3: 35.8-34.3 ppm
d'3: 34.0-33.7 ppm
e3: 32.4-31.8 ppm
f3: 31.4-29.1 ppm
g3: 27.8-26.5 ppm
h3: 24.8-24.2 ppm
i3: 23.0-22.5 ppm
j3: 14.4-13.6 ppm

$$EEE = f3/2 - g3/4-(nL-7) \times (b3+c3+d'3)/4$$

$$EEL + LEE = g3 - e3$$

$$LEL = h3$$

$$ELE = b3$$

$$ELL + LLE = c3$$

$$LLL = a3 - c3/2 \ (LLL = d'3 \text{ when } a3-c3/2<0)$$

Here, nL is the average number of carbon atoms of long chain α-olefin.

$$\text{The number of constitutional unit (A3)} = 100 \times (EEE+EEL+LEE+LEL)/(EEE+EEL+LEE+LEL+ELE+ELL+LLE+LLL)$$

The number of constitutional unit (B3) = 100 - the number of constitutional unit (A3)

[II] Content of unreacted compound having an alkyl group of 14 to 30 carbon atoms (unit: % by weight)

In the preparation of polymer (11) in each Example, the obtained product is a mixture of polymer (11) and an unreacted compound having an alkyl group of 14 to 30 carbon atoms. The content of the unreacted compound having an alkyl group of 14 to 30 carbon atoms contained in the product was measured by the following method using gas chromatography (GC). The content of unreacted compound is a value as determined based on 100 % by weight of the total weight of the obtained polymer (11) and the unreacted compound.

[GC measurement conditions]

GC device: Shimadzu GC2014

Column: DB-5MS (60 m, 0.25mmΦ, 1.0 μm)

Column temperature: the column held at 40°C was heated to 300°C at the rate of 10°C/min and then held at 300°C for 40 minutes.

Vaporizing chamber/detector temperature: 300°C/300°C (FID)

Carrier gas: helium

Pressure: 220 kPa

Full flow: 17.0 mL/min

Column flow rate: 1.99 mL/min

Purge flow rate: 3.0 mL/min

Line speed: 31.8 cm/sec

Injection system/split ratio: split injection/6:1

Injection volume: 1 μL

Sample preparation method: 8 mg/mL (o-dichlorobenzene solution)

(1) Calibration curve preparation

[Solution preparation]

In a 9-mL vial tube, 5 mg of a standard and then 100 mg of n-tridecane as an internal standard substance were weighed, and 6 mL of o-dichlorobenzene was added as a solvent to dissolve the sample completely, and thus, a standard solution for making calibration curve was obtained. Two additional standard solutions were prepared in the same manner except that the amount of the standard was changed to 25 mg and 50 mg, respectively.

[GC measurement]

The standard solution for preparing calibration curve was measured under the GC measurement conditions described above, and a calibration curve, which represents the GC area ratio of the standard to the internal standard substance (horizontal axis) and the weight ratio of the standard to the internal standard substance (vertical axis), was prepared to determine the slope **a** of the calibration curve.

(2) Measurement of content of measuring object (unreacted compound having an alkyl group of 14 to 30 carbon atoms) in sample (product)

[Solution preparation]

In a 9-mL vial tube, 50 mg of a sample and 100 mg of n-tridecane were weighted, and 6 mL of o-dichlorobenzene was added to dissolve the sample completely at 80°C, and thus, a sample solution was obtained.

[GC measurement]

The sample solution was measured under the GC measurement conditions described above, and the content $P_S$ of the measuring object in the sample was calculated according to the following equation.

$P_S$: Content of measuring object in sample (% by weight)

$W_S$: Weight of sample (mg)

$W_{IS}$: Weight of internal standard substance (IS) (mg)

$A_S$: Peak area count number of measuring object

$A_{IS}$: Peak area count number of internal standard substance (IS)

a: Slope of calibration curve of measuring object

$$P_S = \frac{W_{IS} \times A_S}{W_S \times A_{IS} \times a} \times 100$$

[III] Method of evaluating physical properties of polymer (11) (1) Melting peak temperature ($T_m$, unit: °C), melting enthalpy (ΔH, unit: J/g) observed in a temperature range of 10°C or more and less than 60°C

In a differential scanning calorimeter (DSC Q100, TA Instruments), under nitrogen atmosphere, an aluminum pan loaded with approximately 5 mg of sample was (1) held at 150°C for 5 minutes, then (2) cooled from 150°C to -50°C at a rate of 5°C/minute, then (3) held at -50°C for 5 minutes, and then (4) heated from -50°C to about 150°C at a rate of 5°C/minute. The differential scanning calorimetry curve obtained by the calorimetric measurement in Step (4) was defined as a melt curve. The melt curve was analyzed by the method in accordance with JIS K7121-1987 to determine a melting peak temperature.

The melting enthalpy ΔH (J/g) was determined by analyzing the part of the melt curve in a temperature range of 10°C or more and less than 60°C in accordance with JIS K7122-1987.

(2) Ratio A defined by the equation (I) (unit: none)

The absolute molecular weight and the intrinsic viscosity were measured for each of the polymer (11) and the polyethylene standard substance 1475a (available from National Institute of Standards and Technology) by gel permeation chromatography (GPC) using an apparatus equipped with a light scattering detector and a viscosity detector.

GPC device: TOSOH HLC-8121 GPC/HT

Light scattering detector: Precision Detectors PD2040

Differential pressure viscosity detector: Viscotek H502

GPC column: TOSOH GMHHR-H(S) HT, three columns

Sample solution concentration: 2 mg/mL

Injection volume: 0.3 mL

Measurement temperature: 155°C

Dissolution condition: 145°C, 2 hr

Mobile phase: orthodichlorobenzene (added with 0.5 mg/mL of BHT)

Elution flow rate: 1 mL/min

Measurement time: about 1 hour

[GPC device]

TOSOH HLC-8121 GPC/HT was used as a GPC device equipped with a differential refractometer (RI). As a light scattering detector (LS), PD2040 (Precision Detectors) was connected to the GPC device. The scattering angle used for light scattering detection was 90°. As a viscosity detector (VISC), Viscotek H502 was connected to the GPC device. In the column oven of the GPC device, LS and VISC were set and connected in the order of LS, RI and VISC. For the calibration of LS and VISC and the correction of the delay volume between the detectors, a polystyrene standard substance Polycal TDS-PS-N (Malvern, weight average molecular weight Mw: 104,349, polydispersity: 1.04) was used at the concentration of 1 mg/mL. As a mobile phase and a solvent, orthodichlorobenzene added with dibutylhydroxytoluene as a stabilizer at a concentration of 0.5 mg/mL was used. The conditions for dissolving the sample were 145°C and 2 hours. The flow rate was adjusted to 1 mL/minute. Three TOSOH GMHHR-H(S) HT columns were connected and used. The temperatures of the column, the sample injection part, and the detectors were adjusted to 155°C. The concentration of the sample solution was adjusted to 2 mg/mL. The injection volume (sample loop volume) of the sample solution was adjusted to 0.3 mL. The refractive index increment (dn/dc) of NIST1475a and the sample in orthodichlorobenzene was adjusted to -0.078 mL/g. The dn/dc of the polystyrene standard substance was adjusted to 0.079 mL/g. For determining the absolute molecular weight and the intrinsic viscosity ([η]) from the data of the respective detectors, a calculation was carried out using data-processing software OmniSEC (version 4.7, Malvern) with reference to the reference "Size Exclusion Chromatography, Springer (1999)". The refractive index increment is the rate of change of the refractive index relative to the change of concentration.

According to the flowing method, $\alpha_1$ and $\alpha_0$ of the equation (I) were determined, and both of them were substituted into the equation (I) to calculate A.

$$A = \alpha_1/\alpha_0 \quad (I)$$

$\alpha_1$ is a value obtained by a method comprising plotting the measured data with respect to the logarithm of the

absolute molecular weight of the polymer (11) (horizontal axis) and the logarithm of the intrinsic viscosity of the polymer (11) (vertical axis), approximating according to the equation (I-I) in the least squares sense the logarithm of the absolute molecular weight and the logarithm of the intrinsic viscosity within the range from the logarithm of the weight-average molecular weight of the polymer (11) to the logarithm of the z-average molecular weight of the polymer on the horizontal axis, and defining the slope of the line of the equation (I-I) as $\alpha_1$.

$$\log[\eta_1] = \alpha_1 \log M_1 + \log K_1 \quad \text{(I-I)}$$

In the equation (I-I), $[\eta_1]$ represents the intrinsic viscosity (unit: dl/g) of the polymer (11), $M_1$ represents the absolute molecular weight of the polymer (11), and $K_1$ is a constant.

$\alpha_0$ is a value obtained by a method comprising plotting the measured data with respect to the logarithm of the absolute molecular weight of the polyethylene standard substance 1475a (horizontal axis) and the logarithm of the intrinsic viscosity of the polyethylene standard substance 1475a (vertical axis), approximating according to the equation (I-II) in the least squares sense the logarithm of the absolute molecular weight and the logarithm of the intrinsic viscosity within the range of from the logarithm of the weight-average molecular weight of the polyethylene standard substance 1475a to the logarithm of the z-average molecular weight of the polyethylene standard substance 1475a on the horizontal axis, and defining the slope of the line of the equation (I-II) as $\alpha_0$.

$$\log[\eta_0] = \alpha_0 \log M_0 + \log K_0 \quad \text{(I-II)}$$

In the equation (I-II), $[n_0]$ represents the intrinsic viscosity (unit: dl/g) of the polyethylene standard substance 1475a, $M_0$ represents the absolute molecular weight of the polyethylene standard substance 1475a, and $K_0$ is a constant.

[Reference Example 1]

In an autoclave type reactor, ethylene and methyl acrylate were copolymerized using tert-butyl peroxypivalate as a radical polymerization initiator at a reaction temperature of 195°C and a reaction pressure of 160 MPa to obtain ethylene-methyl acrylate copolymer A-1. The composition and MFR of the obtained copolymer A-1 were as follows.

The number of constitutional unit derived from ethylene: 85.3% (65.4% by weight), the number of constitutional unit derived from methyl acrylate: 14.7% (34.6% by weight), MFR (measured at 190°C and 21.18 N): 41 g/10 minutes

The atmosphere in a reactor equipped with a stirrer was replaced with nitrogen, and 100 parts by weight of A-1, 84.4 parts by weight of GINOL-16 (n-hexadecyl alcohol, GODREJ), and 0.2 parts by weight of titanium tetraisopropanolate (Nippon Soda Co., Ltd.) were added and heated with stirring at jacket temperature of 140°C under a pressure reduction of 1 kPa for 12 hours to obtain ethylene-n-hexadecyl acrylate-methyl acrylate copolymer (hereinafter referred to as polymer B). The physical properties of the obtained polymer B are shown in Table 3.

[Reference Example 2]

In an autoclave type reactor, ethylene and methyl acrylate were copolymerized using tert-butyl peroxypivalate as a radical polymerization initiator at a reaction temperature of 195°C and a reaction pressure of 160 MPa to obtain ethylene-methyl acrylate copolymer A-2. The composition and MFR of the obtained copolymer A-2 were as follows.

The number of constitutional units derived from ethylene: 87.1% (68.8% by weight), the number of constitutional units derived from methyl acrylate: 12.9% (31.2% by weight), MFR (measured at 190°C and 21.18 N): 40.5 g/10 minutes

The atmosphere in a reactor equipped with a stirrer was replaced with nitrogen, and 100 parts by weight of A-2, 82.2 parts by weight of Calcol 8098 (n-octadecyl alcohol, Kao Corporation) and 0.8 parts by weight of TA-90 (tetraoctadecyl orthotitanate, Matsumoto Fine Chemical Co., Ltd.) were added and heated with stirring at jacket temperature of 140°C under a pressure reduction of 1 kPa for 12 hours to obtain ethylene-n-hexadecyl acrylate-methyl acrylate copolymer (hereinafter referred to as polymer C). The physical properties of the obtained polymer C are shown in Table 3.

[Reference Example 3]

The example was carried out in the same manner as in Reference Example 1 except that 84.4 parts by weight of GINOL-16 (n-hexadecyl alcohol, GODREJ) was changed to 113.7 parts by weight of GINOL-22 (n-docosyl alcohol, GODREJ) to obtain ethylene-n-docosyl acrylate-methyl acrylate copolymer (hereinafter referred to as polymer D). The physical properties of the obtained polymer D are shown in Table 3.

[Example 1] To a 5-liter autoclave equipped with a stirrer, which was dried under reduced pressure and purged with nitrogen, was added 1.4 L of a toluene solution containing 706 g of $\alpha$-olefin C2024 (a mixture of olefins having 18, 20, 22, 24 and 26 carbon atoms, produced by INEOS), and subsequently, toluene was added so that the liquid volume was 3L. The temperature of the autoclave was raised to 60°C, and then ethylene was added so that the partial pressure thereof was 0.1 MPa to stabilize the system. A solution of triisobutyl aluminum in hexane (0.34 mol/L, 14.7 ml) was added thereto. Then, a solution of dimethylanilinium tetrakis(pentafluorophenyl)borate in toluene (1.0 mmol/13.4 mL) and a solution of diphenylmethylene(cyclopentadienyl) (fluorenyl)zirconium dichloride in toluene (0.2 mmol/L, 7.5 mL) were added to initiate polymerization, and ethylene gas was fed to keep the total pressure. After 3 hours, 2 ml of ethanol was added to stop the polymerization. After the stop of the polymerization, the toluene solution containing the polymer was poured into acetone to precipitate olefin polymer, which was then corrected by filtration and washed twice with acetone. The obtained olefin polymer was vacuum dried at 80°C to yield 369 g of the olefin polymer (hereinafter referred to as polymer A). The polymer A has [$\eta$] of 1.2 dl/g, and the number of branches having 5 or more carbon atoms per 1,000 carbon atoms was of 30. Physical properties of the obtained polymer A are shown in Table 3.

39.00 g of polymer A, 5.00 g of HI-ZEX 3300F (high-density polyethylene, storage modulus E' at 60°C = 7.4 $\times$ $10^8$ Pa, Prime Polymer Co., Ltd.), 4.75 g of M8500 (high-density polyethylene, storage modulus E' at 60°C = 1.2 $\times$ $10^9$ Pa, Keiyo Polyethylene Corporation) and 1.25 g of a herbicidal heterocyclic compound having melting point of 204°C as an active ingredient were placed in Labo Plastomill R-60H (Toyo Seiki Seisakusho, Ltd.) at 210°C and kneaded at 80 rpm for 5 minutes to obtain a resin composition. The MFR of the resin composition was 114 g/10 min. The resin composition was preheated at 180°C for 5 minutes and press-molded by pressing at 180°C and 10 MPa for 5 minutes and then cooling at 30°C and 5 MPa for 5 minutes to obtain a sheet being 500 $\mu$m in thickness, and the temperature switch performance <40°C/25°C> was measured. The results are shown in Table 1.

[Example 2]

The example was carried out in the same manner as in Example 1 except that 24.38 g of polymer A, 12.43 g of HI-ZEX 3300F (high-density polyethylene, storage modulus E' at 60°C = 7.4 $\times$ $10^8$ Pa, Prime Polymer Co., Ltd.), 11.94 g of M8500 (high-density polyethylene, storage modulus E' at 60°C = 1.2 $\times$ $10^9$ Pa, Keiyo Polyethylene Corporation) and 1.25 g of the herbicidal heterocyclic compound having melting point of 204°C were placed in the Labo Plastomill. The results are shown in Table 1.

[Example 3]

The example was carried out in the same manner as in Example 1 except that 9.75 g of polymer A, 19.90 g of HI-ZEX 3300F (high-density polyethylene, storage modulus E' at 60°C = 7.4 $\times$ $10^8$ Pa, Prime Polymer Co., Ltd.), 19.10 g of M8500 (high-density polyethylene, storage modulus E' at 60°C = 1.2 $\times$ $10^9$ Pa, Keiyo Polyethylene Corporation) and 1.25 g of the herbicidal heterocyclic compound having melting point of 204°C were placed in the Labo Plastomill. The results are shown in Table 1.

[Comparative Example 1]

The example was carried out in the same manner as in Example 1 except that 24.86 g of HI-ZEX 3300F (high-density polyethylene, storage modulus E' at 60°C = 7.4 $\times$ $10^8$ Pa, Prime Polymer Co., Ltd.), 23.89 g of M8500 (high-density polyethylene, storage modulus E' at 60°C = 1.2 $\times$ $10^9$ Pa, Keiyo Polyethylene Corporation) and 1.25 g of the herbicidal heterocyclic compound having melting point of 204 °C were placed in the Labo Plastomill. The results are shown in Table 2.

[Comparative Example 2]

The example was carried out in the same manner as in Example 1 except that 48.75 g of polymer A and 1.25 g of the herbicidal heterocyclic compound having melting point of 204°C were placed in the Labo Plastomill. The results are shown in Table 2. An attempt was made to measure the release rate of the herbicidal heterocyclic compound at 40°C, but the sheet shrunk and could not be measured.

[Example 4]

39.00 g of polymer B, 39.00 g of M8500 (high-density polyethylene, storage modulus E' at 60°C = 1.2 $\times$ $10^9$ Pa, Keiyo Polyethylene Corporation) and 2.00 g of a herbicidal heterocyclic compound having melting point of 204°C as an active ingredient were placed in Labo Plastomill R-60H (Toyo Seiki Seisakusho, Ltd.) at 210°C and kneaded at

80 rpm for 5 minutes to obtain a resin composition. The resin composition was preheated at 180°C for 5 minutes and press-molded by pressing at 180°C and 10 MPa for 5 minutes and then cooling at 30°C and 5 MPa for 5 minutes to obtain a sheet being 500 μm in thickness, and the temperature switch performance <40°C/10°C> (6 hours) and the temperature switch performance <40°C/10°C> (20 hours) were measured. The results are shown in Table 4.

[Comparative Example 3]

The example was carried out in the same manner as in Example 4 except that polymer B was not used and 78.00 g of M8500 (high-density polyethylene, storage modulus E' at 60°C = $1.2 \times 10^9$ Pa, Keiyo Polyethylene Corporation) and 2.00 g of the herbicidal heterocyclic compound having melting point of 204°C as an active ingredient were placed in the Labo Plastomill. The results are shown in Table 4.

[Example 5]

The example was carried out in the same manner as in Example 1 except that 54.64 g of polymer C, 23.36 g of M8500 (high-density polyethylene, storage modulus E' at 60°C = $1.2 \times 10^9$ Pa, Keiyo Polyethylene Corporation) and 2.00 g of the herbicidal heterocyclic compound having melting point of 204°C were placed in the Labo Plastomill. The results are shown in Table 5.

[Example 6]

The example was carried out in the same manner as in Example 1 except that 46.80 g of polymer C, 31.20 g of M8500 (high-density polyethylene, storage modulus E' at 60°C = $1.2 \times 10^9$ Pa, Keiyo Polyethylene Corporation) and 2.00 g of the herbicidal heterocyclic compound having melting point of 204°C were placed in the Labo Plastomill. The results are shown in Table 5.

[Example 7]

80 parts by weight of Polymer C, 20 parts by weight of SUMITOMO NOBLEN D101 (propylene homopolymer, storage modulus E' at 60°C = $8.5 \times 10^8$ Pa, Sumitomo Chemical Co., Ltd.), 1.0 part by weight of Kayahexa AD-40C (a mixture comprising 2,5-dimethyl-2,5-di(tert-butylperoxy)hexane and calcium carbonate and amorphous silicon dioxide having a one-minute half-life temperature of 180°C, Kayaku Akzo Corporation), 1.0 part by weight of Hi-Cross MS50 (a mixture of trimethylolpropane trimethacrylate and amorphous silicon dioxide, Seiko Chemical Co., Ltd.), 0.1 part by weight of IRGANOX 1010 (pentaerythritol=tetrakis [3-(3',5'-di-tert-butyl-4'-hydroxyphenyl)propionate], BASF A.G.), and 0.1 part by weight of IRGAFOS 168 (tris(2,4-di-tert-butylphenyl)phosphite, BASF A.G.) were extruded using a twin screw extruder (barrel diameter D = 75 mm, screw effective length L/barrel diameter D = 40) at screw rotate speed of 350 rpm, discharge amount of 200 kg/hr, barrel temperature of 200°C (anterior half section) and 220°C (posterior half section) and die temperature of 200°C to prepare a crosslinked resin composition. The example was carried out in the same manner as in Example 1 except that 39.00 g of the crosslinked resin composition, 39.00 g of M8500 (high-density polyethylene, storage modulus E' at 60°C = $1.2 \times 10^9$ Pa, Keiyo Polyethylene Corporation) and 2.00 g of the herbicidal heterocyclic compound having melting point of 204°C were placed in the Labo Plastomill. The results are shown in Table 5.

[Example 8]

39.00 g of polymer D, 39.00 g of M8500 (high-density polyethylene, storage modulus E' at 60°C = $1.2 \times 10^9$ Pa, Keiyo Polyethylene Corporation) and 2.00 g of a herbicidal heterocyclic compound having melting point of 204°C as an active ingredient were placed in Labo Plastomill R-60H (Toyo Seiki Seisakusho, Ltd.) at 210°C and kneaded at 80 rpm for 5 minutes to obtain a resin composition. The resin composition was preheated at 180°C for 5 minutes and press-molded by pressing at 180°C and 10 MPa for 5 minutes and then cooling at 30°C and 5 MPa for 5 minutes to obtain a sheet being 500 μm in thickness, and the temperature switch performance <60°C/25°C> (6 hours) and the temperature switch performance <60°C/25°C> (20 hours) were measured. The results are shown in Table 6.

[Comparative Example 4]

The example was carried out in the same manner as in Example 8 except that polymer D was not used and 78.00 g of M8500 (high-density polyethylene, storage modulus E' at 60°C = $1.2 \times 10^9$ Pa, Keiyo Polyethylene Corporation) and 2.00 g of the herbicidal heterocyclic compound having melting point of 204°C as an active ingredient were placed in the Labo Plastomill. The results are shown in Table 6.

[Example 9]

The example was carried out in the same manner as in Example 4 except that 14.40 g of polymer B, 57.60 g of M6910 (high-density polyethylene, storage modulus E' at 60°C = 8.9 × $10^8$ Pa, Keiyo Polyethylene Corporation) and 8.00 g of phenothrin (pyrethroid compound) as an active ingredient were placed in the Labo Plastomill to obtain a resin composition, followed by press-molding as described in Example 4 to obtain a sheet of the resin composition, and the temperature switch performance <40°C/10°C> (6 hours) was measured. The results are shown in Table 7.

[Example 10]

The example was carried out in the same manner as in Example 8 except that 14.40 g of polymer D, 57.60 g of M6910 (high-density polyethylene, storage modulus E' at 60°C = 8.9 × $10^8$ Pa, Keiyo Polyethylene Corporation) and 8.00 g of phenothrin (pyrethroid compound) as an active ingredient were placed in the Labo Plastomill to obtain a resin composition, followed by press-molding as described in Example 8 to obtain a sheet of the resin composition, and the temperature switch performance <60°C/25°C> (6 hours) was measured. The results are shown in Table 7.

[Table 1]

| | Example 1 | Example 2 | Example 3 |
|---|---|---|---|
| herbicidal heterocyclic compound [wt%] | 2.5 | 2.5 | 2.5 |
| Polymer A [wt%] | 78 | 48.8 | 19.5 |
| HI-ZEX 3300F [wt%] | 9.9 | 24.8 | 39.8 |
| M8500 [wt%] | 9.6 | 23.9 | 38.2 |
| MFR of resin composition [g/10 min] | 114 | 51 | 11 |
| temperature switch performance <40°C/25°C> | 16.8 | 4.7 | 3.7 |

[Table 2]

| | Comparative Example 1 | Comparative Example 2 |
|---|---|---|
| herbicidal heterocyclic compound [wt%] | 2.5 | 2.5 |
| Polymer A [wt%] | 0 | 97.5 |
| HI-ZEX 3300F [wt%] | 49.7 | 0 |
| M8500 [wt%] | 47.8 | 0 |
| MFR of resin composition [g/10 min] | 1.3 | 153 |
| temperature switch performance <40°C/ 25°C> | 2.2 | N/A (because of shrinkage of sheet at 40°C) |

[Table 3]

| Polymer | | Polymer A | Polymer B | Polymer C | Polymer D |
|---|---|---|---|---|---|
| Constitutional unit (A) | % | 84.6 | 85.3 | 87.1 | 85.3 |
| Constitutional unit (B) | % | 15.4 | 12.5 | 10.8 | 12.6 |
| Constitutional unit (C) | % | 0 | 2.2 | 2.0 | 2.1 |
| Content of unreacted compound having alkyl of 14 to 30 carbon atoms | wt% | - | 1.1 | 0.6 | 0.9 |
| Melting peak temperature Tm | °C | 36 | 23 | 36 | 52 |
| Melting enthalpy ΔH (10 to 60°C) | J/g | 83 | 63 | 81 | 97 |

(continued)

| Polymer | | Polymer A | Polymer B | Polymer C | Polymer D |
|---|---|---|---|---|---|
| Number-Average Molecular Weight Mn | g/mol | 214,000 | 36,000 | 41,000 | 41,000 |
| Weight-Average Molecular Weight Mw | g/mol | 387,000 | 153,000 | 163,000 | 233,000 |
| z-average molecular weight Mz | g/mol | 672,000 | 836,000 | 1,134,000 | 1,763,00 0 |
| Ratio A defined by Equation (I) | | 0.94 | 0.66 | 0.78 | 0.51 |

[Table 4]

| | Example 4 | Comparative Example 3 |
|---|---|---|
| herbicidal heterocyclic compound [wt%] | 2.5 | 2.5 |
| Polymer B [wt%] | 48.75 | 0 |
| M8500 [wt%] | 48.75 | 97.5 |
| MFR of resin composition [g/10 min] | 91 | 4.0 |
| temperature switch performance <40°C/10°C> (6 hours) | 3.5 | 2.9 |
| temperature switch performance <40°C/10°C> (20 hours) | 3.8 | 1.5 |

[Table 5]

| | Example 5 | Example 6 | Example 7 |
|---|---|---|---|
| herbicidal heterocyclic compound [wt%] | 2.5 | 2.5 | 2.5 |
| Polymer C [wt%] | 68.3 | 58.5 | 39.0 |
| M8500 [wt%] | 29.2 | 39 | 48.75 |
| D101 [wt%] | - | - | 9.75 |
| MFR of resin composition [g/10 min] | 175 | 126 | 5.8 |
| temperature switch performance <40°C/25°C> | 5.9 | 4.9 | 16 |

[Table 6]

| | Example 8 | Comparative Example 4 |
|---|---|---|
| herbicidal heterocyclic compound [wt%] | 2.5 | 2.5 |
| Polymer D [wt%] | 48.75 | 0 |
| M8500 [wt%] | 48.75 | 97.5 |
| MFR [g/10 min] | 126 | 4.0 |
| temperature switch performance <60°C/25°C> (6 hours) | 90 | 5.8 |
| temperature switch performance <60°C/25°C> (20 hours) | 78 | 6.8 |

[Table 7]

| | Example 9 | Example 10 |
|---|---|---|
| phenothrin[wt%] | 10 | 10 |
| Polymer B [wt%] | 18 | 0 |

(continued)

| | Example 9 | Example 10 |
|---|---|---|
| Polymer D [wt%] | 0 | 18 |
| M6910 [wt%] | 72 | 72 |
| MFR [g/10 min] | 59 | 67 |
| temperature switch performance <40°C/10°C> (6 hours) | 16 | - |
| temperature switch performance <60°C/25°C> (6 hours) | - | 10 |

**Claims**

**1.** A resin product comprising
an active ingredient;
a thermoplastic resin (2) having a melting enthalpy (ΔH) of 30 J/g or more, as observed in a temperature range of 10°C or more and less than 60°C by differential scanning calorimetry; and
a thermoplastic resin (3) having a storage elastic modulus E' at 60°C of $5.0 \times 10^5$ Pa or more, as determined by dynamic viscoelasticity measurement at a frequency of 10 Hz.

**2.** The resin product according to claim 1 wherein the thermoplastic resin (2) is a polymer (11) comprising a constitutional unit (B) represented by formula (1):

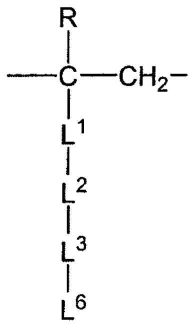

(1)

wherein

R represents hydrogen atom or methyl group,
$L^1$ represents a single bond, -CO-O-, -O-CO-, or -O-,
$L^2$ represents a single bond, $-CH_2-$, $-CH_2-CH_2-$, $-CH_2-CH_2-CH_2-$, $-CH_2-CH(OH)-CH_2-$, or $-CH_2-CH(CH_2OH)-$,
$L^3$ represents a single bond, -CO-O-, -O-CO-, -O-, -CO-NH-, -NH-CO-, -CO-NH-CO-, -NH-CO-NH-, -NH-, or $-N(CH_3)-$,
$L^6$ represents an alkyl group having 14 to 30 carbon atoms; in which the left side of the chemical formula recited for the definitions of the chemical structures of $L^1$, $L^2$ and $L^3$ corresponds to the top side of formula (1) and the right side thereof corresponds to the bottom side of formula (1).

**3.** The resin product according to claim 2 wherein the polymer (11) further comprises a constitutional unit (A) derived from ethylene, and optionally, further at least one constitutional unit (C) selected from the group consisting of constitutional units represented by formula (2):

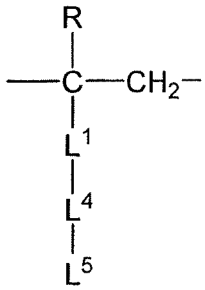

(2)

wherein

R represents hydrogen atom or methyl group,
$L^1$ represents a single bond, -CO-O-, -O-CO-, or -O-,
$L^4$ represents an alkylene group having 1 to 8 carbon atoms,
$L^5$ represents hydrogen atom, an epoxy group, $-CH(OH)-CH_2OH$, carboxyl group, hydroxyl group, amino group, or an alkylamino group having 1 to 4 carbon atoms;
in which the left side of the chemical formula recited for the definitions of the chemical structure of $L^1$ corresponds to the top side of formula (2) and the right side thereof corresponds to the bottom side of formula (2), and

the constitutional unit represented by formula (3):

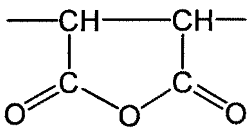

(3) ,

and
the number of the constitutional unit (A) is 70% to 99% and the total number of the constitutional unit (B) and the constitutional unit (C) is 1% to 30%, based on 100% of the total number of the constitutional unit (A), the constitutional unit (B) and the constitutional unit (C), and
the number of the constitutional unit (B) is 1% to 100% and the number of the constitutional unit (C) is 0% to 99%, based on 100% of the total number of the constitutional unit (B) and the constitutional unit (C).

4. The resin product according to claim 3 wherein the polymer (11) is a polymer wherein the total number of the constitutional unit (A), the constitutional unit (B) and the constitutional unit (C) is 90% or more, based on 100% of the total number of all constitutional units contained in the polymer (11).

5. The resin product according to any one of claims 1 to 4 wherein the ratio A defined by the following equation (I) of the thermoplastic resin (2) or the polymer (11) is 0.95 or less

$$A = \alpha_1/\alpha_0 \qquad (I)$$

wherein
$\alpha_1$ is a value obtained by a method comprising: measuring the absolute molecular weight and the intrinsic viscosity of the thermoplastic resin (2) or the polymer (11) by gel permeation chromatography using an apparatus equipped with a light scattering detector and a viscosity detector, plotting the measured data with respect to the logarithm of the absolute molecular weight (horizontal axis) and the logarithm of the intrinsic viscosity (vertical axis), approximating according to the equation (I-I) in the least squares sense the logarithm of the absolute molecular weight and the logarithm of the intrinsic viscosity within the range of from the logarithm of the weight-average molecular weight of the thermoplastic resin (2) or the polymer (11) to the logarithm of the z-average molecular weight of the thermoplastic resin (2) or the polymer (11), and defining the slope of the line of the equation (I-I) as $\alpha_1$

$$\log[\eta_1] = \alpha_1 \log M_1 + \log K_1 \quad \text{(I-I)}$$

wherein $[\eta_1]$ represents the intrinsic viscosity (dl/g) of the thermoplastic resin (2) or the polymer (11), $M_1$ represents the absolute molecular weight of the thermoplastic resin (2) or the polymer (11), and $K_1$ is a constant, and
$\alpha_0$ is a value obtained by a method comprising: measuring the absolute molecular weight and the intrinsic viscosity of a polyethylene standard reference materials 1475a (available from National Institute of Standards and Technology) by gel permeation chromatography using an apparatus equipped with a light scattering detector and a viscosity detector, plotting the measured data with respect to the logarithm of the absolute molecular weight (horizontal axis) and the logarithm of the intrinsic viscosity (vertical axis), approximating according to the equation (I-II) in the least squares sense the logarithm of the absolute molecular weight and the logarithm of the intrinsic viscosity within the range of from the logarithm of the weight-average molecular weight of the polyethylene standard material 1475a to the logarithm of the z-average molecular weight of the polymer, and defining the slope of the line of the equation (I-II) as $\alpha_0$

$$\log[\eta_0] = \alpha_0 \log M_0 + \log K_0 \quad \text{(I-II)}$$

wherein $[\eta_0]$ represents the intrinsic viscosity (dl/g) of the polyethylene standard material 1475a, $M_0$ represents the absolute molecular weight of the polyethylene standard material 1475a, and $K_0$ is a constant, and
in the measurement of the absolute molecular weight and the intrinsic viscosity of the thermoplastic resin (2) or the polymer (11) and the polyethylene standard material 1475a by gel permeation chromatography, the mobile phase is orthodichlorobenzene and the measurement temperature is 155°C.

**6.** The resin product according to any one of claims 1 to 5 wherein the thermoplastic resin (2) or the polymer (11) is a crosslinked polymer.

**7.** The resin product according to any one of claims 1 to 6 wherein the gel fraction is 20% by weight or more, based on 100% by weight of the weight of the resin product.

**8.** The resin product according to any one of claims 1 to 7 wherein the amount of the active ingredient contained in the resin product is 0.0001% to 50% by weight, based on 100% by weight of the total amount of the resin product, and the amount of the thermoplastic resin (2) or the polymer (11) is 1% to 99% by weight, based on 100% by weight of the total amount of the thermoplastic resin (2) or the polymer (11) and the thermoplastic resin (3).

**9.** The resin product according to any one of claims 1 to 8, comprising a first layer containing the thermoplastic resin (2) or the polymer (11) and a second layer containing the thermoplastic resin (3) and an active ingredient.

**10.** The resin product according to any one of claims 1 to 9 which is a molded article.

**11.** A device for sustained-release of an active ingredient, comprising a component consisting of the resin product according to any one of claims 1 to 10.

## INTERNATIONAL SEARCH REPORT

International application No.
PCT/JP2016/085651

A. CLASSIFICATION OF SUBJECT MATTER
*C08L101/00*(2006.01)i, *A01N25/10*(2006.01)i, *A01N25/34*(2006.01)i, *A01N43/00*(2006.01)i, *A01N53/08*(2006.01)i, *A01P7/00*(2006.01)i, *A01P13/00*(2006.01)i, *A61K47/32*(2006.01)i

According to International Patent Classification (IPC) or to both national classification and IPC

B. FIELDS SEARCHED

Minimum documentation searched (classification system followed by classification symbols)
C08L101/00, A01N25/10, A01N25/34, A01N43/00, A01N53/08, A01P7/00, A01P13/00, A61K47/32

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | RABAGLIATI,Franco M et al., Preparation and characterization of a new polymer/ pharmaceutical-based composite. Part I: Meloxicam, Polymer Bulletin, 2014.09.13, Vol.71, Issue 12, P3323-3331 | 1-5,8-11<br>6,7 |
| A | JP 3-31201 A (Nippon Shokubai Kagaku Kogyo Co., Ltd.), 12 February 1991 (12.02.1991), claims; examples (Family: none) | 1-11 |
| A | JP 4-297415 A (Mitsui Petrochemical Industries, Ltd.), 21 October 1992 (21.10.1992), claims; examples (Family: none) | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 December 2016 (26.12.16) | 10 January 2017 (10.01.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003128587 A
- WO 2015156416 A


### Non-patent literature cited in the description


- Size Exclusion Chromatography. Springer, 1999
- **KAKUGO, M. ; NAITO, Y. ; MIZUNUMA, K. ; MIYATAKE, T.** *Macromolecules,* 1982, vol. 15, 1150
- **A. ZAMBELLI et al.** *Macromolecules,* 1973, vol. 6, 925